# EUROPEAN PATENT APPLICATION

(11) **EP 4 582 430 A1**
(43) Date of publication of application: **09.07.2025**
(21) Application number: 23860459.9
(22) Date of filing: 30.08.2023
(51) Int. Cl.: C07D 495/14, A61K 31/519, A61K 31/635, A61P 27/02, A61P 27/14, A61P 43/00, C07D 495/22

(54) **COMPOUND, ENDOPLASMIC RETICULUM STRESS INHIBITOR, PHARMACEUTICAL PRODUCT, OPHTHALMIC AGENT, EYE DROPS, COMPOSITION, AND METHOD FOR PRODUCING COMPOUND**

(30) Priority: 31.08.2022 JP 2022138149
(71) Applicant: Kyoto University, Kyoto-shi, Kyoto 606-8501 (JP); Keio University, Tokyo, 108-8345 (JP); The Doshisha, Kyoto 602-8580 (JP)
(72) Inventor: UESUGI, Motonari, Kyoto-shi, Kyoto 606-8501 (JP); ABO, Masahiro, Kyoto-shi, Kyoto 606-8501 (JP); KOIZUMI, Noriko, Kyotanabe-shi, Kyoto 610-0394 (JP); OKUMURA, Naoki, Kyotanabe-shi, Kyoto 610-0394 (JP); KURIHARA, Toshihide, Tokyo 160-8582 (JP); IKEDA, Shin-ichi, Tokyo 160-8582 (JP)
(74) Representative: Leonard, Thomas Charles
(86) International application number: PCT/JP2023/031659
(87) International publication number: WO 2024/048697

(57) **Abstract**

A novel compound having self-assembly activity is provided. Provided is a compound, a tautomer or stereoisomer of the compound, or a salt of the compound or of the tautomer or stereoisomer of the compound, the compound being represented by the following formula: 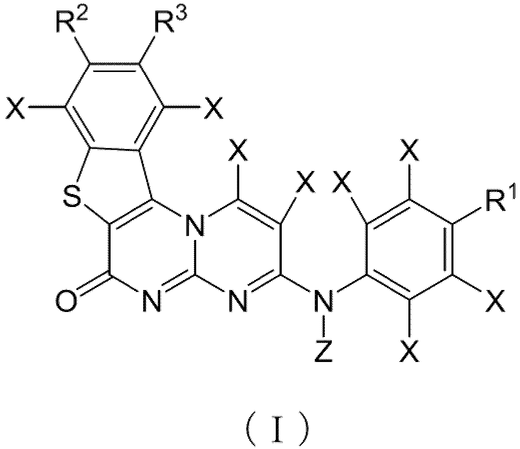 wherein
R¹ is a hydrogen atom or a substituent represented by -SO₂-R¹¹, wherein R¹¹ is a hydrogen atom or any substituent,
R² and R³ each represent a hydrogen atom, a halogen, a nitro group, a linear or branched alkyl group, a linear or branched alkoxy group, or a cyano group, wherein at least one hydrogen atom of the alkyl group or of the alkoxy group is optionally further substituted with a substituent, R² and R³ may be joined together to form a cyclic structure with the benzene ring to which they are attached, Xs independently represent a hydrogen atom or any substituent, and may be the same or different, and Z is a hydrogen atom or any substituent.

## Description

### Technical Field

The present disclosure relates to a compound, an endoplasmic reticulum stress inhibitor, a medicament, an ophthalmic agent, eye drops, a composition, and a method for producing the compound.

### Background Art

A phenomenon caused by accumulation of abnormal proteins (denatured proteins) in the endoplasmic reticulum is called "endoplasmic reticulum stress." Endoplasmic reticulum stress has been reported to be involved in age-related diseases such as neurodegenerative diseases like Alzheimer's disease, metabolic diseases and, ophthalmic diseases. As pharmaceutical agents that inhibit endoplasmic reticulum stress, chemical chaperones, such as PBA and TUDCA, are known (Non-Patent Literature (NPL) 1). Further, usefulness of such chemical chaperones in cells and animal models has been suggested (NPL 2).

### Citation List

### Patent Literature

NPL 1: Oezcan, U. et al., Chemical Chaperones Reduce ER Stress and Restore Glucose Homeostasis in a Mouse Model of Type 2 Diabetes, Science 313, 1137-1140 (2006).
NPL 2: Cortez, L. & Sim, V., The therapeutic potential of chemical chaperones in protein folding diseases, Prion 8, 197-202 (2014).

### Summary of the Invention

### Technical Problem

However, existing chemical chaperones have not yet been clinically applied. The problem with existing chemical chaperones is that their activity is limited to the effect of inhibiting the denaturation of normal proteins, and the existing chemical chaperones cannot remove proteins that have already been denatured. A novel compound having self-assembly activity, if provided, can provide, for example, a new technique that targets a denatured protein, which is also prone to self-assemble.

Accordingly, an object of the present disclosure is to provide a novel compound that has self-assembly activity, an endoplasmic reticulum stress inhibitor, a medicament, an ophthalmic agent, eye drops, a composition, and a method for producing the compound.

### Solution to Problem

In order to achieve the above object, the compound of the present disclosure is
a compound represented by the following chemical formula (I), a tautomer or stereoisomer of the compound, or a salt of the compound or of the tautomer or stereoisomer of the compound: 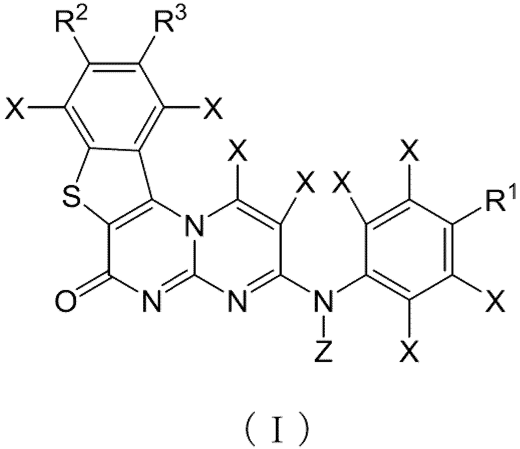 wherein in chemical formula (I)
R¹ is a hydrogen atom or a substituent represented by -SO₂-R¹¹, wherein R¹¹ is a hydrogen atom or any substituent,
R² and R³ each represent a hydrogen atom, a halogen, a nitro group, a linear or branched alkyl group, a linear or branched alkoxy group, or a cyano group, wherein at least one hydrogen atom of the alkyl group or of the alkoxy group is optionally further substituted with a substituent, or
R² and R³ may be joined together to form a cyclic structure with the benzene ring to which they are attached,
Xs independently represent a hydrogen atom or any substituent, and each X may be the same or different, and
Z is a hydrogen atom or any substituent.

The endoplasmic reticulum stress inhibitor of the present disclosure has a feature of containing the compound of the present disclosure, a tautomer or stereoisomer of the compound, or a salt of the compound or of the tautomer or stereoisomer of the compound.

The medicament of the present disclosure has a feature of containing the compound of the present disclosure, a tautomer or stereoisomer of the compound, or a salt of the compound or of the tautomer or stereoisomer of the compound.

The ophthalmic agent of the present disclosure has a feature of containing the compound of the present disclosure, a tautomer or stereoisomer of the compound, or a salt of the compound or of the tautomer or stereoisomer of the compound.

The eye drops of the present disclosure have a feature of containing the compound of the present disclosure, a tautomer or stereoisomer of the compound, or a salt of the compound or of the tautomer or stereoisomer of the compound.

The method for producing the compound of the present disclosure, a tautomer or stereoisomer of the compound, or a salt of the compound or of the tautomer or stereoisomer of the compound comprises the steps of:
a hydrolysis step of hydrolyzing a compound represented by the following chemical formula (II), a tautomer or stereoisomer of the compound, or a salt of the compound or of the tautomer or stereoisomer of the compound: 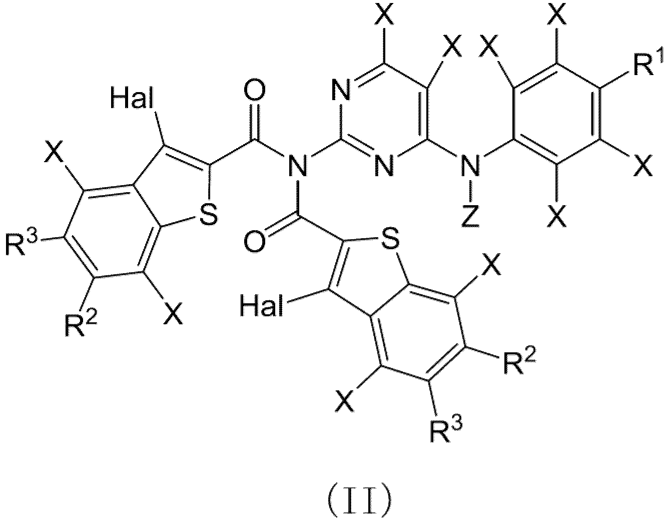 wherein in chemical formula (II), R¹, R², R³, Xs, and Z are the same as those defined in chemical formula (I), and Hal is a halogen; and
a cyclization step of cyclizing the product of the hydrolysis step.

### Effect of the Invention

According to the present disclosure, a novel compound that has self-assembly activity, an endoplasmic reticulum stress inhibitor, a medicament, an ophthalmic agent, eye drops, a composition, and a method for producing the compound can be provided.

### Brief Description of Drawings

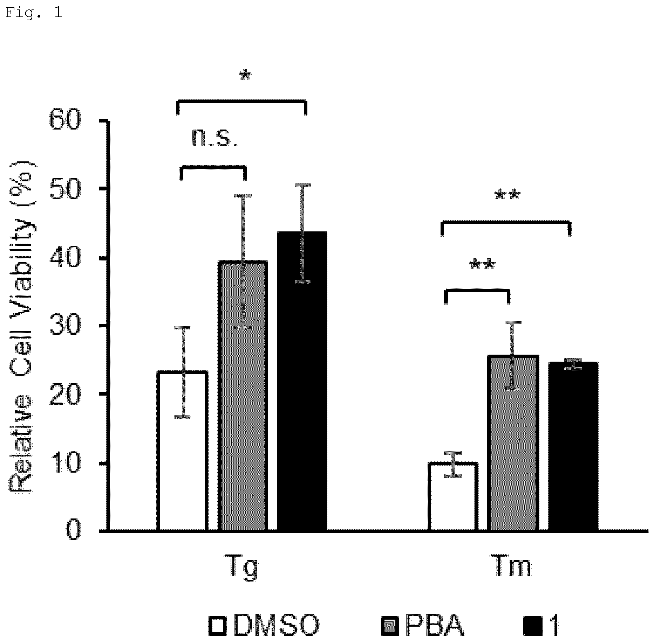

Fig. 1 shows the biological activity of compound 1 for reducing cytotoxicity under ER stress. This figure shows the rescue of cells from death under ER stress by PBA or compound 1. HEK293 cells were pre-incubated with PBA (2.0 mM) or compound 1 (100 µM) for 12 hours and then treated with 10 µM thapsigargin (Tg) or 5.0 µg/mL tunicamycin (Tm) for 48 hours. Then, the cell viability was compared between DMSO, PBA, and compound 1. The vertical axis of the graph represents cell viability.

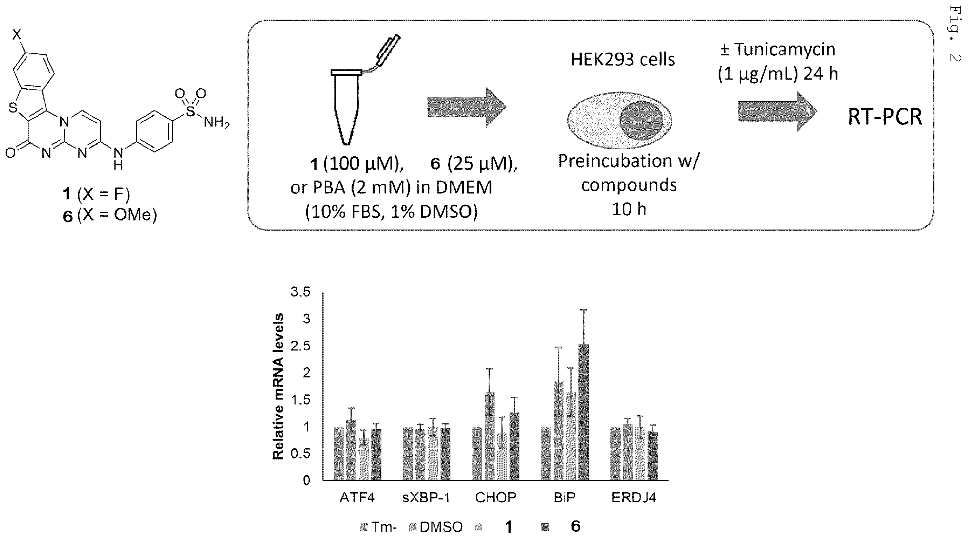

Fig. 2 shows the results of qPCR measurement of changes in mRNA levels of ER stress-related genes due to tunicamycin treatment. The mRNA expression levels of ER stress-related genes induced by compound 1 or compound 6 were measured by quantitative PCR. HEK293 cells were treated with compound 1 (100 µM) for 10 hours and then incubated with 1.0 µg/mL tunicamycin (Tm) for 24 hours. Isolated RNA samples were subjected to reverse transcription and quantitative RT-PCR. The bars in the bar graph show, from the left, the measurement results in the presence of tunicamycin (without compounds 1 and 6), in the absence of tunicamycin (without compounds 1 and 6, with DMSO only), in the presence of tunicamycin and compound 1, and in the presence of tunicamycin and compound 6.

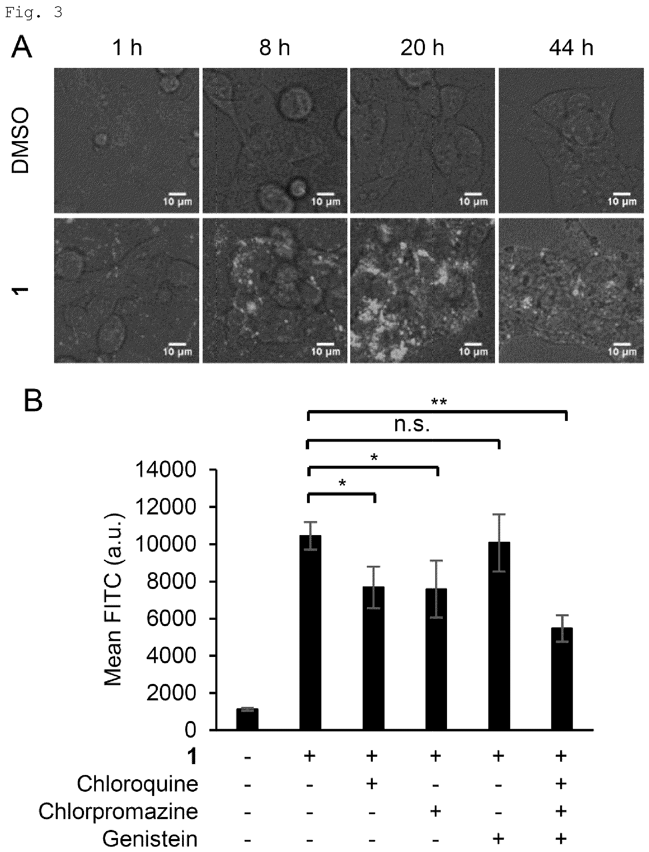

Fig. 3 shows the self-assembly of compound 1 in living HEK293 cells. Fig. 3(A) shows fluorescence images of HEK293 cells treated with compound 1 (100 µM) and DCVJ (1.0 µM). Fig. 3(B) shows quantification of compound 1 (100 µM) in cells stained with DCVJ (1.0 µM) by flow cytometry in the presence (+) or absence (-) of an endocytosis inhibitor (100 µM chloroquine, 20 µM chlorpromazine, and/or 100 µM genistein). The vertical axis of the graph represents the mean value of fluorescein isothiocyanate (FITC) in an arbitrary unit (a.u.).

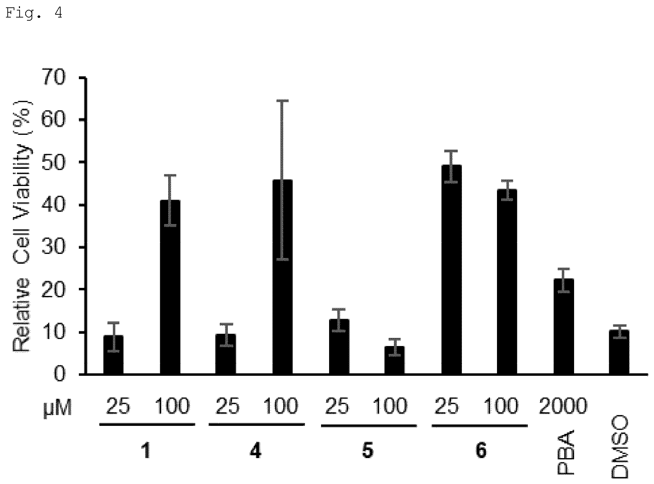

Fig. 4 shows changes in cell viability caused by compound 1 and its derivatives, compounds 4 to 6, under ER stress. The vertical axis of the graph represents cell viability (unit: %), and the horizontal axis of the graph represents the measurement results for compound 1, compound 4, compound 5, compound 6, PBA, and DMSO, from the left.

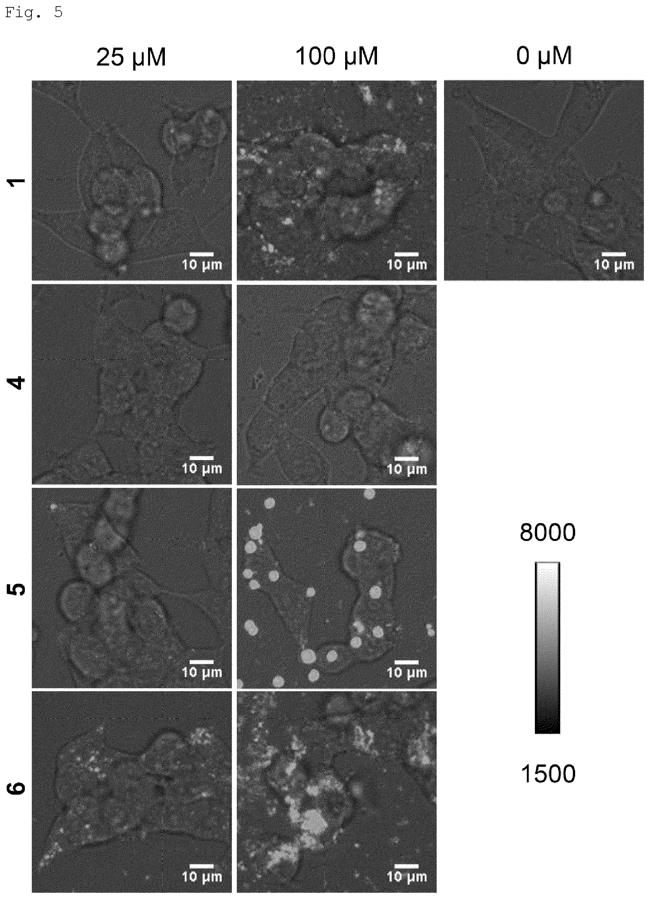

Fig. 5 shows fluorescence images of the self-assemblies of compound 1 and its derivatives in living cells.

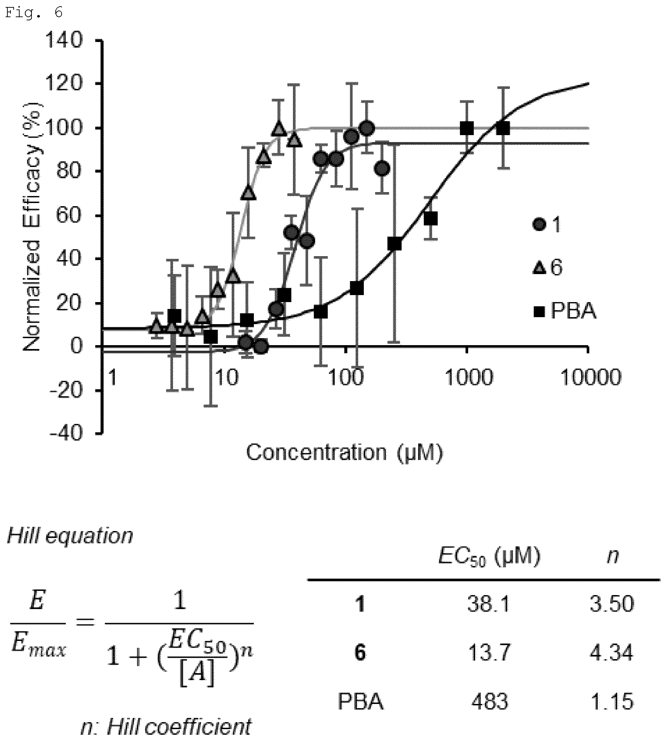

Fig. 6 is a graph showing dose-response curves in the tunicamycin assay using compound 1, compound 6, and PBA.

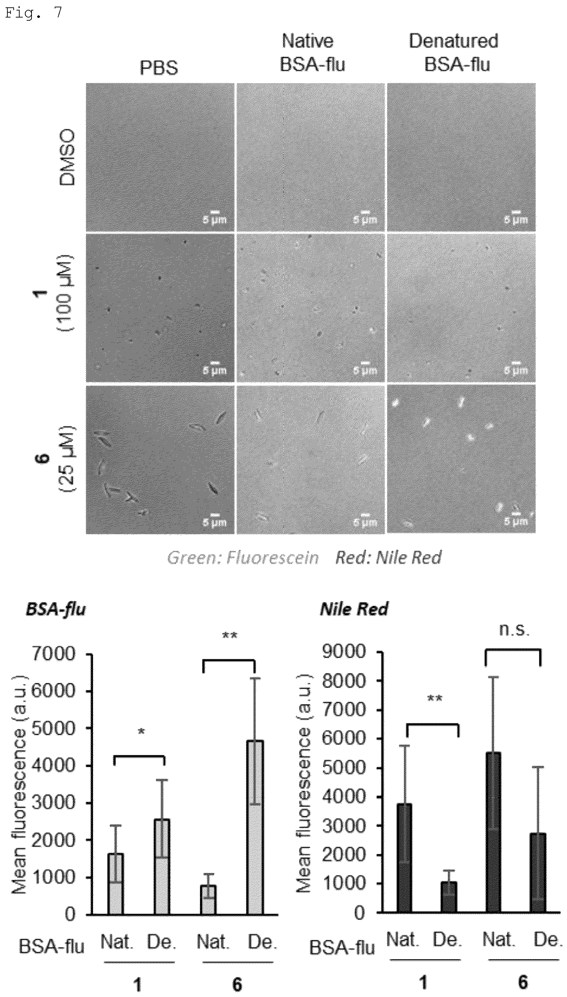

Fig. 7 shows the formation of a co-assembly between a self-assembling chemical (compound 1 or compound 6) and fluorescently labeled BSA (BSA-flu).

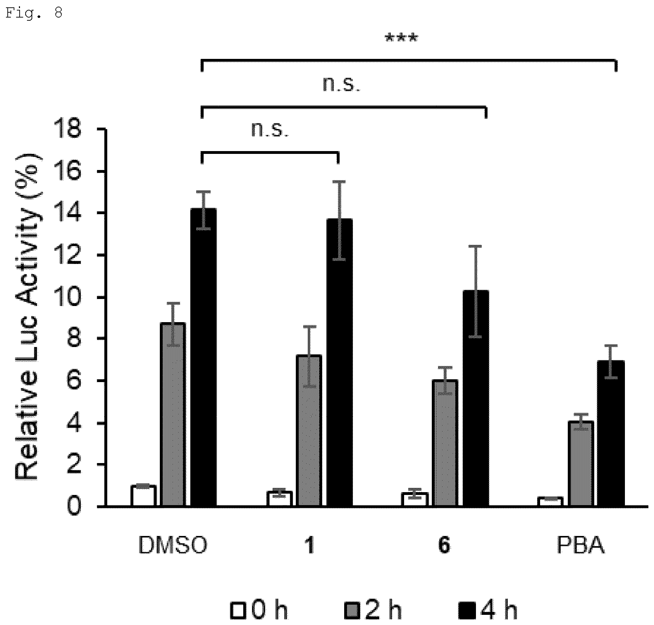

Fig. 8 is a graph showing the results of a luciferase-based refolding assay in living HEK293 cells. The vertical axis of the graph represents the relative activity of luciferase (unit: %), and the horizontal axis of the graph represents, from the left, the measurement results for DMSO alone, compound 1, compound 6, and PBA alone. The bars in the bar graph are color-coded according to the time of refolding in living cells with the results of refolding in living cells for 0 hours (0 h), 2 hours (2 h), and 4 hours (4 h) from the left.

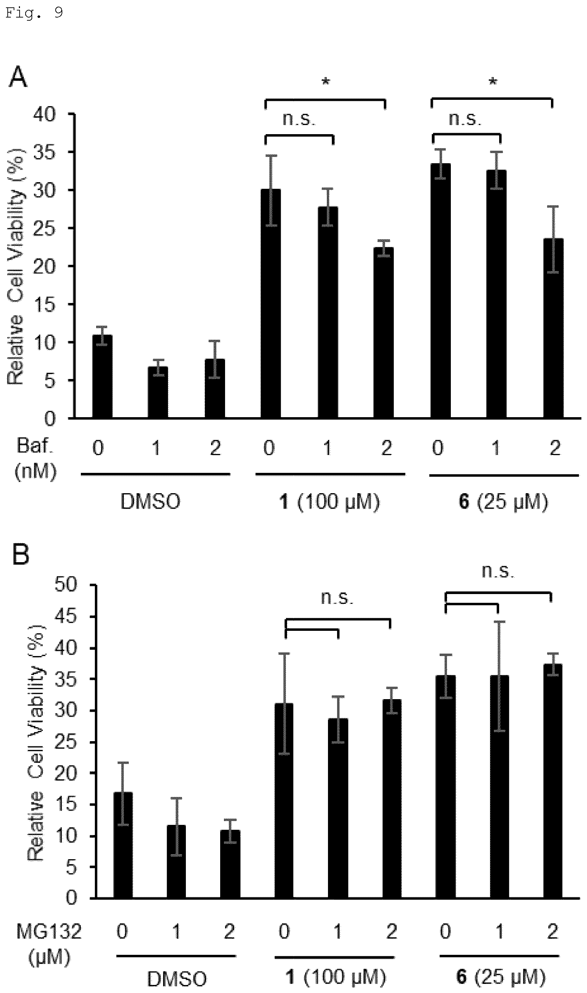

Fig. 9 shows the results of a tunicamycin assay using an autophagy or proteasome inhibitor. Fig. 9(A) is a graph showing measurements in the presence or absence of bafilomycin A1 (Baf A1, autophagy inhibitor). Fig. 9(B) is a graph showing measurements in the presence or absence of MG132 (proteasome inhibitor).

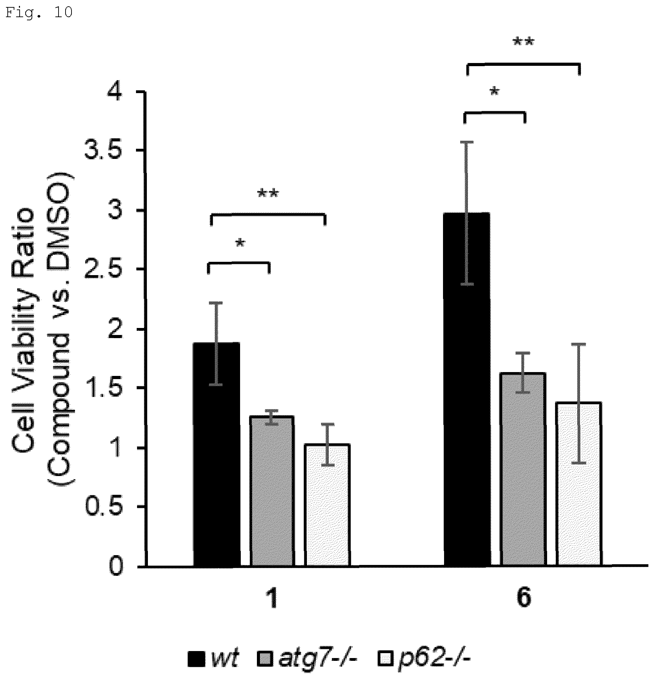

Fig. 10 is a graph showing the results of a tunicamycin assay using autophagy-deficient cells. The vertical axis of the graph represents cell viability.

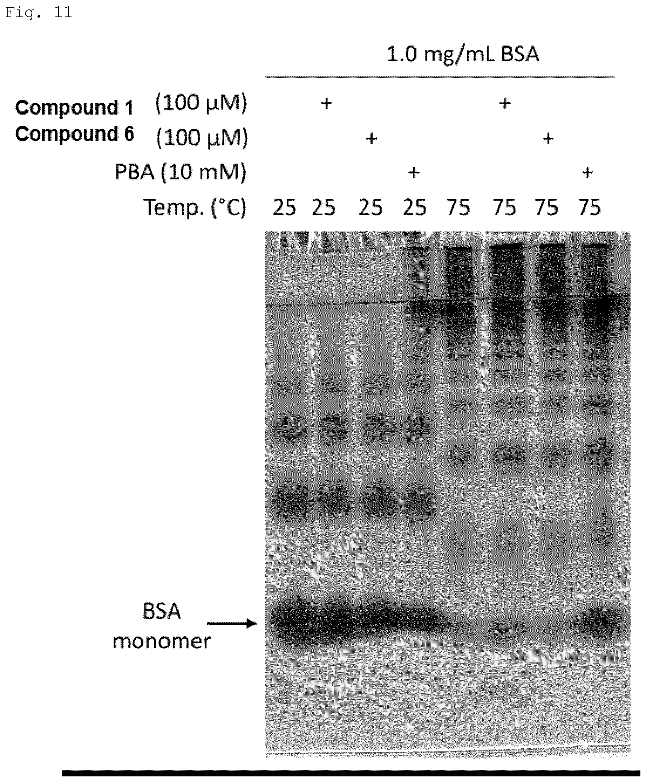

Fig. 11 is a photograph showing the results of CBB staining in electrophoresis by native PAGE (8% acrylamide gel). Specifically, Fig. 11 shows native-PAGE analysis of the heat-induced denaturation of BSA in the presence of compound 1, compound 6, or PBA. BSA (2.0 mg/mL) was incubated for 1 hour at 25°C or 75°C in the presence (denoted as +) or absence of compound 1 (100 µM), compound 6 (100 µM), or PBA (10 mM).

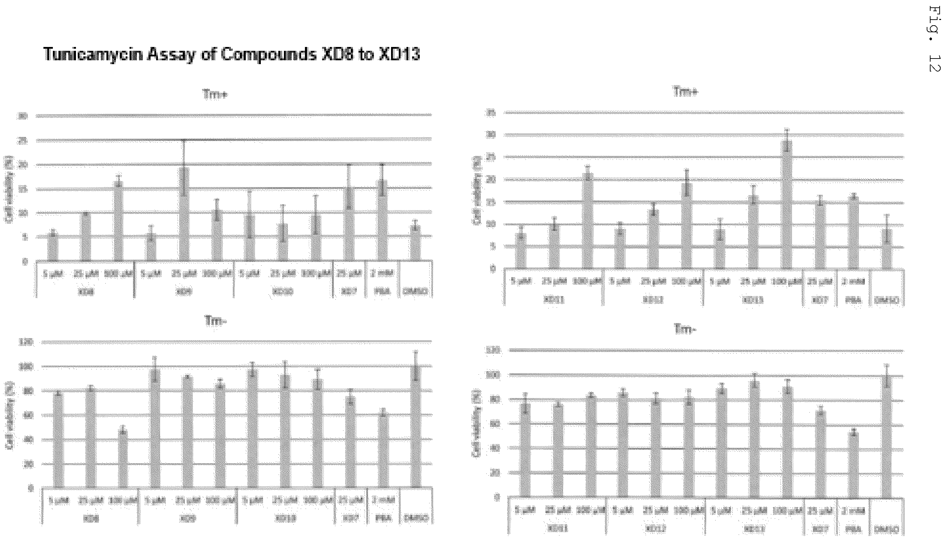

Fig. 12 shows data from a tunicamycin (Tm) assay for compounds XD8 to XD13 and XD7 (compound 6). The vertical axis of the graph represents cell viability (unit: %). In the two graphs on the left, the horizontal axis indicates, from the left, the measurement results for compound XD8, compound XD9, compound XD10, compound XD7 (compound 6), PBA, and DMSO. In the two graphs on the right, the horizontal axis indicates, from the left, the measurement results for compound XD11, compound XD12, compound XD13, compound XD7 (compound 6), PBA, and DMSO.

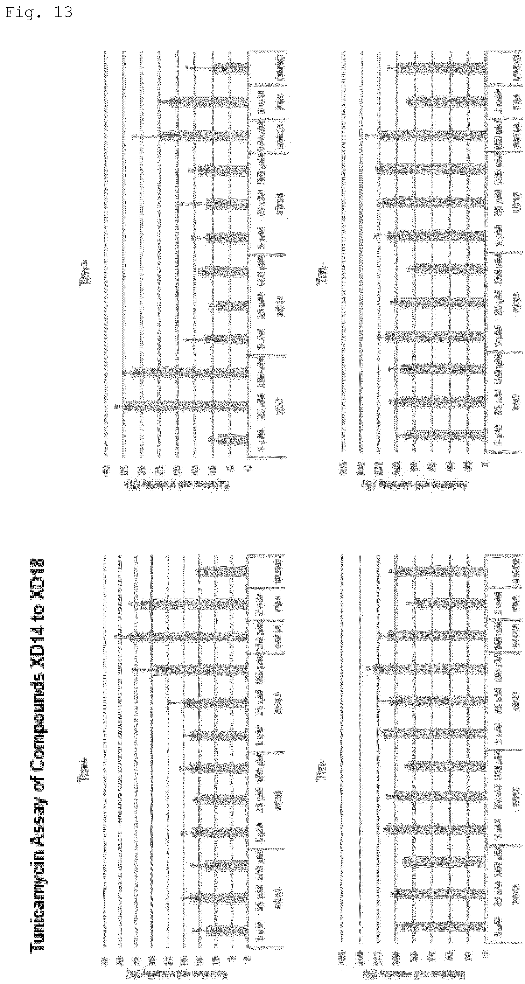

Fig. 13 shows data from a tunicamycin assay for compounds XD14 to XD18 and X441A (compound 1). The vertical axis of the graph represents relative cell viability (unit: %). In the two graphs on the left, the horizontal axis indicates, from the left, the measurement results for compound XD15, compound XD16, compound XD17, X441A (compound 1), PBA, and DMSO. In the two graphs on the right, the horizontal axis indicates, from the left, the measurement results for compound XD7 (compound 6), compound XD14, compound XD18, X441A (compound 1), PBA, and DMSO.

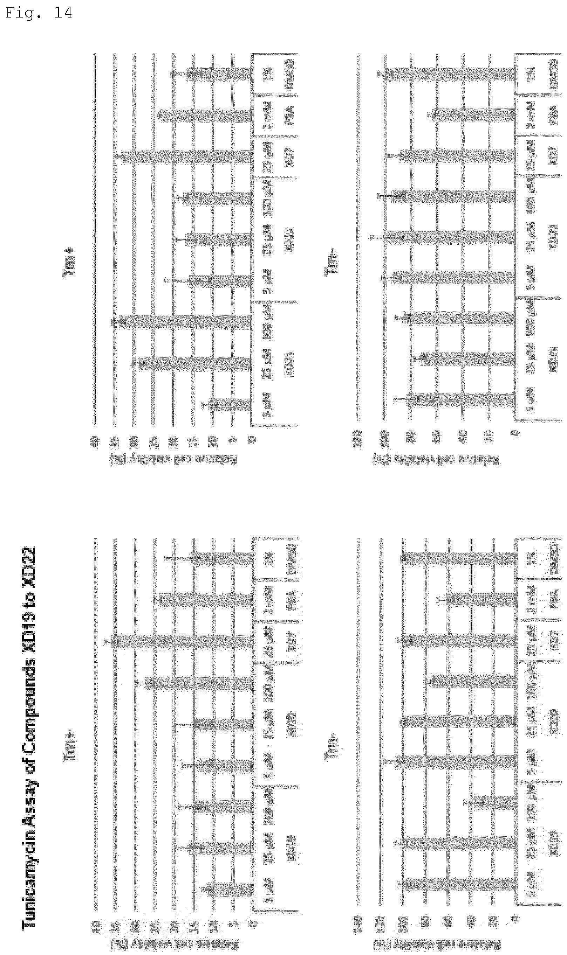

Fig. 14 shows data from a tunicamycin assay for compounds XD19 to XD22 and XD7 (compound 6). The vertical axis of the graph represents relative cell viability (unit: %). In the two graphs on the left, the horizontal axis indicates, from the left, the measurement results for compound XD19, compound XD20, compound XD7 (compound 6), PBA, and DMSO. In the two graphs on the right, the horizontal axis indicates, from the left, the measurement results for compound XD21, compound XD22, compound XD7 (compound 6), PBA, and DMSO.

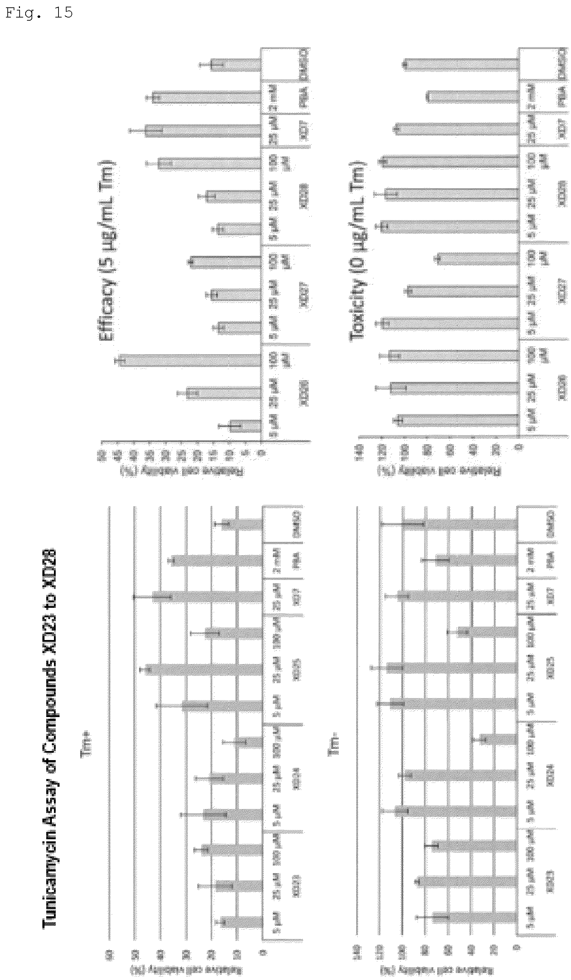

Fig. 15 shows data from a tunicamycin assay for compounds XD23 to XD28 and XD7 (compound 6). The vertical axis of the graph represents relative cell viability (unit: %). In the two graphs on the left, the horizontal axis indicates, from the left, the measurement results for compound XD23, compound XD24, compound XD25, compound XD7 (compound 6), PBA, and DMSO. In the two graphs on the right, the horizontal axis indicates, from the left, the measurement results for compound XD26, compound XD27, compound XD28, compound XD7 (Compound 6), PBA, and DMSO.

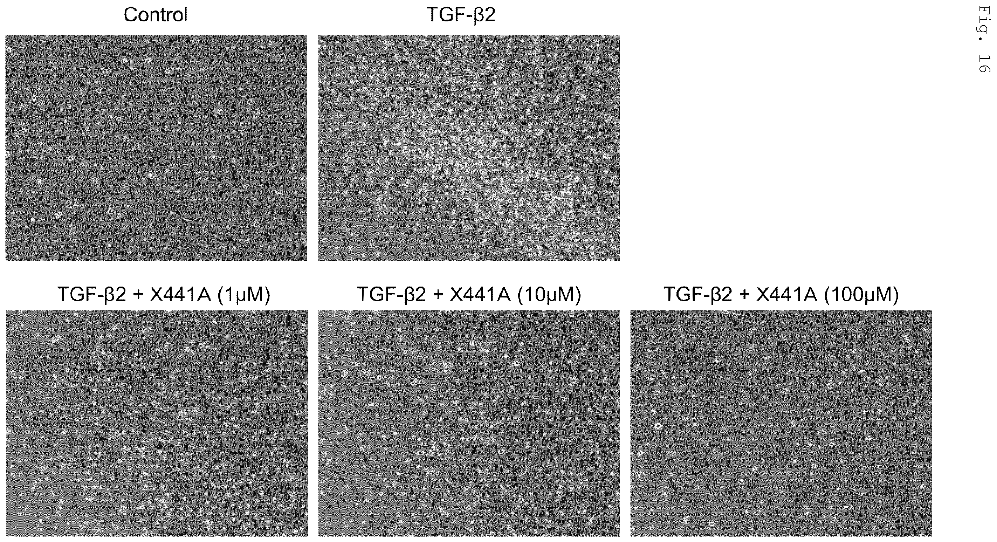

Fig. 16 shows phase-contrast micrographs of immortalized human corneal endothelial cells (iFECD) after 30 hours of stimulation with TGF-β2. The iFECD was derived from a patient with Fuchs' endothelial corneal dystrophy and had been pretreated with compound 1 (X441A), which is an endoplasmic reticulum stress inhibitor (upper panel: from the left, control and TGF-β2 are shown; lower panel: from the left, TGF-β2+X441A (compound 1, 1 µM), TGF-β2+X441A (compound 1, 10 µM), and TGF-β2+X441A (compound 1, 100 µM) are shown). Compound 1 (endoplasmic reticulum stress inhibitor) inhibits cell damage caused by TGF-β signaling in a corneal endothelial disorder model of Fuchs' endothelial corneal dystrophy.

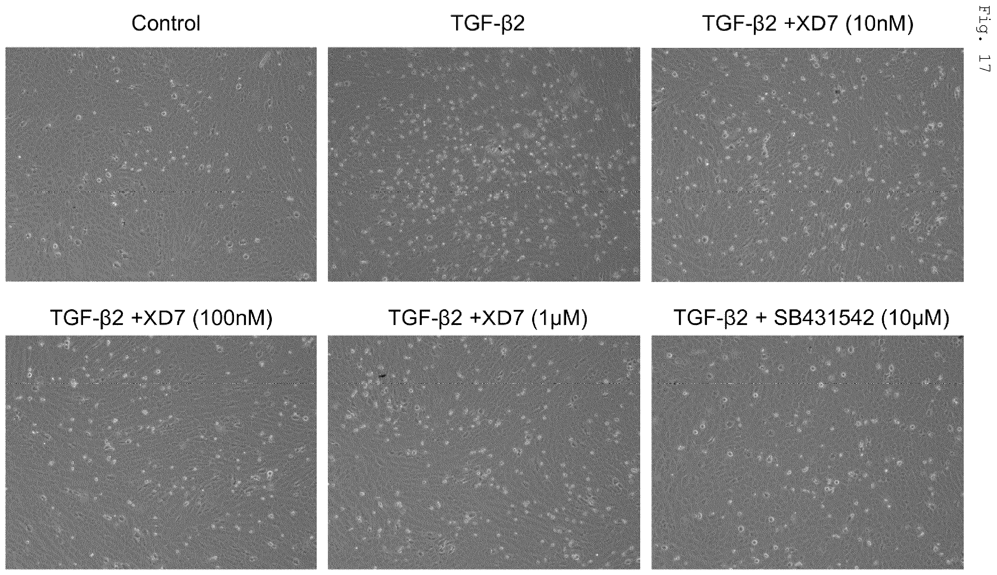

Fig. 17 shows phase-contrast micrographs of immortalized human corneal endothelial cells derived from a patient with Fuchs' endothelial corneal dystrophy after 30 hours of stimulation with TGF-β2; the immortalized human corneal endothelial cells had been pretreated with compound 6 (XD7), which is an endoplasmic reticulum stress inhibitor (upper panel: from the left, control, TGF-β2, and TGF-β2+XD7 (Compound 6, 10 nM) are shown). Lower panel: from the left, TGF-β2+XD7 (compound 6, 100 nM), TGF-β2+XD7 (compound 6, 1 µM), and TGF-β2+SB431542 (10 µM) are shown). Compound 6 (endoplasmic reticulum stress inhibitor) inhibits cell damage caused by TGF-β signaling in a corneal endothelial disorder model of Fuchs' endothelial corneal dystrophy.

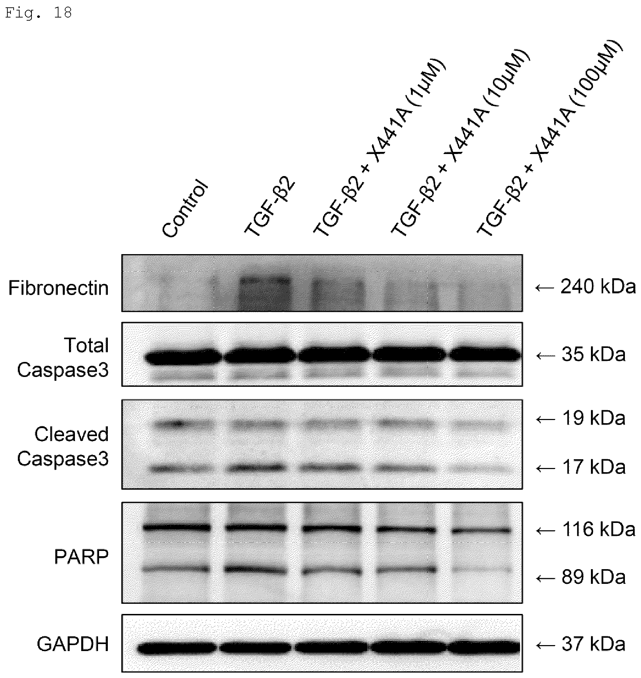

Fig. 18 shows, from the top, the results of western blotting for fibronectin, total caspase 3, cleaved caspase 3, PARP, and GAPDH. From the left lane, the results are shown for iFECD, iFECD+TGF-β2, iFECD+TGF-β2+X441A (compound 1, 1 µM), iFECD+TGF-β2+X441A (compound 1, 10 µM), and iFECD+TGF-β2+X441A (compound 1, 100 µM).

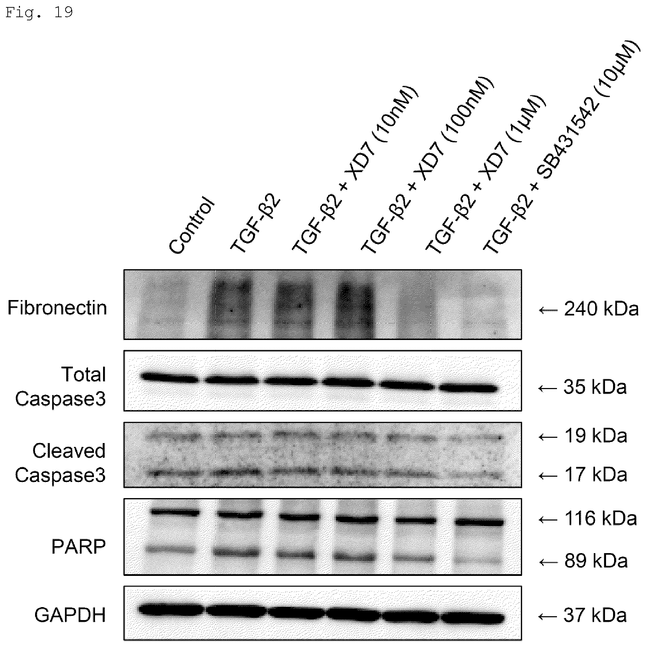

Fig. 19 shows, from the top, the results of western blotting for fibronectin, total caspase 3, cleaved caspase 3, PARP, and GAPDH. From the left lane, the results are shown for iFECD, iFECD+TGF-β2, iFECD+TGF-β2+XD7 (compound 6, 10 nM), iFECD+TGF-β2+XD7 (compound 6, 100 nM), iFECD+TGF-β2+XD7 (compound 6, 1 µM), and iFECD+TGF-β2+SB431542 (10 µM).

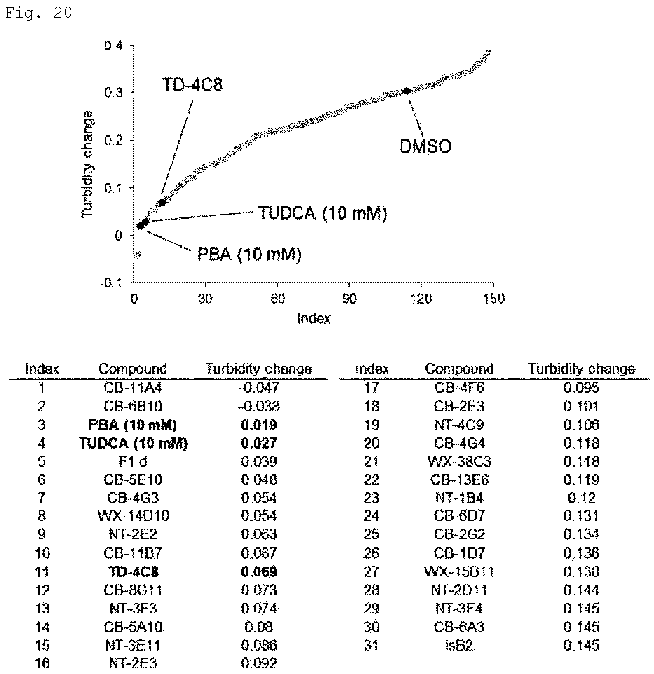

Fig. 20 shows the results of turbidity-based primary screening of 29 types of compounds. In the upper graph, the vertical axis indicates change in turbidity, and the horizontal axis indicates index. PBA and TUDCA were used at high concentrations as positive controls. The table below summarizes the individual compounds for index and their changes in turbidity.

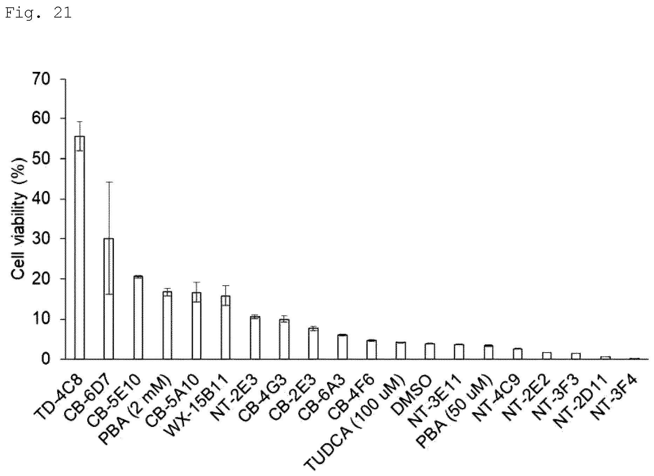

Fig. 21 shows the results of a tunicamycin assay using HEK293 cells for the second round of screening. The figure shows the results of three experiments using the top 16 compounds (50 µM). The vertical axis of the graph represents cell viability (unit: %). Error bars represent s.d. (n=3).

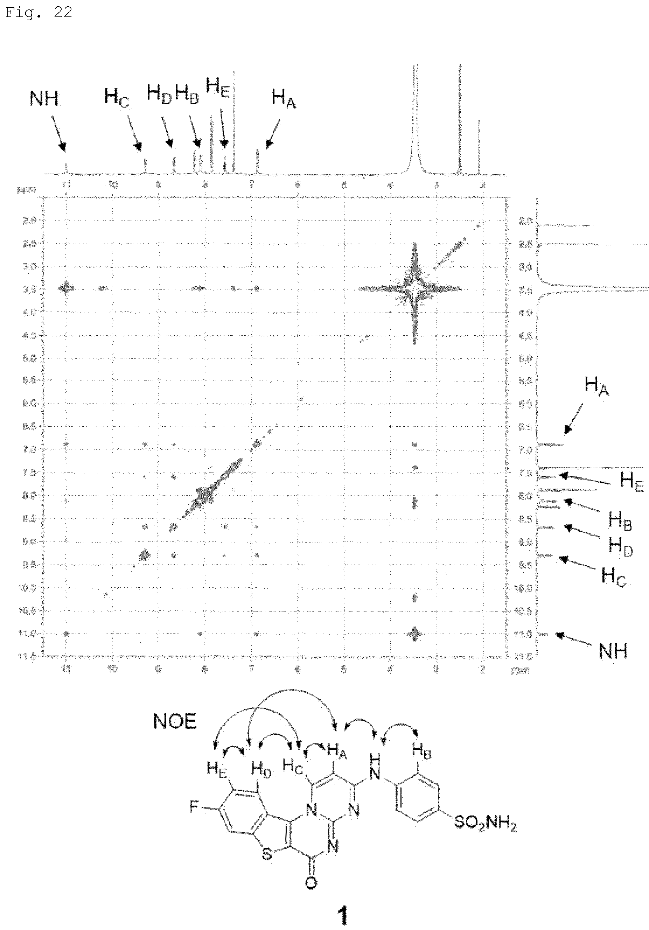

Fig. 22 shows a ¹H-¹H NOESY NMR spectrum of compound 1. ¹H pairs with a significant NOE cross signal are indicated by arrows.

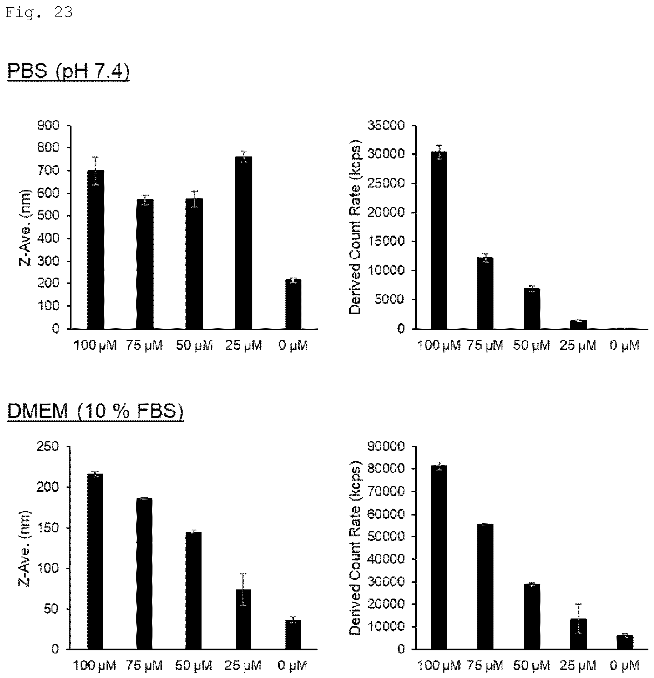

Fig. 23 shows dynamic light scattering of molecules of compound 1 in PBS (pH of 7.4) and DMEM (10% FBS). In the graphs on the left, the vertical axis "Z-ave. (nm)" represents the average particle size. In the graphs on the right, the vertical axis "Derived Count Rate (kcps)" represents the derived count rate. The horizontal axis represents the molar concentration (µM) of PBS (pH of 7.4) and DMEM (10% FBS). Error bars represent s.d. (n=3).

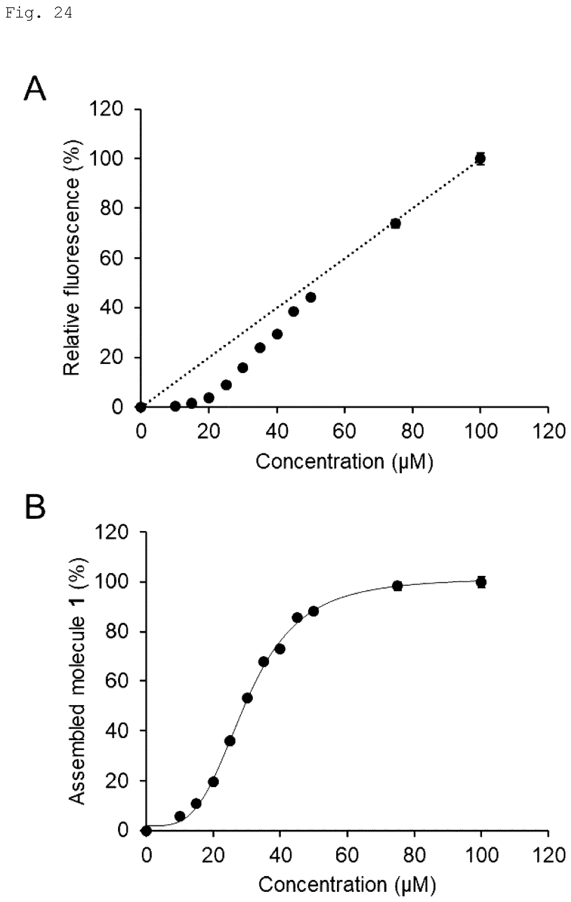

Fig. 24 shows the quantification of self-organaization of compound 1 in cell culture medium. (A) Compound 1 (10 to 100 µM) was dissolved in 100 µL of DMEM (10% FBS, 1% DMSO) containing 1.0 µM DCVJ, which is an environmentally sensitive dye. Fluorescence (example: 365 nm, em: 530 nm) was measured with a microplate reader to quantify the relative amount of self-organaization 1. The dashed line represents the theoretical fluorescence signal expected if compound 1 were fully assembled at each concentration. (B) Normalized dose-structure curve of compound 1 in DMEM. The ratio of assemblies to total molecules 1 at each concentration was estimated by normalizing the observed fluorescence signal with the theoretical signal in (A). The saturation level of the dose-assembly curve was assumed to be 100% assembled. Fitting with a sigmoid curve gave an apparent maximal assembly concentration (AC50) of compound 1 of 29.7 µM. Error bars represent s.d. (n=4).

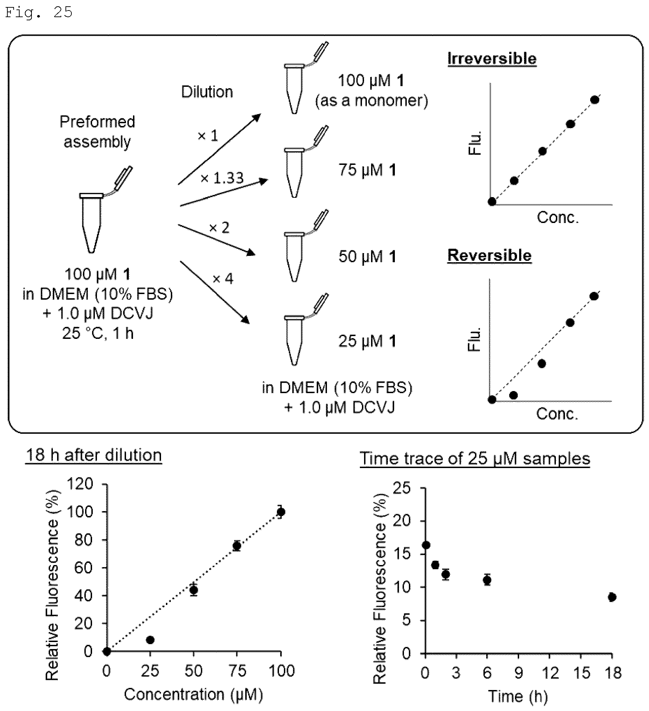

Fig. 25 shows the reversibility of self-assembly of compound 1. Compound 1 (100 µM) was incubated in DMEM (10% FBS, 0.5% DMSO) at 25°C for 1 hour to allow it to form self-assemblies. A solution of pre-formed assemblies of compound 1 was diluted in various ratios with DMEM (10% FBS, 0.5% DMSO). All samples contained 1.0 µM DCVJ, an environmentally sensitive dye, to quantify the amount of self-assembly of compound 1 by fluorescence. If the self-assembly is irreversible (upper graph in the frame), the fluorescence signal is linear with respect to the amount of the compound. In contrast, reversible self-assembly (lower graph in the frame) exhibits fluorescence lower than the linear level at low concentrations due to disassembly of preformed particles. In the two graphs within the frame, the vertical axis represents fluorescence, and the horizontal axis represents concentration. The graph on the left outside the frame shows the results after 18 hours from dilution, with the vertical axis representing relative fluorescence (unit: %) and the horizontal axis representing concentration (unit: µM). The graph on the right outside the frame shows the results of time trace of samples diluted to 25 µM, with the vertical axis representing relative fluorescence (unit: %) and the horizontal axis representing elapsed time (unit: h). The self-assembly of compound 1 showed a significant decrease in fluorescence upon dilution to 25 µM (as a monomer of compound 1), indicating that the assembly process is reversible. A time-dependent decrease in fluorescence at 25 µM (as a monomer of compound 1) was also observed. Error bars represent s.d. (n=3).

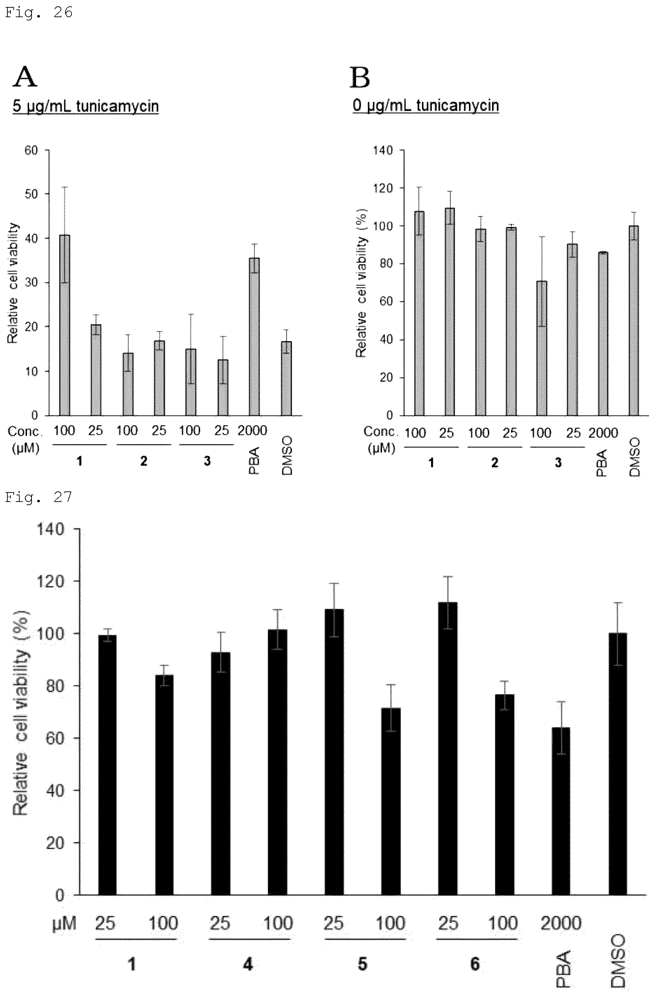

Fig. 26 shows the results of a tunicamycin assay using compound 1, compound 2, compound 3, PBA, and DMSO. HEK293 cells were treated with compound 1, compound 2, or compound 3 for 16 hours, and then with tunicamycin (0 µg/mL or 5 µg/mL) for 48 hours. Fig. 26A is a graph showing the changes in cell viability caused by compound 1 and its derivatives, compounds 2 to 6, under ER stress, as a result of treatment with 5 µg/mL tunicamycin. Fig. 26B is a graph showing the results of treatment with 0 µg/mL tunicamycin (i.e., no tunicamycin). In Figs. 26A and 26B, the vertical axis represents relative cell viability (unit: %), and the horizontal axis represents concentration (unit: µM). Cell viability was measured using a WST-8 cell counting kit (Dojindo). Error bars represent s.d. (n=3).
Fig. 27 is a graph showing the results of a cell viability assay using compound 1, compound 4, compound 5, compound 6, PBA, and DMSO. HEK293 cells were treated with compound 1, compound 4, compound 5, or compound 6 (25 µM or 100 µM, respectively) for 64 hours. The vertical axis represents relative cell viability (unit: %), and the horizontal axis represents concentration (unit: µM). Cell viability was measured using a WST-8 cell counting kit. Error bars represent s.d. (n=3).

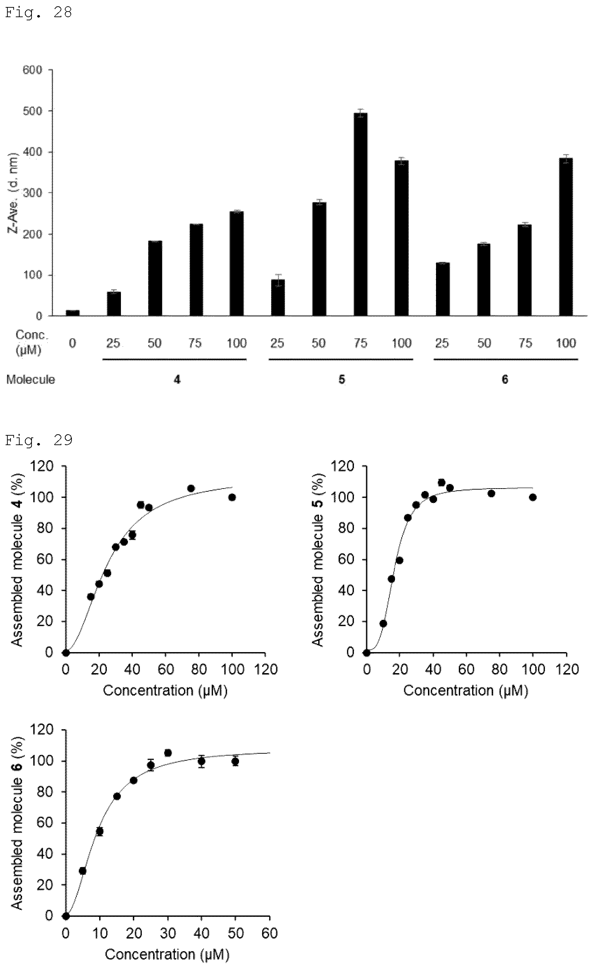

Fig. 28 shows the dynamic light scattering of compound 4, compound 5, and compound 6 in DMEM (10% FBS). "Z-ave." represents the average size of particles. Error bars represent s.d. (n=3).
Fig. 29 is a graph showing a normalized dose-assembly curve for compound 4, compound 5, and compound 6 in a cell culture medium. The compounds of various concentrations were dissolved in 100 µL of DMEM containing 1.0 µM DCVJ (10% FBS, 1% DMSO). To quantify the relative amount of self-assembled molecules, fluorescence (example: 365 nm, em: 530 nm) was measured with a microplate reader. The ratio of assemblies to total molecules at each concentration was estimated according to the method in Fig. 23. The saturation level of the dose-assembly curve was assumed to be 100% assembled. Fitting with a sigmoid curve gave an apparent maximal assembly concentration (AC50) of 25.1 µM, 16.9 µM, and 9.30 µM for the molecules of compound 4, compound 5, and compound 6, repsectively. Error bars represent s.d. (n=4).

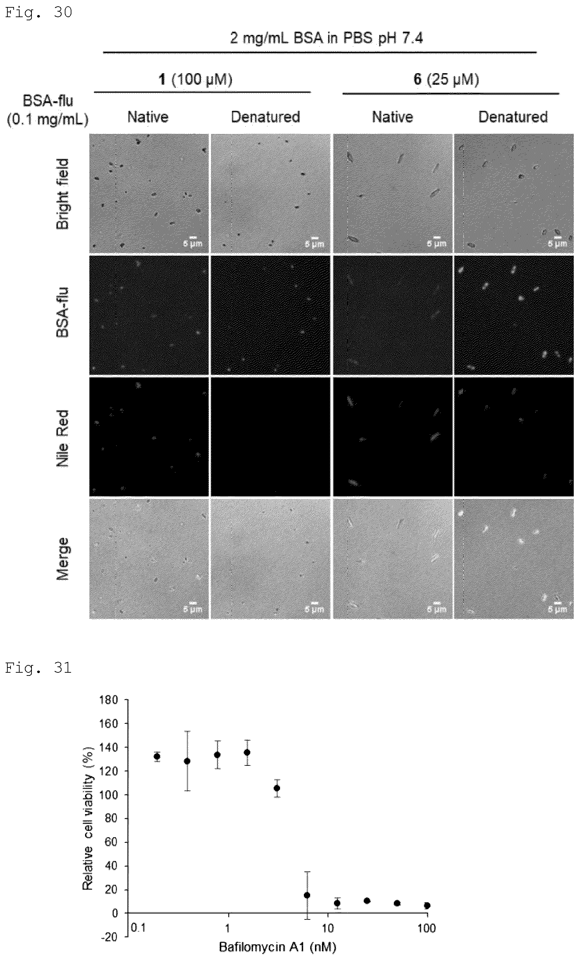

Fig. 30 shows photographs of separated fluorescence images and bright-field images from Fig. 7. Fig. 30 shows the formation of a co-assembly between a self-assembling chemical (compound 1 or 6) and a fluorescently labeled BSA (BSA-flu), with the two columns on the left showing the results for compound 1 and the two columns on the right showing the results for compound 6. "Native" denotes undenatured BSA-flu, and "Denatured" denotes denatured BSA-flu.
Fig. 31 is a graph showing the results of a cell viability assay using bafilomycin A1. HEK293 cells were treated with bafilomycin A1 in DMEM (10% FBS) for 48 hours. The vertical axis represents relative cell viability (unit: %), and the horizontal axis represents the concentration of bafilomycin A1 (unit: µM). Cell viability was measured using a WST-8 cell counting kit. Error bars represent s.d. (n=3).

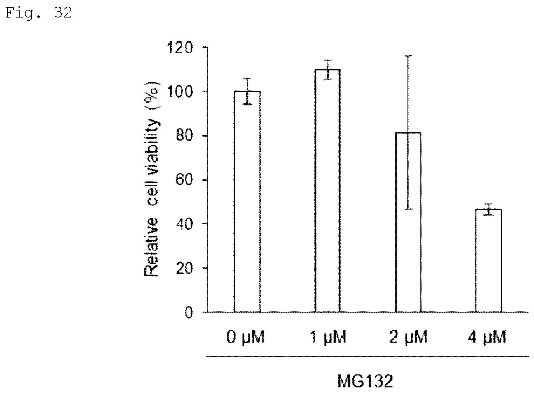

Fig. 32 is a graph showing the results of a cell viability assay using MG132. HEK293 cells were treated with MG132 in DMEM (10% FBS) for 48 hours. The vertical axis represents relative cell viability (unit: %), and the horizontal axis represents the concentration of MG132 (unit: µM). Cell viability was measured using a WST-8 cell counting kit. Error bars represent s.d. (n=3).

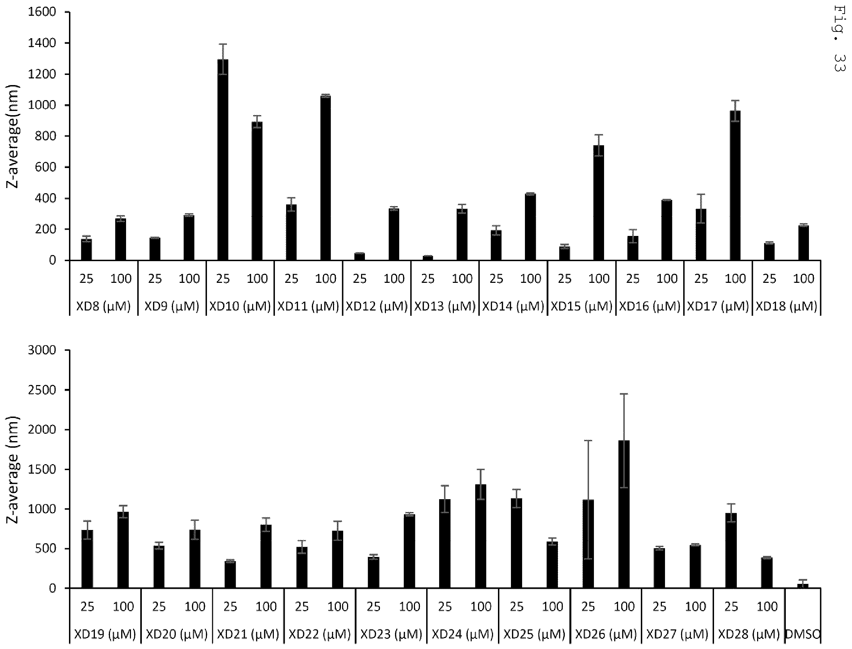

Fig. 33 shows dynamic light scattering of molecules of compounds XD8 to XD28 in PBS (pH of 7.4) and DMEM (10% FBS), illustrating the self-assembly activity of compounds XD8 to XD28. "Z-average" represents the average size of particles. Error bars represent s.d. (n=3).

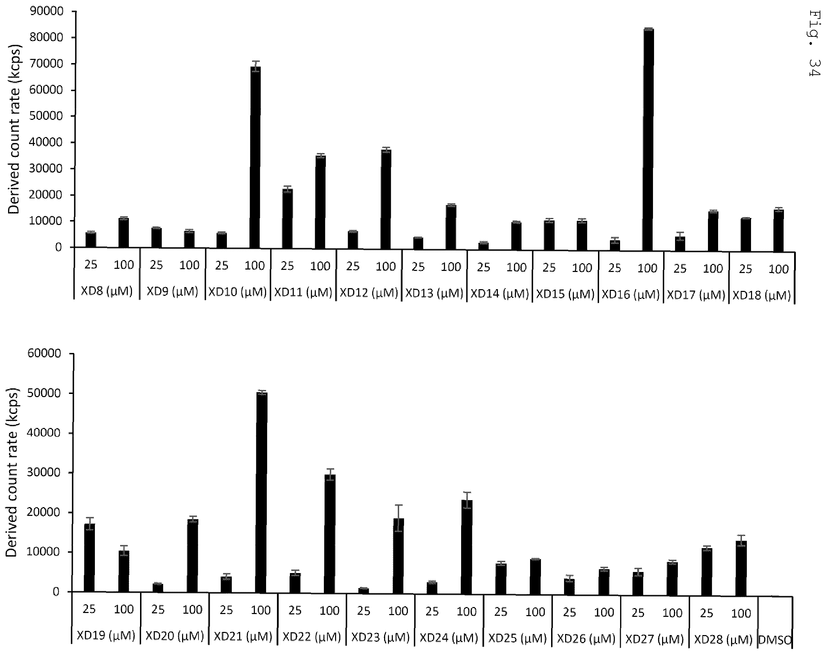

Fig. 34 shows dynamic light scattering of molecules of compounds XD8 to XD28 in PBS (pH of 7.4) and DMEM (10% FBS), illustrating the self-assembly activity of compounds XD8 to XD28. "Derived count rate (kcps)" represents the derived count rate. Error bars represent s.d. (n=3).

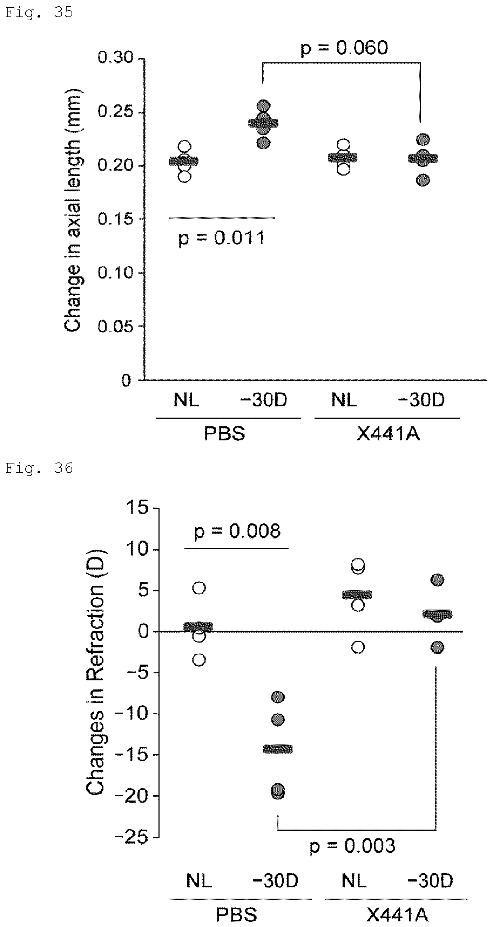

Fig. 35 is a graph showing the results of measuring the change in axial length of eyes in order to examine the inhibitory effect of ophthalmic solution X441A on the progression of myopia. A -30 diopter (unit of refractive index: D) lens (-30D) was attached to the right eye of 3-week-old male C57BL6J mice, and only a lens frame (NL) was attached to the left eye, which served as a control eye. The lenses were worn for three weeks, during which X441A was instilled into the eyes once a day (group administered with X441A), and PBS was instilled into the eyes of the control group (group administered with PBS). The amount of change in axial length of eyes was compared between the group administered with X441A and the group administered with PBS before and after the period of 3-week lens attachment and eye-drop administration. The vertical axis of the graph represents the change in axial length of eyes (unit: mm), and p represents the realized value of probability.
Fig. 36 is a graph showing the results of measuring the change in refractive index in order to examine the inhibitory effect of ophthalmic solution X441A on the progression of myopia. A -30 diopter (unit of refractive index: D) lens (-30D) was attached to the right eye of 3-week-old male C57BL6J mice, and only a lens frame (NL) was attached to the left eye, which served as a control eye. The lenses were worn for three weeks, during which X441A was instilled into the eyes once a day (group administered with X441A), and PBS was instilled into the eyes of the control group (group administered with PBS). The change in refractive index was compared between the group administered with X441A and the group administered with PBS before and after the period of 3-week lens attachment and eye drop administration. The vertical axis of the graph represents the change in refractive index (unit: D), and p represents the realized value of probability.

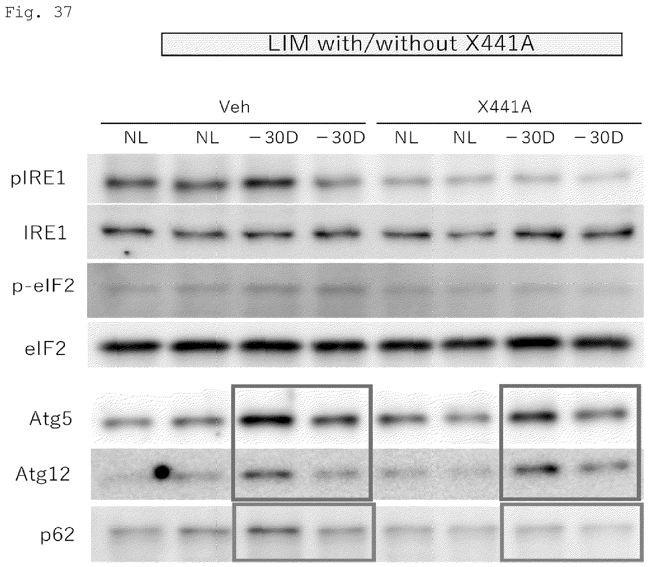

Fig. 37 shows the results of analyzing the expression levels of p62, Atg5, and Atg12 in the group administered with X441A and the group administered with PBS, obtained by enucleating eyeballs from mice after 3 weeks of myopia induction and eye-drop administration and isolating their sclera, followed by western blotting. For 3-week myopia induction, a -30 diopter (unit of refractive index: D) lens (-30D) was attached to the right eye of 3-week-old male C57BL6J mice, and only a lens frame (NL) was attached to the left eye, which served as a control eye. The four columns on the left under "Veh" show the results of the group that received PBS eye drops for 3 weeks, and the four columns on the right under "X441A" show the results of the group that received X441A eye drops for 3 weeks.

### Description of Embodiments

The present disclosure is described in more detail below with reference to examples. However, the following descriptions are only for illustration purposes and the present disclosure is not limited by the following descriptions.

In the present disclosure, when a compound (for example, a compound represented by formula (I) or a compound represented by formula (II)) exists as an isomer, such as a tautomer or a stereoisomer (e.g., geometric isomers, conformational isomers, and optical isomers), any isomer can be used in the present disclosure, unless otherwise specified. When the compound can form a salt, the salt can also be used in the present disclosure, unless otherwise specified. The salt may be an acid addition salt or a base addition salt. Further, the acid that forms such an acid addition salt may be an inorganic acid or an organic acid, and the base that forms such a base addition salt may be an inorganic base or an organic base. Examples of the inorganic acid include, but are not limited to, sulfuric acid, phosphoric acid, hydrofluoric acid, hydrochloric acid, hydrobromic acid, hydroiodic acid, hypofluorite acid, hypochlorous acid, hypobromite acid, hypoiodite acid, fluorite acid, chlorous acid, bromite acid, iodite acid, fluoric acid, chloric acid, bromic acid, iodic acid, perfluoric acid, perchloric acid, perbromic acid, and periodic acid. Examples of the organic acid include, but are not limited, to p-toluenesulfonic acid, methanesulfonic acid, oxalic acid, p-bromobenzenesulfonic acid, carbonic acid, succinic acid, citric acid, benzoic acid, and acetic acid. Examples of the inorganic base include, but are not limited to, ammonium hydroxide, alkali metal hydroxides, alkaline earth metal hydroxides, carbonates, and hydrogen carbonates, and more specifically include sodium hydroxide, potassium hydroxide, potassium carbonate, sodium carbonate, sodium hydrogen carbonate, potassium hydrogen carbonate, calcium hydroxide, and calcium carbonate. Examples of the organic base include, but are not limited to, ethanolamine, triethylamine, and tris(hydroxymethyl)aminomethane. The method for producing these salts is also not particularly limited, and such salts can be produced by a method comprising, for example, appropriately adding an acid or a base, such as those mentioned above, to the above compound by a known method.

In the present disclosure, unless otherwise specified, the chain substituent (e.g., a hydrocarbon group, such as an alkyl group or an unsaturated aliphatic hydrocarbon group) may be linear or branched, and the number of carbon atoms in the chain substituent is not particularly limited. The chain substituent may contain, for example, 1 to 40 carbon atoms, 1 to 32 carbon atoms, 1 to 24 carbon atoms, 1 to 18 carbon atoms, 1 to 12 carbon atoms, 1 to 6 carbon atoms, or 1 to 2 carbon atoms (2 or more carbon atoms when the chain substituent is an unsaturated hydrocarbon group). In the present disclosure, the number of ring members (the number of atoms that constitute the ring) of a cyclic group (e.g., an aryl group or a heteroaryl group) is not particularly limited, and can be, for example, 5 to 32, 5 to 24, 6 to 18, 6 to 12, or 6 to 10. Further, when the substituent or the like can exist as isomers, any isomer can be used, unless otherwise specified. For example, a "naphthyl group" can mean a 1-naphthyl group or a 2-naphthyl group.

In the compound of the present disclosure, X in the chemical formula (I) may be, for example, a hydrogen atom, a nonaromatic hydrocarbon group (that may be linear or branched, and saturated or unsaturated, and optionally contain a cyclic structure), an aromatic group (e.g., an aromatic group containing no heteroatoms (an aryl group), a heteroaromatic group containing a heteroatom (a heteroaryl group), and may be a single ring or a condensed ring), a halogen, an amino group, a nitro group, a sulfo group, or a cyano group, and at least one hydrogen atom of each substituent is optionally further substituted with any other substituent.

In the compound of the present disclosure, Z in chemical formula (I) may be, for example, a hydrogen atom, a nonaromatic hydrocarbon group (that may be linear or branched, and saturated or unsaturated, and optionally contains a cyclic structure), an aromatic group (an aromatic group containing no heteroatoms (an aryl group), a heteroaromatic group containing a heteroatom (a heteroaryl group), and may be a single ring or a condensed ring), a halogen, an amino group, a nitro group, a sulfo group, or a cyano group, and at least one hydrogen atom of each substituent may be optionally further substituted with any other substituent.

The present inventors developed self-assembling compounds that can directly target denatured proteins, and found that at least some of these compounds reduce the toxicity of denatured proteins to cells and endoplasmic reticulum stress caused by denatured proteins.

The compound of the present disclosure may not necessarily have endoplasmic reticulum stress suppression activity, but preferably has endoplasmic reticulum stress suppression activity. Since the compound of the present disclosure has endoplasmic reticulum stress suppression activity, the compound can be used, for example, as an endoplasmic reticulum stress inhibitor or as an active ingredient of an endoplasmic reticulum stress inhibitor. According to the present disclosure, it is possible to provide, for example, an endoplasmic reticulum stress inhibitor that is more active than existing drugs.

The compound of the present disclosure may be, for example, a self-assembling compound that co-assembles with a denatured protein and that inhibits endoplasmic reticulum stress. Denatured proteins are important drug targets that relate to age-related diseases, such as neurodegenerative diseases, metabolic diseases, and ophthalmic diseases; however, there have been no existing pharmaceutical agents that can target denatured proteins. By utilizing self-assembling compounds that are usually less likely to be considered as candidate compounds for drug discovery, the present disclosure succeeded in developing a series of small bioactive molecules that directly target denatured proteins. Denatured proteins that have co-assembled with the compound of the present disclosure have the possibility of being decomposed or removed, for example, through the cellular autophagy pathway. The compound of the present disclosure can be useful, for example, as a research tool for endoplasmic reticulum stress research and as a drug discovery lead compound for diseases derived from protein toxicity. Further, for example, as described in the Examples described below, experiments using cells derived from a patient with Fuchs' endothelial corneal dystrophy showed that cell death due to endoplasmic reticulum stress can be inhibited by the compound of the present disclosure.

Among the compounds of the present disclosure, compounds that particularly strongly inhibit endoplasmic reticulum stress include, but are not limited to, for example, compound 1 and compound 6 described below. The compound of the present disclosure can find applications, for example, as a medicament for treating or preventing an eye condition, disorder, or disease, or as an active ingredient of such a medicament. However, the application of the compound of the present disclosure is not limited thereto. The compound can be used for any purpose.

Visual information is recognized by the following mechanism. Light that enters through the cornea, which is transparent tissue on the anterior-most surface of the eyeball, reaches the retina and excites the retinal nerve cells, and the electrical signals generated are transmitted via the optic nerve to the visual cortex of the cerebrum. In order to obtain good vision, the cornea must be clear. The transparency of the cornea is maintained by keeping the water content constant by the pumping function and barrier function of the corneal endothelial cells.

Human corneal endothelial cells are present at a density of about 3000 cells per 1 mm² at birth. However, once such corneal endothelial cells are damaged, the ability to regenerate the cells is very limited. Fuchs' endothelial corneal dystrophy is a disease that causes an abnormality in endothelial cells inside the cornea, resulting in corneal edema. The cause of this disease is unknown. In Fuchs' endothelial corneal dystrophy, the extracellular matrix, such as collagen, is deposited in part of the back surface of Descemet's membrane at the back of the cornea, resulting in corneal guttae and Descemet's membrane thickening. Corneal guttae and Descemet's membrane thickening are causes of glare or blurred vision in patients with Fuchs' endothelial corneal dystrophy, which compromises the QOL of the patients. It is understood that there is no effective therapeutic method other than corneal transplantation for Fuchs' endothelial corneal dystrophy. However, there is a shortage in cornea donation in Japan, where the number of patients waiting for corneal transplantation is about 2600, whereas the number of corneal transplantation performed in Japan is about 1700 annually.

Fuchs' endothelial corneal dystrophy is a disease that causes corneal stromal edema etc. due to overproduction of the extracellular matrix and death of corneal endothelial cells. At present, there is no therapeutic drug in clinical use, and the only treatment available is corneal transplantation.

The present inventors found that the suppression of endoplasmic reticulum stress inhibits cell death (apoptosis) of the eye, in particular, corneal endothelial cells. Further, the present inventors found that the compound of the present disclosure can be used for treating or preventing an ophthalmic disease, such as corneal endothelial disorder caused by transforming growth factor-β (TGF-β) (in particular, corneal endothelial disorder in Fuchs' endothelial corneal dystrophy). Further, the present inventors found that the suppression of endoplasmic reticulum stress inhibits overexpression of extracellular matrix proteins, such as fibronectin. As a result, the present inventors found that, for example, the compound of the present disclosure, which has endoplasmic reticulum stress suppression activity, is applicable to improve, treat, or prevent corneal endothelial disorders (e.g., guttae, Descemet's membrane thickening, opacity symptoms, such as corneal opacity, and leukoplakia) due to overexpression of the extracellular matrix. Since cell death and extracellular matrix overexpression are independent events, being able to inhibit both cell death and extracellular matrix overexpression is preferable.

One or more features of the present disclosure can be provided, for example, in further combinations, in addition to the explicitly stated combinations. A person skilled in the art would recognize further embodiments and advantages of the present disclosure upon reading and understanding the following detailed description, if necessary.

According to the present disclosure, for example, a novel medicament capable of treating or preventing conditions, disorders, or diseases of the corneal endothelium due to a transforming growth factor-β (TGF-β) signal can be provided.

Myopia is a type of refractive error, which is a condition in which the focus is formed in front of the retina and correction is required with glasses or contact lenses in order to focus on the retina. As the human eyeball grows larger after birth, the cornea becomes flatter and the crystalline lens of the eye becomes thinner, thus decreasing the refractive power. On the other hand, the axial length of eyes increases to 20 mm at 1 year of age, 22 mm by 6 years of age, and about 24 mm in adults, while refraction attempts to maintain correct vision. Axial myopia, which generally progresses during school ages of between 9 and 15, is a condition in which the equilibrium of the normal viewing state is broken and axial length of eyes further elongates. Most myopia is caused by axial myopia. There is also refractive myopia, which progresses in middle age due to metabolic disorders, such as diabetes and changes in the crystalline lens of the eye due to aging. It is known that if myopia progresses excessively, strain is placed on the retina and optic nerve, and the risk of various eye diseases, such as retinal detachment, glaucoma, and macular degeneration, as well as the associated visual impairment, increases.

According to the present disclosure, for example, a novel medicament capable of treating or preventing a myopic condition, disorder, or disease can be provided.

In the present disclosure, unless otherwise defined, technical terms, scientific and technical terminology, etc., have the same meaning as those commonly understood by a person skilled in the art to which the present disclosure belongs. When the meaning of a term is defined, the meaning of the term follows the definition.

### Definition

In the present disclosure, the term "about" or "approximately" written before a numerical value means, for example, that the value is within the range of ±10% of the numerical value after the word "about" or "approximately," although this is not limitative.

In the present disclosure, "endoplasmic reticulum stress inhibitor" refers to any pharmaceutical agent that co-assembles with denatured proteins and directly inhibits their activity (toxicity).

### Definition

In the present disclosure, "iFECD" (immobilized Fuchs' endothelial corneal dystrophy) is an abbreviation for immortalized Fuchs' endothelial corneal dystrophy cells.

In the present disclosure, "HCEC" (human corneal endothelial cells) is an abbreviation for human corneal endothelial cells. "iHCEC" is an abbreviation for immortalized (immobilized) human corneal endothelial cells.

In the present disclosure, "transforming growth factor-β" (also denoted with the abbreviation "TGF-β") is used in the same meaning as used in the art. It is a homodimer multifunctional cytokine with a molecular weight of 25 kD that exhibits a variety of biological activity, such as being responsible for pathogenesis of various sclerotic diseases, rheumatoid arthritis, and proliferative vitreoretinopathy, being deeply involved in hair loss, suppressing the functioning of immunocompetent cells while suppressing overproduction of protease to prevent degradation of pulmonary tissue resulting in pulmonary emphysema, and suppressing cancer cell growth. In humans, TGF-β has three isoforms: TGF-β1 to TGF-β3. TGF-β is produced as an inactive latent form with a molecular weight of about 300 kD that is unable to bind to a receptor. TGF-β exerts its action by being activated on a target cell surface or in the surroundings to become an active form capable of binding to a receptor.

The action of TGF-β in a target cell is understood to be transmitted by a phosphorylation channel of a series of proteins responsible for transmitting information called "Smad." However, this theory in no way limits the present disclosure. According to this theory, first, when activated TGF-β binds to a TGF-β type II receptor on a target cell surface, a receptor complex consisting of two molecules of type II receptors and two molecules of TGF-β type I receptors is formed, and the type II receptors phosphorylate the type I receptors. Subsequently, when the phosphorylated type I receptors phosphorylate Smad2 or Smad3, the phosphorylated Smad2 and Smad3 forms a complex with Smad4, which migrates to a nucleus and binds to a target sequence called "CAGA box," which is present in a target gene promotor region, to induce transcription and expression of a target gene with a coactivator.

A transforming growth factor-β (TGF-β) signaling pathway can modulate many cellular activities, such as cell growth and differentiation, growth arrest, apoptosis, and epithelial mesenchymal transition (EMT), by modulating the target gene. Members of the TGF-β family, including TGF-β itself (e.g., TGF-β1, TGF-β2, and TGF-β3), activin, and bone morphogenetic proteins (BMP) are potent modulators of cell growth, differentiation, migration, apoptosis, and the like.

TGF-β is a protein of about 24 Kd produced by many cells, including B lymphocytes, T lymphocytes, activated macrophages and by many other cell types. Effects of TGF-β on the immune system include IL-2 receptor induction, inhibition of IL-1 induced thymocyte growth, and blocking of IFN-γ induced macrophage activation. TGF-β is considered to be involved in various pathological conditions (Border et al. (1992) J. Clin. Invest. 90:1) and is thoroughly proven to function as either a tumor-suppressing substance or a tumor promotor.

Signaling of TGF-β is mediated by two serine/threonine kinase cell surface receptors: TGF-βRII and ALK5. TGF-β signaling is initiated by ligand induced receptor dimerization, which enables TGF-βRII to phosphorylate an ALK5 receptor. The phosphorylation activates ALK5 kinase activity, and the activated ALK5 then phosphorylates a downstream effector Smad protein (vertebrate homologue of MAD or "Mothers against DPP (decapentaplegic)" protein), Smad2 or Smad3. A p-Smad2/3 complex with Smad4 enters a nucleus and activates transcription of a target gene.

Smad3 is a member of the R-Smad (receptor-activated Smad) subgroup of Smad and a direct mediator of transcription activation by a TGF-β receptor. TGF-β stimulation results in phosphorylation and activation of Smad2 and Smad3, which form a complex with Smad4 ("common Smad" or "co-Smad" in vertebrates). This accumulates in the nucleus and modulates transcription of a target gene. R-Smad is localized in cytoplasm, forms a complex with co-Smad through ligand-induced phosphorylation by a TGF-β receptor, and migrates to the nucleus, where the complex modulates chromatin and gene expression associated with a cooperative transcription factor. Smad6 and Smad7 are inhibitory Smad ("I-Smad"); i.e., they are transcriptionally induced by TGF-β and function as a TGF-β signaling inhibitor (Feng et al. (2005) Annu. Rev. Cell. Dev. Biol. 21: 659). Smad6 and Smad7 obstruct receptor-mediated activation of R-Smad to thereby exert their inhibitory effect, and they are associated with a type I receptor, which competitively obstructs mobilization and phosphorylation of R-Smad. Smad6 and Smad7 are known to replenish E3 ubiquitin ligase, which induces ubiquitination and degradation of Smad6/7-interacting proteins.

TGF-β signaling pathways further have other pathways using BMP-7 transmission or the like, which go through ALK-1/2/3/6 via Smad1/5/8 to express a function. For TGF-β signaling pathways, see, for example, J. Massagué, Annu. Rev. Biochem., 1998, 67: 753-91; Vilar JMG, Jansen R, Sander C (2006) PLoS Comput Biol 2 (1): e3; Leask, A., Abraham, D.J. FASEB J. 18, 816-827 (2004 ); Coert Margadant & Arnoud Sonnenberg EMBO reports (2010) 11, 97-105; and Joel Rosenbloom et al., Ann Intern Med. 2010; 152: 159-166.

In the present disclosure, "corneal endothelial condition, disorder, or disease due to transforming growth factor-β (TGF-β)" refers to any corneal endothelial condition, disorder, or disease induced by TGF-β in corneal endothelial cells. The exposure of corneal endothelial cells, such as model cells of Fuchs' endothelial corneal dystrophy (e.g., iFECD), to TGF-β2 surprisingly resulted in various disorders, for example, as shown in the examples below. Such a phenomenon is a phenomenon that had not been elucidated yet. The present inventors conducted further analysis of the corneal endothelial condition, disorder, or disease due to a TGF-β signal, and unexpectedly found that such disorders can be inhibited by the compound of the present disclosure (e.g., compound 1 and compound 6 described in the Examples below) that is an endoplasmic reticulum stress inhibitor. The corneal endothelial condition, disorder, or disease due to a TGF-β signal is different from those of mTOR signaling pathways. Examples of the corneal endothelial condition, disorder, or disease due to a TGF-β signal include, but are not limited to, Fuchs' endothelial corneal dystrophy, post-corneal transplant disorder, corneal endotheliitis, trauma, post-ophthalmic surgery disorder, post-ophthalmic laser surgery disorder, aging, posterior polymorphous dystrophy (PPD), congenital hereditary endothelial dystrophy (CHED), idiopathic corneal endothelial disorder, cytomegalovirus corneal endotheliitis, and the like with observed TGF-β expression. Since the disorder found in the present disclosure or an associated disorder is considered to be expressed or advanced especially in corneal endothelial cells or corneal endothelial tissue with TGF-β2 expression higher than normal, any corneal endothelial condition, disorder, or disease in which such corneal endothelial cells or corneal endothelial tissue are observed is especially intended as the target of the present disclosure.

In the present disclosure, "overexpression of the extracellular matrix (ECM) in corneal endothelial cells" refers to expression of the extracellular matrix at an abnormal level compared to extracellular matrix expression levels in normal corneal endothelial cells. "Expression of the extracellular matrix at an abnormal level" means that an extracellular matrix protein, such as fibronectin, is produced in an amount greater than the amount produced in the extracellular matrix in a normal form. The production status includes production with no stimulation, as well as an increased level of expression due to a response to transforming growth factor (TGF) β as needed. For example, the expression level in human corneal endothelial cells can be about 1.1 fold or greater, about 1.2 fold or greater, about 1.3 fold or greater, about 1.4 fold or greater, about 1.5 fold or greater, about 1.6 fold or greater, about 1.7 fold or greater, about 1.8 fold or greater, about 1.9 fold or greater, or about 2.0 fold or greater than the amount of the extracellular matrix produced in normal cells. The difference relative to normal is preferably, but not necessarily, statistically significant. It is sufficient as long as the difference is a medically significant difference.

In the present disclosure, "corneal endothelial disorder due to overexpression of the extracellular matrix (ECM)" or a "condition" thereof is mainly a disorder or a condition associated with clouding, deposition, thickening, or the like due to the extracellular matrix, which causes guttata on the corneal endothelium surface, Descemet's membrane thickening, such as turbid guttae of Descemet's membrane, or the like, and is associated with a condition that causes reduced vision. In corneal endothelial disorders, such as Fuchs' corneal dystrophy, overproduction of the extracellular matrix worsens the vision or visual sense even without a reduction in cell count, unlike exacerbation in a condition due to death (especially apoptosis) of corneal endothelial cells. Thus, even if cell death can be suppressed, this needs to be addressed. "Corneal endothelial disorder due to overproduction of the extracellular matrix (ECM)" or a "condition" thereof include, but are not limited to, opacity, scarring, corneal nebula, corneal macula, corneal leucoma, and the like.

In a preferred embodiment, the condition, disorder, or disease targeted by the present disclosure is a disorder related to Fuchs' endothelial corneal dystrophy. TGF-β induction in corneal endothelial cells is shown to be involved in Fuchs' endothelial corneal dystrophy. It is also shown that TGF-β induction can be involved in cell loss in FECDs. Therefore, inhibition of a TGF-β signaling pathway is naturally expected to be an effective therapy for FECDs. However, the present inventors unexpectedly found that compounds of the present disclosure, which have endoplasmic reticulum stress suppression activity (e.g., compounds 1 and 6 described in the Examples below), can inhibit a disorder due to a TGF-β signal.

Since the medicament of the present invention can treat cell damage or the like that is induced by TGF-β2, which can be one of the major causes of abnormalities or disorders in Fuchs' endothelial corneal dystrophy, the medicament is useful in treating or preventing Fuchs' endothelial corneal dystrophy. In particular, the present disclosure can inhibit cell damage or programmed cell death induced by TGF-β2 in a Fuchs' endothelial corneal dystrophy model as described below in the Examples. Therefore, the present disclosure is considered to be usable for treating patients with a severe TGF-β2-associated disease in a Fuchs' endothelial corneal dystrophy model. The medicament of the present invention can also, unexpectedly, suppress overexpression of the extracellular matrix (ECM), and the medicament can treat or prevent a disorder or the like in corneal endothelia, such as ECM deposition in the Descemet's membrane. Therefore, the present invention can treat or prevent damage to corneal endothelial cells in Fuchs' endothelial corneal dystrophy, decreased corneal endothelial density, guttae formation, Descemet's membrane thickening, corneal hypertropy, corneal epithelial disorder, opacity, scarring, corneal stroma opacity, photophobia, blurred vision, visual impairment, eye pain, epiphora, hyperemia, pain, bullous keratopathy, eye discomfort, diminished contrast, halo, glare, corneal stroma edema, and the like.

In the present disclosure, "myopic condition, disorder, or disease" refers to a condition, disorder, or disease related to myopia, such as refractive abnormalities, axial elongation of eyes, choroidal thinning, and reduced choroidal blood flow.

In a preferred embodiment, the condition, disorder, or disease that is a target in the present disclosure is a myopia-related disorder. In myopic scleral fibroblasts, endoplasmic reticulum stress occurs and is shown to be involved in the progression of myopia (Reference Literature 47). The present inventors found that the compound having an endoplasmic reticulum stress suppression activity (e.g., compound 1 described in the Examples below) reduces the expression level of p62, which acts as a receptor for a degraded protein. Further, the present inventors found that the compound of the present disclosure, which has endoplasmic reticulum stress suppression activity (e.g., compound 1 described in the Examples below), can suppress the progression of myopia and have the possibility of promoting autophagy flux in the sclera.

The endoplasmic reticulum stress response is mainly controlled by the activity of three pathways: the PERK pathway, the IRE1a pathway, and the ATF-6 pathway. The activation of these pathways is controlled by the ER chaperone protein BiP, which binds to and repairs misfolded proteins. Accumulation of misfolded proteins causes BiP to dissociate from PERK and ATF-6, which leads to the activation of PERK and ATF-6. Active PERK phosphorylates Thr980 and phosphorylates Ser51 in eIF2a. Most mRNAs are thereby repressed in translation, but the expression of ATF-4, an important transcription factor in this pathway, is induced by altered translation. Further, upon dissociation of BiP due to endoplasmic reticulum stress, ATF-6 migrates to the Golgi body and is activated by protease. Accumulation of misfolded proteins by BiP directly activates IRE1a. Activated IREa induces alternative splicing of XBP1 mRNA. Selectively spliced XBP1 (XBP-1s) functions as a transcription factor and induces the expression of stress response genes. These pathways induce the transcription of stress response genes, such as CHOP and GADD34. These function cooperatively to control cellular responses, such as cell death and autophagy.

Induction of macroautophagy forms an autophagosome, which is double-membrane vesicles, while partially encircling the cytoplasm. The autophagosome fuses with the lysosome to become an autolysosome, which decomposes the encircled cytoplasm. An autophagosome fuses with a lysosome to form an autolysosome, resulting in degradation of the encircled cytoplasm. The target of degradation by autophagy includes not only cytoplasmic molecules, such as proteins and RNAs, but also organelles, such as nuclei, endoplasmic reticulum, mitochondria, peroxisomes, and lipid droplets. Accordingly, autophagy results in a large influx of lipids that make up organelles into lysosomes/vacuoles. "Autophagy" is a general term for degradation pathways of intracellular components in lysosomes/vacuoles.

### General Techniques

Molecular biological approaches, biochemical approaches, and microbiological approaches used in the present disclosure can be, for example, techniques well known and conventionally used in the art. Such techniques are described, for example, in Sambrook J. et al. (1989), Molecular Cloning: A Laboratory Manual, Cold Spring Harbor, 3rd Ed. (2001); Ausubel, F.M. (1987), Current Protocols in Molecular Biology, Greene Pub. Associates and Wiley-Interscience; Ausubel, F.M. (1989), Short Protocols in Molecular Biology: A Compendium of Methods from Current Protocols in Molecular Biology, Greene Pub. Associates and Wiley-Interscience; Innis, M.A. (1990), PCR Protocols: A Guide to Methods and Applications, Academic Press; Ausubel, F.M. (1992), Short Protocols in Molecular Biology: A Compendium of Methods from Current Protocols in Molecular Biology, Greene Pub. Associates; Ausubel, F.M. (1995), Short Protocols in Molecular Biology: A Compendium of Methods from Current Protocols in Molecular Biology, Greene Pub. Associates; Innis, M.A. et al. (1995), PCR Strategies, Academic Press; Ausubel, F.M. (1999), Short Protocols in Molecular Biology: A Compendium of Methods from Current Protocols in Molecular Biology, Wiley, and annual updates; Sninsky, J.J. et al. (1999), PCR Applications: Protocols for Functional Genomics, Academic Press, Gait, M.J. (1985), Oligonucleotide Synthesis: A Practical Approach, IRL Press; Gait, M.J. (1990), Oligonucleotide Synthesis: A Practical Approach, IRL Press; Eckstein, F. (1991), Oligonucleotides and Analogues: A Practical Approach, IRL Press; Adams, R.L. et al. (1992), The Biochemistry of the Nucleic Acids, Chapman & Hall; Shabarova, Z. et al. (1994), Advanced Organic Chemistry of Nucleic Acids, Weinheim; Blackburn, G.M. et al. (1996), Nucleic Acids in Chemistry and Biology, Oxford University Press; Hermanson, G.T. (1996), Bioconjugate Techniques, Academic Press, Bessatsu Jikken Igaku [Experimental Medicine, Supplemental Volume], Idenshi Donyu Oyobi Hatsugen Kaiseki Jikken Ho [Experimental Methods for Transgenesis and Expression Analysis], Yodosha, 1997 and the like. For corneal endothelial cells, reports of Nancy Joyce et al. (Joyce 2004, #161; Joyce 2003, #7} are well known. Since long-term culture and subculture results in fibroblast-like transformation as discussed above, research is currently being conducted for efficient culturing methods. The relevant portions (which can be the entire document) of the above documents are incorporated herein by reference.

### Description of Preferred Embodiments

Preferred embodiments are described below. It should be understood that the embodiments are examples of the present disclosure, and the scope of the present disclosure is not limited to such preferred embodiments. It should also be understood that those skilled in the art could readily make alterations or modifications within the scope of the present disclosure by referring to the following preferred embodiments.

### Medicaments

In one aspect, the present disclosure provides a composition for preventing or treating an eye condition, disorder, or disease, the composition comprising the compound of the present disclosure, which has endoplasmic reticulum stress suppression activity (e.g., compound 1 or compound 6 described in the Examples below). In particular, the compound of the present disclosure, which has endoplasmic reticulum stress suppression activity, is effective against a corneal endothelial condition, disorder, or disease in corneal endothelia.

In one embodiment, a corneal endothelial condition, disorder, or disease due to transforming growth factor-β (TGF-β) in corneal endothelial cells is selected from the group consisting of Fuchs' endothelial corneal dystrophy, post-corneal transplant disorder, corneal endotheliitis, trauma, post-ophthalmic surgery disorder, post-ophthalmic laser surgery disorder, aging, posterior polymorphous corneal dystrophy (PPD: posterior polymorphous dystrophy), congenital hereditary endothelial dystrophy (CHED), idiopathic corneal endothelial disorder, and cytomegalovirus corneal endotheliitis.

In still another preferred embodiment, the present invention provides a medicament or a preventive or therapeutic method having an action and effect for treating or preventing a condition due to overexpression of the extracellular matrix in Fuchs' endothelial corneal dystrophy, the medicament or the preventive or therapeutic method being for use in treating or preventing such a condition. Examples of such a condition include guttata on a corneal endothelial surface, turbid guttae of Descemet's membrane, Descemet's membrane thickening, blurred vision, halo, glare, reduced vision, corneal opacity, leucoma, abnormality in visual sense, and the like. Conditions due to overproduction of the extracellular matrix are further discussed below.

In still another aspect, the present disclosure provides a medicament for use in treating or preventing a corneal endothelial condition, disorder, or disease due to overexpression of the extracellular matrix in corneal endothelial cells, comprising the compound of the present disclosure, which has endoplasmic reticulum stress suppression activity (e.g., compound 1 or compound 6 described in the Examples below). As discussed above, the compound of the present disclosure, which has endoplasmic reticulum stress suppression activity (e.g., compound 1 or compound 6 described in the Examples below), can treat or prevent a corneal endothelial disorder or the like due to a TGF-β signal, but it was surprising that compounds 1 and 6 can further suppress the overexpression of the extracellular matrix in corneal endothelial cells. This suggests that the compound of the present disclosure, which has endoplasmic reticulum stress suppression activity (e.g., compound 1 or compound 6 described in the Examples below), can simultaneously treat both corneal endothelial disorders due to a TGF-β signal and overexpression of the extracellular matrix in corneal endothelial cells. In particular, Fuchs' endothelial corneal dystrophy is a disease in which the density of corneal endothelial cells significantly decreases due to a TGF-β signal, and the extracellular matrix is deposited in Descemet's membrane, resulting in corneal guttae and Descemet's membrane thickening. For this reason, suppression of the overexpression of the extracellular matrix means that therapy and prophylaxis of Fuchs' endothelial corneal dystrophy can be significantly improved, and is capable of complete healing in some cases. It is also possible to improve, treat, or prevent corneal guttae and Descemet's membrane thickening, as well as other conditions associated with opacity or deposition (irreversible opacity in corneal stroma due to protracted corneal edema or the like) that can occur due to overproduction of the extracellular matrix in corneal endothelial disorders, such as Fuchs' endothelial corneal dystrophy.

In one embodiment, the corneal endothelial condition, disorder, or disease due to overexpression of the extracellular matrix in corneal endothelial cells can be caused by overexpression of fibronectin in corneal endothelial cells.

In one embodiment, the corneal endothelial condition, disorder, or disease due to overexpression of the extracellular matrix in corneal endothelial cells is selected from the group consisting of Fuchs' endothelial corneal dystrophy, guttae formation, Descemet's membrane thickening, corneal hypertropy, opacity, scarring, opacity in corneal stroma, corneal epithelial edema, corneal epithelial disorder, nubecula corneae, corneal macula, corneal leukoma, photophobia, and blurred vision.

In another aspect, the present invention provides a medicament for use in treating or preventing a corneal endothelial condition, disorder, or disease due to a TGF-β signal and overexpression of the extracellular matrix in corneal endothelial cells, the medicament comprising the compound of the present disclosure, which has endoplasmic reticulum stress suppression activity (e.g., compound 1 or compound 6 described in the Examples below). The compound of the present disclosure, which has endoplasmic reticulum stress suppression activity (e.g., compound 1 or compound 6 described in the Examples below), can simultaneously treat or prevent corneal endothelial disorders due to a TGF-β signal and overexpression of the extracellular matrix in corneal endothelial cells.

In one embodiment, the corneal epithelial condition, disorder, or disease due to a TGF-β signal and overexpression of the extracellular matrix in corneal epithelial cells is selected from the group consisting of Fuchs' endothelial corneal dystrophy, other corneal endothelial dystrophies, and corneal epithelial disorders due to drugs, surgery, trauma, infections, uveitis, or the like.

In one embodiment, the corneal endothelial condition, disorder, or disease due to a TGF-β signal and overexpression of the extracellular matrix in corneal endothelial cells includes Fuchs' endothelial corneal dystrophy. Fuchs' endothelial corneal dystrophy is a disease in which the density of corneal endothelial cells significantly decreases due to a TGF-β signal and the extracellular matrix is deposited in Descemet's membrane, resulting in a disorder, such as corneal guttae and Descemet's membrane thickening. For this reason, suppression of the overexpression of the extracellular matrix means that therapy can significantly improve Fuchs' endothelial corneal dystrophy, and may achieve complete healing in some cases. Improvement of a disorder or the like, such as corneal guttae and Descemet's membrane thickening in a disease such as Fuchs' endothelial corneal dystrophy by using compounds 1 and 6, which are endoplasmic reticulum stress inhibitors, brings about qualitative improvement in an ophthalmic disease and provides an unprecedented therapeutic effect against a disease such as Fuchs' endothelial corneal dystrophy for which the patient previously had no other option but to await death.

In one embodiment, examples of use methods of the present disclosure include, but are not limited to, eye drops, as well as administration methods, such as injection into the anterior chamber, impregnation into a controlled-release agent, subconjunctival injection, and systemic administration (oral administration and intravenous injection).

In the medicament of the present disclosure, the compound of the present disclosure (e.g., compound 1 or compound 6 described in the Examples below) may be used alone or in combination. The compound of the present disclosure may be, for example, but is not limited to, the compound of the present disclosure having endoplasmic reticulum stress suppression activity (i.e., endoplasmic reticulum stress inhibitor), but are not limited thereto.

The concentration of the compound of the present disclosure used in the present disclosure (e.g., compound 1) can be, for example, but is not limited to, at least about 1 µM (nmol/L).

The upper limit of the concentration of the compound of the present disclosure used in the present disclosure (e.g., the compound of the present disclosure, which is an endoplasmic reticulum stress inhibitor) includes, but is not limited to, for example, about 100 µM (µmol/L). Examples of the concentration range of the compound of the present disclosure used in the present disclosure (e.g., compound 1) include, but are not limited to, about 1 µM to about 100 µM, about 10 µM to about 100 µM, and about 1 µM to about 10 µM.

The concentration of the compound of the present disclosure used in the present disclosure (e.g., compound 6) can be, but is not limited to, for example, at least about 10 nM (nmol/L).

The upper limit of the compounds of the present disclosure used in the present disclosure (e.g., the compound of the present disclosure, which is an endoplasmic reticulum stress inhibitor) is, for example, but is not limited to, about 1 µM (µmol/L). The concentration range of the compounds of the present disclosure (e.g., compound 6) include, for example, but are not limited to, about 10 nM to about 1 µM, about 100 nM to about 1 µM, and about 10 nM to about 100 nM.

In the present disclosure, for example, when two or more compounds of the present disclosure (e.g., compounds of the present disclosure that are endoplasmic reticulum stress inhibitors) are used in combination, the concentration of each compound of the present disclosure can be appropriately changed.

When used as eye drops, the formulation concentration can be determined using about 1 to 10000-fold, preferably about 100 to 10000-fold, for example, about 1000-fold of the above effective concentration as a reference while considering dilution with tear fluid or the like and paying attention to toxicity. It is also possible to set a higher concentration.

The concentration of the compound used in the present disclosure (e.g., compound 1) includes, but is not limited to, for example, about 1 mM to about 100 mM, about 10 mM to about 100 mM, or about 1 mM to about 10 mM. The upper limit and the lower limit can be appropriately set in combination, and when two or more compounds are used in combination, the concentration of each compound can be appropriately changed.

The concentration of the compound used in the present disclosure (e.g., compound 6) includes, but is not limited to, for example, about 10 µM to about 1 mM, about 100 µM to about 1 mM, and about 10 µM to about 100 µM. The upper limits and lower limits can be appropriately set in combination, and when two or more compounds are used in combination, the concentration of each compound can be appropriately changed.

In one embodiment, the therapeutic or prophylactic medicament of the present disclosure can target any animal with a corneal endothelium, such as mammals. Such a medicament is preferably intended for treating or preventing primate corneal endothelium. The subject of therapy or prophylaxis is preferably human corneal endothelium.

### Preservation and Growth of Corneal Endothelial Cells

In another aspect, the present disclosure provides a composition for preserving corneal endothelial cells, comprising the compound of the present disclosure, which has endoplasmic reticulum stress suppression activity (e.g., compound 1 or compound 6). The present invention also provides a method for preserving corneal endothelial cells, the method comprising bringing an effective amount of the compound of the present disclosure, which has endoplasmic reticulum stress suppression activity (e.g., compound 1 or compound 6), into contact with corneal endothelial cells. It is understood that an embodiment of the endoplasmic reticulum stress inhibitor or the like used for preservation of corneal endothelial cells can use any embodiment disclosed in the Medicament section in the present disclosure. Contact with corneal endothelial cells can be performed in vivo, ex vivo, or in vitro, and can be used in the manufacturing of a cell formulation.

In another aspect, the present disclosure provides a composition for growing or promoting the growth of corneal endothelial cells, the composition comprising the compound of the present disclosure, which has endoplasmic reticulum stress suppression activity (e.g., compound 1 or compound 6). The present invention also provides a method for growing or promoting the growth of corneal endothelial cells, the method comprising bringing an effective amount of the endoplasmic reticulum stress inhibitor into contact with corneal endothelial cells. It is understood that an embodiment of the endoplasmic reticulum stress inhibitor or the like used for growing or promoting the growth of corneal endothelial cells can use any embodiment described in the Medicament section in the present disclosure. Contact with corneal endothelial cells can be performed in vivo, ex vivo, or in vitro, and can be used in the manufacturing of a cell formulation.

Reference literature, such as scientific literature, patents, and patent applications, cited herein are incorporated herein by reference to the same extent that the entirety of each document is specifically described.

The present disclosure has been explained above while describing preferred embodiments to facilitate understanding. The present disclosure is explained below based on Examples. The above explanation and the following Examples are not provided to limit the present invention, but for the sole purpose of exemplification. Accordingly, the scope of the present disclosure is not limited to the embodiments and Examples that are specifically described in the present specification.

### Examples

Examples of the present disclosure are described below. However, the present disclosure is not limited to only these examples.

Biological samples or the like, if applicable, were handled in compliance with the standards enacted by the Ministry of Health, Labour and Welfare, Ministry of Education, Culture, Sports, Science and Technology, or the like, and, if applicable, based on the Declaration of Helsinki or ethical codes prepared based the Declaration of Helsinki. For the donation of eyes used for the study, consent was obtained from close relatives of all deceased donors. The study was approved by the ethics committee or an equivalent body of the University of Erlangen-Nuremberg (Germany) and SightLife^{™} (Seattle, Washington) eye bank.

### Reagents, Equipment, and Method

Chemicals were of the highest grade available, supplied by Tokyo Chemical Industry Co., Ltd., Fujifilm Wako Pure Chemical Corporation, Nacalai Tesque, or Sigma-Aldrich Co., LLC, and were used without further purification. Phosphate-buffered saline (PBS), Dulbecco's modified Eagle's medium (DMEM)/high glucose medium, and trypsin-EDTA were purchased from Gibco or Sigma. Penicillin/streptomycin (PS) was purchased from Nacalai Tesque. Protein concentrations were quantified using a BCA protein assay kit (Pierce). NMR spectra were obtained using a JEOL 600 MHz JNM-ECP spectrometer. High-resolution mass spectra were obtained using a JEOL M Station JMS-700.

### Cell Culture

HEK293 cells and mouse embryonic fibroblasts were cultured in DMEM supplemented with 10% FBS and 1% PS at 37°C under humidified air containing 5% CO₂.

### Measurement Method

In the Examples below, each measurement method was carried out as follows.

### Measurement of Heat-induced Turbidity Change in Cell Lysates

Self-assembling chemicals (about 140 compounds, including compounds of the present disclosure: final concentration: 50 µM) were added to 100 µL of whole-cell HEK293 lysate (1.0 mg/mL protein, 1% DMSO final concentration) in a 96-well plate. The mixture was heated at 60°C for 17 hours while being rotated and shaken at 300 rpm with an MBR-022UP incubator (TAITEC). Turbidity at 492 nm was measured with an MTP-880 microplate reader (Corona Electric Co., Ltd.).

### Tunicamycin Assay

HEK293 cells were seeded in 96-well plates (1×10⁴ cells/well) and incubated at 37°C for 2 hours. The medium was then replaced with 100 µL of DMEM (10% FBS) containing self-assembling chemicals (20 or more compounds, including compounds of the present disclosure) and 1% DMSO. After incubation with any one of compounds 1 to 6 at a given concentration for 12 to 18 hours, HEK293 cells were treated with tunicamycin (5 µg/mL) or thapsigargin (10 µM) for 48 hours, and cell viability was measured using a WST-8 cell counting kit (Dojindo Laboratories).

### Flow Cytometry

HEK293 cells were seeded in 12-well plates at 3 × 10⁵ cells/well. After 2 hours of incubation at 37°C, cells were treated with compound 1 (100 µM) and 9-(2,2-dicyanovinyl)julolidine (DCVJ, 1.0 µM) in the presence or absence of chloroquine (100 µM), chlorpromazine (20 µM), and genistein (100 µM) in DMEM (10% FBS) at 37°C for 16 hours. Cells were harvested by incubation in 2 mM EDTA/PBS (pH of 7.4) for 10 minutes. Fluorescence of the cells was measured using a BD FACSAria II and analyzed with BD FACSDiva software.

### Fluorescence Imaging with compound 1 and Derivatives Thereof in Living Cells

HEK293 cells were incubated with compound 1, 4, 5, or 6 (25 or 100 µM) in the presence of 1.0 µM DCVJ in DMEM (10% FBS). Fluorescence and bright-field images were taken with a CV1000 confocal microscope equipped with a 40× objective lens (Yokogawa Electric Corporation).

### Imaging of Co-assembly of Compound 1, Compound 6, and BSA-Fluorescein

To prepare denatured BSA-flu, a solution of BSA-flu (1.1 mg/mL) in PBS (pH of 7.4) was incubated at 80°C for 1 hour. Native or denatured BSA-fluorescein (final concentration: 0.1 mg/mL) was added to a solution of compound 1 (100 µM) or compound 6 (25 µM) in PBS (pH of 7.4) containing 2.0 mg/mL BSA. The mixture was incubated at 25°C for 19 hours and then images were taken with a CV1000 confocal microscope. The fluorescence intensity of the co-assemblies was quantified from the images obtained using ImageJ software.

### Luciferase-based Refolding Assay in Live Cells

HEK293 cells were seeded in 96-well plates (2 × 10⁴ cells/well) and transfected with a luciferase expression plasmid (0.1 µg/well) using FuGENE HD (Promega). After 24 hours of incubation, cells were treated with compound 1 (100 µM), compound 6 (25 µM), or PBA (2.0 mM) for 20 hours. Refolding assay was performed as described in Walther, T.V.; Maddalo, D. Intracellular Refolding Assay. J Vis Exp 2012, No. 59. https://doi.org/10.3791/3540. Briefly speaking, luciferase was denatured in live cells by incubation at 45°C for 30 minutes in the presence of 200 mM MOPS and 20 µg/mL cycloheximide. After the denaturation step, the cells were incubated at 37°C for various times to allow the luciferase to refold. After cells were lysed with Reporter Lysis 5X Buffer (Promega), luciferase activity was measured.

### Quantitative RT-PCR

First, total RNA was isolated using ISOGEN (tradename of Nippon Gene Co., Ltd.). cDNA was prepared using a Prime Script (trade name) first-strand cDNA synthesis kit (TaKaRa). qRT-PCR was performed with Fast SYBR Green Master Mix (Thermo Fisher) on a QuantStudio 3 real-time PCR system (Thermo Fisher). The copy number of each transcript is expressed relative to β-actin. The sequences of the primers (forward and reverse) are as follows: ATF4 (GTTCTCCAGCGACAAGGCTA and ATCCTGCTTGCTGTTGTTGG), sXBP-1 (CTGAGTCCGAATCAGGTGCAG and ATCCATGGGGAGATGTTCTGG), CHOP (GAACGGCTCAAGCAGGAAATC and TTCACCATTCGGTCAATCAGAG), BIP (TAGCGTATGGTGCTGCTGTC and TGACACCTCCCACAGTTTCA), and ERDJ4 (TGGTGGTTCCAGTAGACAAAGG and CTTCGTTGAGTGACAGTCCTGC).

### Native PAGE Analysis of Heat-induced BSA Denaturation

A solution of BSA (1.0 mg/mL) in PBS (100 µL, pH of 7.4) was incubated at 25°C or 75°C for 1 hour in the presence or absence of 100 µM compound 1, compound 6, or 10 mM PBA. Samples were subjected to native PAGE analysis using 8% acrylamide gels in 0.1 M Tris-HCl, pH of 7.8 (cathode) and 68 mM glycine, 53 mM Tris-HCl, pH of 8.9 (anode). After staining the gel with Coomassie Brilliant Blue, images were taken using an ImageQuant LAS500 imager (Cytiva).

### Reference Example: Screening of Self-assembling Compounds

To search for seed compounds, the present inventors used an in-house library of 142 self-organizing compounds (Jin, S.; Vu, H.T.; Hioki, K.; Noda, N.; Yoshida, H.; Shimane, T.; Ishizuka, S.; Takashima, I.; Mizuhata, Y.; Pe, K.B.; Ogawa, T.; Nishimura, N.; Packwood, D.; Tokitoh, N.; Kurata, H.; Yamasaki, S.; Ishii, K.J.; Uesugi, M. Discovery of Self-Assembling Small Molecules as Vaccine Adjuvants. Angewandte Chemie Int Ed 2021, 60 (2), 961-969. https://doi.org/10.1002/anie.202011604). In the primary screen, heat-induced turbidity change of HEK293 cell lysates was measured as an indication of aggregation of denatured protein in the presence of 50 µM of each library compound. From the library compounds, 29 compounds were selected that showed inhibition comparable to that of 10 mM PBA and TUDCA, which are detergent-like chemical chaperones commonly used for protein solubilization (Fig. 20). In a secondary screen, 29 candidates were assayed for their ability to prevent ER stress-induced cell death. HEK293 cells were treated with tunicamycin (Tm, 5.0 µg/mL) in the presence of each compound (50 µM). Tunicamycin is an ER stress inducer that inhibits protein N-glycosylation and generates incompletely processed proteins within the ER. Tunicamycin treatment for 48 hours resulted in a significant decrease in cell viability. Of the 29 compounds, two different compounds (TD-4C8 and CB-6D7) significantly rescued cells from tunicamycin-induced cell death at 50 µM, whereas PBA and TUDCA had little effect even at higher concentrations (Fig. 21). For detailed evaluation, the present inventors decided to focus on TD-4C8, which showed the highest recovery rate.

### Example 1: Production of Compound 1

As shown in the following scheme 1, compound (TD-4C8) was hydrolyzed, and the hydrolysis product was cyclized by UV (ultraviolet) irradiation to produce compound 1. A more detailed synthesis scheme is shown in Scheme 2 below. As shown in Scheme 2 below, compounds 2 to 6 described below were also produced in the same manner. The structure of compound 1 was confirmed by ¹D NMR (¹H, ¹³C, ¹⁹F), 2D ¹H-¹H NOESY (Fig. 22), HRMS, and single-crystal X-ray crystallography (Table S1 below).

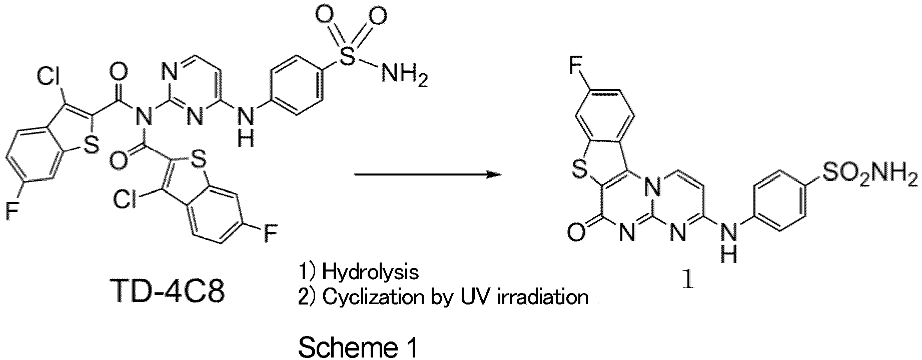

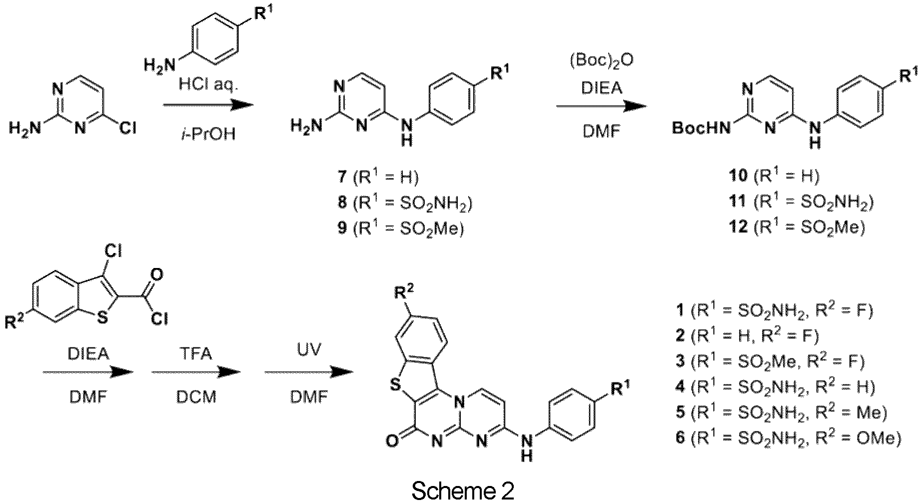

The compound shown in Scheme 2 was synthesized (produced) as follows.

### Synthesis of N⁴-Phenylpyrimidine-2,4-diamine (compound 7)

2-Amino-4-chloropyrimidine (500 mg, 3.86 mmol) and aniline (0.530 mL, 5.80 mmol) were mixed in 5 mL of 2-propanol. To this mixture was added a few drops of concentrated HCl and stirred at 80°C for 3 hours. The stirred mixture was cooled to room temperature and diluted with 25 mL of ethyl acetate. The organic layer was washed twice with an aqueous sodium bicarbonate solution and then once with saturated brine. The washed organic phase was dried over sodium sulfate and concentrated. The resulting residual solid was recrystallized from ethyl acetate and hexane to obtain the target compound 7 as a pale-yellow powder (484 mg, 2.60 mmol; yield 67.4%).

### Instrumental Analysis Data of Compound 7

¹H NMR (600 MHz, DMSO-d₆) δ9.08 (s, 1H), 7.80 (d, J = 5.4 Hz, 1H), 7.71 (dd, J = 8.4, 1.2 Hz, 2H), 7.26 (dd, J=9.0, 7.8 Hz, 2H), 6.94 (tt, J=7.2, 1.2 Hz, 1H), 6.20 (s, 2H), 6.00 (d, J=5.4 Hz, 1H)
¹³CNMR (150 MHz, DMSO-d₆) δ162.9, 160.7, 156.2, 140.5, 128.5, 121.3, 119.2, 96.5
HRMS (ESI+) [M+H] calcd. 187.0978; found 187.0980 (0.2 mmu)

### Synthesis of 4-((2-aminopyrimidin-4-yl)amino)benzenesulfonamide (compound 8)

2-Amino-4-chloropyrimidine (530 mg, 4.09 mmol) and sulfanilamide (784 mg, 4.55 mmol) were suspended in 20 mL of 2-propanol. A few drops of concentrated hydrochloric acid were added to the mixture, and the resulting mixture was stirred at 100°C for 5 hours. The stirred mixture was cooled to room temperature and concentrated on a rotary evaporator. The remaining solid was suspended in 35 mL of a 2N (2 mol/L) aqueous HCl solution. The suspension was neutralized with 70 mL of a saturated aqueous NaHCO₃ solution and extracted with ethyl acetate (60 mL x 5). The extracted organic layer was washed once with saturated aqueous NaHCO₃ solution, and then washed once with saturated brine. This was concentrated to give the target product, compound 8, as a pale-yellow powder (1.09 g, 4.10 mmol; yield 100%).

### Instrumental Analysis Data of Compound 8

¹HNMR (600 MHz, DMSO-d₆) δ9.50 (s, 1H), 7.92 (d, J = 9.0 Hz, 2H), 7.87 (d, J = 6.0 Hz, 1H), 7.69 (d, J = 9.0 Hz, 2H), 7.20 (s, 2H), 6.37 (s, 2H), 6.05 (d, J = 6.0 Hz, 1H)
¹³CNMR (150 MHz, DMSO-d₆) δ162.8, 160.4, 156.8, 143.7, 135.9, 126.4, 118.1, 97.0
HRMS (ESI+) [M+H] calcd., 266.0706; found, 266.0705 (-0.1 mmu)

### Synthesis of N⁴-(4-(methylsulfonyl)phenyl)pyrimidine-2,4-diamine (compound 9)

2-Amino-4-chloropyrimidine (257 mg, 1.98 mmol) and 4-(methylsulfonyl)aniline (383 mg, 2.24 mmol) were suspended in 2 mL of 2-propanol. A few drops of concentrated hydrochloric acid were added to the mixture, and the resulting mixture was stirred at 80°C for 3 hours. The stirred mixture was cooled to room temperature, and 10 mL of aqueous 100 mM HCl was added. The mixture was neutralized with NaHCO₃ to give a yellow precipitate. The precipitate was collected by filtration and washed twice with water. Recrystallization from ethanol gave the target product, compound 9, as a pale-yellow powder (328 mg, 1.24 mmol; yield 63%) .

### Instrumental Analysis Data of Compound 9

¹HNMR (600 MHz, DMSO-d₆) δ9.64 (s, 1H), 8.02 (d, J = 8.4 Hz, 2H), 7.91 (d, J = 5.4 Hz, 1H), 7.77 (d, J = 9.0 Hz, 2H), 6.41 (s, 2H), 6.08 (d, J = 5.4 Hz, 1H), 3.15 (s, 3H)
¹³CNMR (150 MHz, DMSO-d₆) δ162.8, 160.3, 157.0, 145.3, 132.0, 127.9, 118.3, 97.2, 43.9
HRMS (ESI+) [M+H] calcd., 265.0754; found, 265.0753 (-0.1 mmu)

### Synthesis of Tert-butyl (4-(phenylamino)pyrimidin-2-yl)carbamate (compound 10)

Compound 7 (95.3 mg, 0.512 mmol) and di-tert-butyl dicarbonate (135 mg, 0.619 mmol) were mixed in 1 mL of DMF (dimethylformamide). DIEA (N,N-diisopropylethylamine, 0.174 mL, 1.00 mmol) was added to the mixture, and the resulting mixture was stirred at 25°C for 18 hours. The stirred mixture was diluted with ethyl acetate and washed twice with a saturated aqueous NaHCO₃ solution, once with water, and once with saturated brine. The washed organic layer was dried over Na₂SO₄ and concentrated. The resulting crude product was purified by column chromatography (50% to 100% ethyl acetate/hexanes) to give the target product, compound 10, 76.1 mg (0.266 mmol, yield 52%) as a white powder.

### Instrumental Analysis Data of Compound 10

¹HNMR (600 MHz, DMSO-d₆) δ9.69 (s, 1H), 9.52 (s, 1H), 8.06 (d, J = 6.0 Hz, 1H), 7.92 (d, J = 7.8 Hz, 2 H), 7.27 (dd, J=9.0, 7.2 Hz, 2H), 6.97 (tt, J=7.2, 1.2 Hz, 1H), 6.42 (d, J=6.0 Hz, 1H), 1.48 (s, 9H)
¹³CNMR (150 MHz, DMSO-d₆) δ160.3, 157.1, 155.7, 150.9, 140.0, 128.5, 121.7, 119.2, 101.9, 78.9, 27. 9
HRMS (ESI+) [M+H] calcd., 287.1503; found, 287.1501 (-0.2 mmu)

### Synthesis of tert-butyl (4-((4-sulfamoylphenyl)amino)pyrimidin-2-yl)carbamate (compound 11)

Compound 8 (530 mg, 2.00 mmol) and di-tert-butyl dicarbonate (895 mg, 4.10 mmol) were mixed in 5 mL of dry DMF. DIEA (0.600 mL, 3.44 mmol) was added to the mixture and the resulting mixture was stirred at 25°C for 18 hours. The stirred mixture was diluted with 60 mL of ethyl acetate and washed 5 times with a saturated aqueous NaHCO₃ solution and once with saturated brine. The organic layer was dried over Na₂SO₄ and concentrated on a rotary evaporator to give the target product, compound 11, as a white powder (350 mg, 0.958 mmol; yield 48%).

### Instrumental Analysis Data of Compound 11

¹HNMR (600 MHz, DMSO-d₆) δ9.92 (s, 1H), 9.85 (s, 1H), 8.17 (d, J = 9.0 Hz, 2H), 8.15 (d, J=5.4 Hz, 1H), 7.71 (d, J=9.0 Hz, 2H), 7.21 (s, 2H), 6.48 (d, J=5.4 Hz, 1H), 1.50 (s, 9H)
¹³CNMR (150 MHz, DMSO-d₆) δ159.9, 157.1, 156.4, 150.9, 143.1, 136.4, 126.4, 118.4, 102.3, 79.1, 27.9
HRMS (ESI+) [M+H] calcd., 366.1231; found, 366.1227 (-0.4 mmu)

### Synthesis of Tert-butyl (4-((4-(methylsulfonyl)phenyl)amino)pyrimidin-2-yl)carbamate (compound 12)

Compound 9 (106 mg, 0.401 mmol) and di-tert-butyl dicarbonate (196 mg, 0.898 mmol) were mixed in 2 mL of dry DMF. DIEA (0.150 mL, 0.861 mmol) was added to the mixture and the resulting mixture was stirred at 50°C for 16 hours. The stirred mixture was cooled to room temperature, diluted with 20 mL of ethyl acetate, and washed twice with a saturated aqueous NaHCO₃ solution and once with saturated brine. The organic layer was dried over Na₂SO₄ and concentrated. The resulting residual oil was solidified in 2 mL of ethyl acetate by sonication. The resulting solid was collected by filtration and dried under vacuum to give the target product, compound 12 (62.0 mg, 0.170 mmol, yield 42%).

### Instrumental Analysis Data of Compound 12

¹HNMR (600 MHz, DMSO-d₆) δ10.0 (s, 1H), 9.89 (s, 1H), 8.26 (d, J = 9.0 Hz, 2H), 8.18 (d, J=6.0 Hz, 1H), 7.78 (d, J=9.0 Hz, 2H), 6.51 (d, J=6.0 Hz, 1H), 3.17 (s, 3H), 1.50 (s, 9H)
¹³CNMR (150 MHz, DMSO-d₆) δ159.9, 157.1, 156.6, 150.9, 144.7, 132.6, 127.9, 118.7, 102.5, 79.2, 43. 9, 27.9
HRMS (ESI+) [M+H] calcd. 365.1278; found 365.1275 (-0.3 mmu)

3-Chlorobenzo[b]thiophene-2-carbonyl chloride and derivatives thereof were prepared according to the methods described in the following Reference Literature 4 to 6.
Reference Literature 4: Wright, W.B., Jr.; Brabander, H.J., Preparation of 3-chlorobenzo[b]thiophene derivatives from cinnamic acids, J. Heterocycl, Chem. 1971, 8 (5), 711-714.
Reference Literature 5: Higa, T.; Krubsack, A.J, Oxidations by thionyl chloride, 8, A convenient synthesis of benzo[b]thiophenes from carboxylic acids and ketones, J. Org. Chem. 1976, 41, 21, 3399-3403.
Reference Literature 6: Pakray, S.; Castle, R.N. The synthesis of dimethoxy[1]benzothieno[2,3-c]quinolines, J. Heterocycl. Chem. 1986, 23 (5), 1571-1577.

### Synthesis of Compound 1

Compound 11 (109 mg, 0.299 mmol) was dissolved in 3 mL of dry THF (tetrahydrofuran). To this solution was added 3-chloro-6-fluorobenzo[b]thiophene-2-carbonyl chloride (83.0 mg, 0.333 mmol) and DIEA (62.6 µL, 0.359 mmol) at 25°C. The mixture was stirred at 25°C for 20 minutes, then diluted with 30 mL of ethyl acetate and washed twice with a saturated aqueous NaHCO₃ solution and once with saturated brine. The washed organic layer was dried over Na₂SO₄ and concentrated. The resulting residual solid was suspended in 1 mL of dry dichloromethane. Trifluoroacetic acid (1 mL) was added to the mixture at 25°C, and the resulting mixture was stirred at 25°C for 1 hour. The obtained mixture was concentrated on a rotary evaporator. Addition of 2 mL of dichloromethane and evaporation were repeated 3 times. 5 mL of ethyl acetate was added to the residual oil to obtain a precipitate of the product. The precipitate was collected by filtration and washed with ethyl acetate (yield: 136 mg). A portion of the crude product thus obtained (38.2 mg) was dissolved in 20 mL of dry DMF. The solution was irradiated with UV (365 nm, 80 W) in a cold room (4°C) for 3 hours, and the reaction was monitored by LC-MS. After completion of the reaction, DMF was removed by evaporation and 2 mL of acetone was added to the mixture to give a precipitate of compound 1. The precipitate was transferred to a 1.5 mL microtube and centrifuged briefly. The resulting pellet was washed 3 times with 1 mL of acetone and dried overnight to give target compound 1 as a pale-yellow solid (10.6 mg, 0.0197 mmol as TFA (trifluoroacetate) salt; yield obtained by the three-step procedure: 29%).

### Instrumental Analysis Data of Compound 1

¹HNMR (600 MHz, DMSO-d₆) δ11.0 (s, 1H), 9.29 (d, 1H, J = 9.6 Hz), 8.68 (dd, 1H, J = 9.6, 3.5 Hz), 8.24 (d, 1H, J=2.4 Hz ), 8.11 (d, 2H, J = 6.6 Hz), 7.87 (d, 2H, J = 8.4 Hz), 7.59 (dd, 1H, J = 9.0, 2.4 Hz), 7.38 (s, 2H), 6.89 (d, 1H, J=7.2 Hz)
¹³CHMR (150 MHz, DMSO-d₆) δ163.2, (162.0, 160.4), 157.5, 151.4, (141.54, 141.46), (141.1, 139.0), 138.8, 134.2, 126.7, (126.3, 126.2), 126.1, 125.6, 124.7, (123.2, 120.1), (115.0, 114.8), (111.0, 110.9), 102.8
HRMS (ESI+) [M+H] calcd., 442.0438; found, 442.0439 (0.1 mmu)

### Synthesis of Compound 2

Compound 2 was synthesized from compound 10 and 3-chloro-6-fluorobenzo[b]thiophene-2-carbonyl chloride following the same synthetic procedure as for compound 1. As a result, the target compound 2 was obtained as a pale-yellow solid (yield obtained by the three-step procedure: 24%). The NMR spectrum showed that the solid contained about 25% of another positional isomer (regioisomer) that could not be separated by HPLC.

### Instrumental Analysis Data of Compound 2

¹HNMR (600 MHz, DMSO-d₆) δ10.8 (s, 1H), 9.26 (d, J = 7.2 Hz, 1H), 8.70 (m, 1H), 8.26 (dd, J = 9.0, 2.4 Hz, 2H), 7.95 (d, J = 4.8 Hz, 1H), 7.60 (td, J = 9.0, 2.0 Hz, 1H), 7.45 (m, 2H), 7.20 (m, 1H), 6.86 (d, J=6.0 Hz)
¹³CNMR (150 MHz, DMSO-d₆) δ (162.0, 160.3), 157.6, 151.4,0 (141.6, 141.5), 138.1, 137.0, 134.3, 128. 9, 128.5, (126.4, 126.3), 125.9, 125.7, 124.4, (123.8, 120.6), (114.9, 114.8), (111.0, 110.9), 102.9
HRMS (ESI+) [M+H] calcd. 363.0710; found 363.0711 (0.1 mmu)

### Synthesis of Compound 3

Compound 3 was synthesized from compound 12 and 3-chloro-6-fluorobenzo[b]thiophene-2-carbonyl chloride following the same synthetic procedure as for compound 1. As a result, the target compound 3 was obtained as a pale-yellow solid (yield obtained by the three-step procedure: about 39%). NMR spectra showed that the resulting solid contained about 15% of another positional isomer (regioisomer) that could not be separated by HPLC.

### Instrumental Analysis Data of Compound 3

¹HNMR (600 MHz, DMSO-d₆) δ10.9 (s, 1H), 9.30 (d, J = 7.8 Hz, 1H), 8.69 (dd, J = 9.6, 4.8 Hz, 1H) , 8.26 (dd, J=9.0, 2.4 Hz, 1H), 8.18 (d, J=9.0 Hz, 2H), 7.98 (d, J=9.0 Hz, 2H), 7.59 (td, J=9.0, 2.4 Hz, 1H), 6.84 (d, J=7.2 Hz, 1H), 3.22 (s, 3H)
¹³CNMR (150 MHz, DMSO-d₆) δ164.6, (161.9, 160.3), 157.6, 151.9, 143.1, (141.33, 141.26), 138.9, (135.0, 134 .2), 128.2, 127.6, (126.3, 126.2), 125.9, 125.2, (123.2, 120.0), (114.7, 114.6), (111.0, 110.9), 102.1, 43.7
HRMS (ESI+) [M+H] calcd., 441.0486; found, 441.0485 (-0.1 mmu)

### Synthesis of Compound 4

Compound 4 was synthesized from compound 11 and 3-chlorobenzo[b]thiophene-2-carbonyl chloride following the same synthetic procedure as for compound 1. As a result, the target compound 4 was obtained as a pale-yellow solid (yield obtained by the three-step procedure: about 27%).

### Instrumental Analysis Data of Compound 4

¹HNMR (600 MHz, DMSO-d₆) δ10.9 (s, 1H), 9.34 (d, J=7.2 Hz, 1H), 8.65 (d, J=7.8 Hz, 1H), 8.29 (dd, J=7.8, 1.2 Hz, 1H), 8.11 (d, J=7.8 Hz, 2H), 7.87 (d, J=9.0 Hz, 2H), 7.70 (m, 2H), 7.37 (s, 2H), 6.87 (d, J=7.8 Hz, 1H)
¹³CNMR (150 MHz, DMSO-d₆) δ164.2, 157.4, 151.8, 141.4, 139.5, 138.9, 138.8, 134.5, 128.8, 127.9, 126.7, 125.9, 125.0, 124.8, 124.1, 119.9, 102.4
HRMS (ESI+) [M+H] calcd., 424.0533; found, 424.0530 (-0.3 mmu)

### Synthesis of Compound 5

Compound 5 was synthesized from Compound 11 and 3-chloro-6-methylbenzo[b]thiophene-2-carbonyl chloride following the same synthetic procedure as for compound 1. As a result, the target product was obtained as a pale-yellow solid (yield obtained by the three-step procedure: 23%).
¹HNMR (600 MHz, DMSO-d₆, 50°C) δ11.0 (s, 1H), 9.32 (d, J=7.8 Hz, 1H), 8.53 (d, J=9.0 Hz, 1H), 8.10 (m, 3H), 7.88 (d, J=9.0 Hz, 2H), 7.53 (dd, J=9.0, 1.2 Hz, 1H), 7.27 (s, 2H), 6.94 (d, J=7.8 Hz, 1H), 2.53(s, 3H)
¹³CNMR (150 MHz, DMSO-d₆) δ163.8, 157.4, 151.4, 141.3, 139.9, 138.9, 138.8, 138.3, 134.5, 127.6, 126.7, 126.4, 124.4, 123.9, 123.7, 119.9, 102.6, 20.9
HRMS (ESI+) [M+H] calcd., 438.0689; found, 438.0686 (-0.3 mmu)

### Synthesis of Compound 6

Compound 6 was synthesized from compound 11 and 3-chloro-6-methoxybenzo[b]thiophene-2-carbonyl chloride following to the same synthetic procedure as for compound 1. As a result, the target product was obtained as a pale-yellow solid (yield obtained by the three-step procedure: 24%).

### Instrumental Analysis Data of Compound 6

¹HNMR (600 MHz, DMSO-d₆) δ10.7 (s, 1H), 9.22 (d, J=8.4 Hz, 1H), 8.49 (d, J=9.6 Hz, 1H), 8.10 (d, J=8.4 Hz, 2H), 7.87(d , J=9.0 Hz, 2H), 7.84 (d, J=2.4 Hz, 1H), 7.35 (s, 2H), 7.22 (dd, J=9.0, 2.4 Hz, 1H), 6.80 (d, J=7.8 Hz, 1H), 3.91 (s, 3H)
^{13C}NMR (150 MHz, DMSO-d₆) δ159.2, 157.3, 151.1, 142.3, 141.2, 139.0, 138.7, 134.6, 126.7, 125.1, 122.0, 120.0, 118.5, 116.0, 106.9, 104.9, 102.8, 55.7
HRMS (ESI+) [M+H] calcd., 454.0638; found, 454.0636 (-0.2 mmu)

### HPLC Chromatogram

HPLC analysis of compounds 1 to 6 was performed on a Prominence HPLC System (trade name of Shimadzu Corporation) equipped with Intertsil ODS-3 columns using a linear gradient (20% acetonitrile/H₂O (0 min) to 90% acetonitrile/H₂O (30 min), and then 100% acetonitrile (30 to 40 min), each containing 0.1% trifluoroacetic acid, flow rate: 1.0 mL/min). The HPLC analysis demonstrated that compounds 1 to 6 were all synthesized with a purity of higher than 94%.

The X-ray crystal structure analysis of compound 1, DMSO, and methanol was carried out as follows. Crystals of compound 1 were prepared by the vapor diffusion method using DMSO and methanol. Suitable crystals were mounted in Fomblin^{®} Y oil on glass fiber. Low-temperature (90 K) data were collected on a Bruker D8 VENTURE system equipped with IµS Diamond MoOptics using MoKα radiation (λ=0.71073 Å (0.071073 nm)) in ω scans. Empirical absorption corrections were applied to the diffraction data using SADABS-2016/2 (Reference Literature 1 below). The structure was solved using SHELXT-2014/5 (Reference Literature 2 below) and refined to F² for all data by full-matrix least squares using SHELXL-2018/1 (Reference Literature 3 below). All non-hydrogen atoms were refined anisotropically. All hydrogen atom parameters were refined. Details of data quality and a summary of residual values for all structural improvements are included in Table S1 below. Accession number 2121059 contains supplemental crystallographic data for this article. These data are provided free of charge by the Cambridge Crystallographic Data Centre in collaboration with the Fachinformationszentrum Karlsruhe Access Structures Service www.ccdc.cam.ac.uk/structures.
Reference Literature 1: Krause, L.; Herbst-Irmer, R.; Sheldrick G.M.; Stalke D. Comparison of Silver and Molybdenum Microfocus X-ray Sources for Single-crystal Structure Determination, J. Appl. Cryst. 2015, 48 (1), 3-10.
Reference Literature 2: Sheldrick, G.M. SHELXT - Integrated Space-Group and Crystal-Structure Determination, Acta Cryst. A 2015, 71 (1), 3-8.
Reference Literature 3: Sheldrick, G.M. Crystal Structure Refinement with SHELXL. Acta Cryst. C 2015, 71 (1), 3-8.

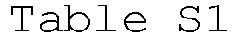

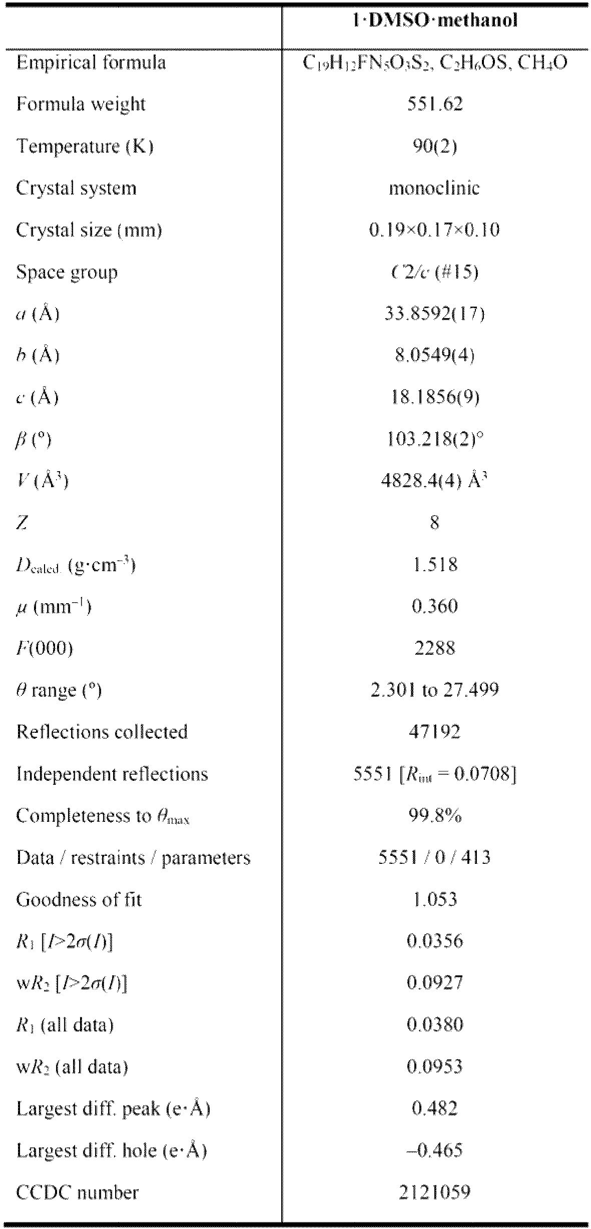

### Example 2: Biological Activity of Compound 1

The ability to alleviate endoplasmic reticulum (ER) stress of compound 1 was examined in HEK293 cells. Figs. 1 and 2 show the biological activity of compound 1 for decreasing cytotoxicity under ER stress. Fig. 1 shows the rescue from cell death under ER stress by PBA and compound 1. HEK293 cells were pre-incubated with compound 1 (100 µM) or PBA (2.0 mM) for 12 hours and then treated with thapsigargin (10 µM) or tunicamycin (5.0 µg/mL) for 48 hours. Error bars represent s.d. (n=3). Statistical analysis was performed according to one-way ANOVA and a Dunnett's test using R software. As shown in the figure, treatment with compound 1 enhanced cell viability under ER stress conditions and restored activity of the screening compound. To further study the effect of compound 1 on ER stress, mRNA levels of typical UPR genes, such as ATF4, s-XPB1, CHOP, BiP, and ERDJ4, were examined. In addition to compound 1, compound 6 was also tested in the same experiment. Fig. 2 shows the results of measuring the mRNA expression levels of genes associated with ER stress by quantitative PCR. Specifically, the figure shows the relative changes in mRNA levels induced by compound 1 or compound 6 under ER stress. The quantitative RT-PCR was performed according to the method described above. HEK293 cells were treated with compound 1 (100 µM) for 10 hours and then incubated with tunicamycin (1.0 µg/mL) for 24 hours. Isolated RNA samples were subjected to reverse transcription and quantitative RT-PCR. The bars in the bar graph indicate, from the left, the measurement results in the presence of tunicamycin (without compounds 1 and 6), in the absence of tunicamycin (without compounds 1 and 6, with DMSO only), in the presence of tunicamycin and compound 1, and in the presence of tunicamycin and compound 6. Error bars represent s.d. (n=3). Statistical analysis was performed according to the Student's t-test. *P<0.05, **P<0.01; n.s.: not significant. From these measurement results, compound 1 and compound 6 were confirmed to limit the mRNA level of CHOP. Moreover, under these conditions, tunicamycin treatment increased the mRNA levels of two genes, CHOP for apoptosis induction and BiP, which is a molecular chaperone for the ER. The increase in CHOP levels was completely suppresed by pretreatment with compound 1 or compound 6, whereas the same pretreatment had a limited effect on Bip. This response pattern is consistent with the possibility that compound 1 reduces the apoptosis-promoting UPR and results in a higher level of cell viability under ER stress.

### Example 3: Synthesis of Compounds 1 to 6

Furthermore, compounds 2 to 6, which are derivatives of compound 1, were prepared. The structures of compounds 1 to 6 are as shown in the following chemical formulas. Compounds 2 to 6 were produced in the same manner as in the production of compound 1, except that starting materials having structures corresponding to compounds 2 to 6 were used instead of TD-4C8, as shown in scheme 2 above. In Examples 4 to 9 described below, the biological activity of Compounds 1 to 6 was measured.

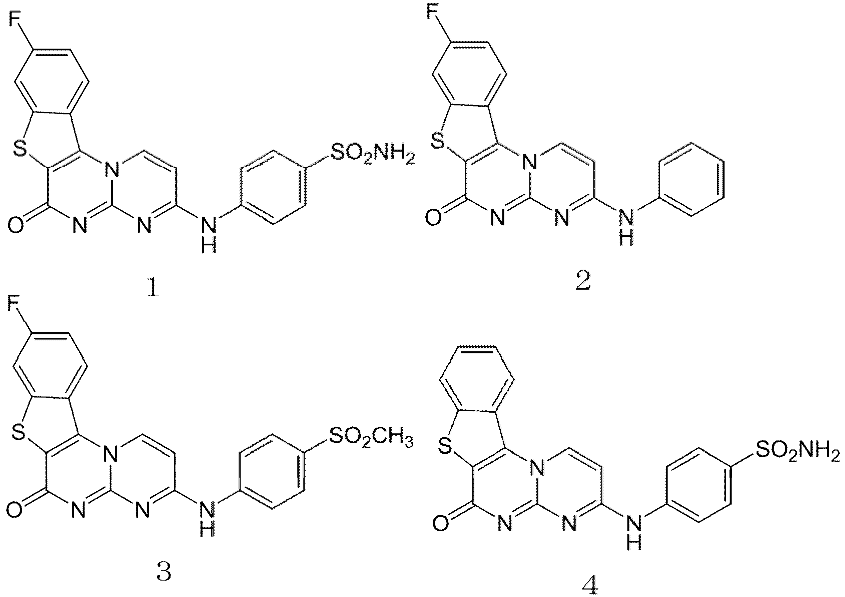

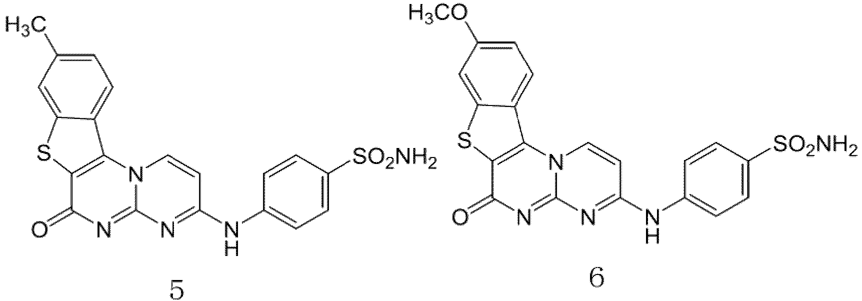

### Example 4: Assembly of Compound 1 in Aqueous Solution and Living Cells

Compound 1 is readily soluble in DMSO, but readily forms discrete assemblies at 100 µM in a PBS buffer and a cell culture medium (DMEM containing 10% FBS). Dynamic light scattering (DLS) analysis revealed that the average size of the assemblies was about 700 and 200 nm in diameter respectively in PBS (pH of 7.4) and DMEM (10% FBS) (Fig. 23). Doping with the environmentally sensitive dyes, Nile red and 9-(2,2-dicyanovinyl)julolidine (DCVJ), enabled detection of assembly formation by fluorescence spectroscopy and confocal microscopy. The relative amount of self-organaization of compound 1 was quantified using DCVJ to form a DMEM-normalized dose-organization curve (Fig. 24). Fitting with a sigmoid curve gave an apparent half-maximal assembly concentration (AC50) of 29.7 µM for the molecules of compound 1. Dilution of the preformed assemblies of compound 1 resulted in disintegration of the particles, demonstrating the reversibility of self-assembly (Fig. 25). In confocal microscopy, assemblies of compound 1 stained with Nile red were readily detectable in PBS (pH of 7.4). In the cell culture medium, assemblies of compound 1 stained with Nile red were initially not visible due to the small particle size, but prolonged incubation (>5 hr) led to the formation of larger detectable assemblies (>1 µm in diameter).

Fig. 3 shows the self-assembly of compound 1 in living HEK293 cells. Fig. 3(A) is a fluorescence image of HEK293 cells treated with compound 1 (100 µM) and DCVJ (1.0 µM). Fig. 3(B) shows quantification of compound 1 (100 µM) in cells stained with DCVJ (1.0 µM) by flow cytometry in the presence or absence of endocytosis inhibitors (100 µM chloroquine, 20 µM chlorpromazine, and 100 µM genistein). Statistical analysis was performed according to the one-way ANOVA and Dunnett's test. *P<0.05, **P<0.01, n.s.: not significant. The fluorescence images were measured as described above. When HEK293 cells were treated with 100 µM of compound 1 and 1.0 µM of DCVJ, fluorescent particles were observed within the cells after 8 hours of incubation, whereas treatment with DCVJ alone did not display any detectable particles (Fig. 3A). The intracellular localization of the assemblies can be explained by two possibilities: (i) self-assembled compound 1 is taken up into cells by endocytosis (endocytosis) or (ii) nanoparticles of compound 1 directly permeate into cells (direct permeation) (Reference Materials 15 to 17 below). To estimate the contribution of endocytosis, the amount of intracellular assemblies of compound 1 was quantified by flow cytometry, using three inhibitors acting on different endocytosis pathways (chloroquine, chlorpromazine, and genistein). Of the inhibitors tested, chloroquine and chlorpromazine reduced the average DCVJ signal by about 30%, suggesting that endocytosis contributes in part to the intracellular accumulation of compound 1 (Fig. 3B). The results indicate that endocytosis and direct permeation both contribute equally to the intracellular accumulation of compound 1. Given the reversibility of assembly, it is possible that preformed particles may partially disassemble, and free monomers may passively diffuse into cells to form assemblies within the cells.
Reference Material 15: Verma, A.; Uzun, O.; Hu, Y.; Hu, Y.; Han, H.S.; Watson, N.; Chen, S.; Irvine, D.J.; Stellacci, F. Surface-Structure-Regulated Cell-Membrane Penetration by Monolayer-Protected Nanoparticles. Nat Mater 2008, 7 (7), 588-595. https://doi.org/10.1038/nmat2202.
Reference Material 16: Nakamura, H.; Watano, S. Direct Permeation of Nanoparticles Across Cell Membrane: A Review. Kona Powder Part J 2018, 35 (0), 2018011. https://doi.org/10.14356/kona.2018011.
Reference Material 17: Guo, Y.; Terazzi, E.; Seemann, R.; Fleury, J.B.; Baulin, V.A. Direct Proof of Spontaneous Translocation of Lipid-Covered Hydrophobic Nanoparticles through a Phospholipid Bilayer. Sci Adv 2016, 2 (11), e1600261.
https://doi.org/10.1126/sciadv.1600261.

### Example 5: Measurement of Biological Activity of Compound 6

As described above, five derivatives of compound 1, which are referred to as "compounds 2 to 6," were synthesized to evaluate the structure-activity relationship. Figs. 4, 26, and 27 show the structure-activity relationship of compound 1 and its derivatives, compounds 2 to 6. Figs. 4 and 26A show changes in cell viability due to compound 1 and its derivatives, compounds 2 to 6, under ER stress. HEK293 cells were treated with tunicamycin (5.0 µg/mL) in the presence of the indicated compounds for 48 hours. Replacement of the sulfonamide at the R¹ position with hydrogen (compound 2) or a methylsulfenyl group (compound 3) eliminated the ability of the compounds to rescue cells from tunicamycin-induced ER stress (Fig. 26A). Replacement with a fluoride group at the R² position also had a significant effect on activity. For example, whereas the derivative with a methyl group (compound 5) lost activity, the derivative with a methoxy group (compound 6) showed activity at a lower concentration (25 µM) than compound 1 (100 µM) (Fig. 4). Although no obvious cytotoxicity was observed for compounds 1 and 4 up to 100 µM, and compounds 5 and 6 at 25 µM, treatment with compound 5 or 6 at 100 µM slightly reduced cell viability (Fig. 27).

### Example 6: Measurement of Ability of Compounds 1 to 6 to Form Self-assemblies

The ability of compound 1 and its derivatives, compounds 4 to 6, to form assemblies was also monitored in living cells. Fig. 5 shows fluorescence images of the self-assemblies of compound 1 and its derivatives in living cells. HEK293 cells were treated with compound 1, 4, 5, or 6 (25 µM or 100 µM) and 1.0 µM of DCVJ for 8 hours. Fluorescence and bright-field images were taken with a CV1000 confocal microscope. HEK293 cells were treated with compound 1, 4, 5, or 6 (25 or 100 µM) and DCVJ (1.0 µM) for 8 hours and observed with a confocal microscope. All compounds showed particle formation at 100 µM. The active derivatives, compounds 1, 4, and 6, showed relatively small (<1 µm) assemblies within the cells, whereas compound 5 (inactive derivative 5) formed larger particles (~5 µm) that did not appear to be internalized within the cells. At 25 µM, only the molecules of compound 6 were the derivative that showed detectable assemblies within cells. The self-assembling ability of the molecules of compound 6 may be due to its cellular activity at that concentration, even at 25 µM. DLS analysis indicated that molecules of compound 6 (25 µM) were formed as assemblies with an average size of 130 nm in diameter in the cell culture medium (Fig. 28). The normalized dose-assembly curve for molecules of compound 6 showed a half-maximal assembly concentration (AC50) of 9.30 µM in the cell culture medium (Fig. 29), indicating that molecules of compound 6 form assemblies at lower concentrations than molecules of compound 1. To further support the importance of self-organization formation in cellular activity, dose-response curves for compounds 1 and 6 were determined. The biological activity of the compounds' molecular assembly is expected to be more cooperative than that of the monomer and to show a steeper dose-response curve (Reference Material 18 below). A tunicamycin assay was performed using compound 1, compound 6, and PBA of various concentrations. Fig. 6 is a graph showing dose-response curves in the tunicamycin assay using compound 1, compound 6, and PBA. HEK293 cells were treated with tunicamycin (5.0 µg/mL) for 48 hours in the presence of compound 1, compound 6, or PBA of various concentrations. Dose-response curve calculations using the Hill equation gave EC50 values of 38.1, 13.7, and 483 µM for compound 1, compound 6, and PBA, respectively. Importantly, compound 1 and compound 6 exhibited steep dose-response curves with high Hill coefficients (n=3.50 and 4.34 for compound 1 and compound 6, respectively). These results indicate that self-organization is a key factor for compound 1 and its derivatives in exerting their biological activity, with compound 6 being the most potent molecule. Compound 6 is referred to as "selfonamide" below. Compounds 2 and 3 were also confirmed to have self-assembly activity (self-assembling ability) in the same manner as for compounds 1, 4, 5, and 6.
Reference Material 18: Feng, B.Y.; Simeonov, A.; Jadhav, A.; Babaoglu, K.; Inglese, J.; Shoichet, B.K.; Austin, C.P. A High-Throughput Screen for Aggregation-Based Inhibition in a Large Compound Library. J Med Chem 2007, 50 (10), 2385-2390. https://doi.org/10.1021/jm061317y.

### Example 7: Cellular Activity of Self-Assemblies of Compound 1 and Self-Assemblies of Compound 6 (1)

Validation experiments were performed to gain insight into how the molecular self-assemblies of compound 1 and molecular self-assemblies of compound 6 exert their cellular activity. These compounds were first examined for their ability to inhibit the heat-induced denaturation of BSA in vitro. BSA (1.0 mg/mL) in PBS (pH of 7.4) was heated at 75°C for 1 hour in the presence or absence of compound 1, compound 6, or PBA. The samples were analyzed by native PAGE to monitor the denaturation of BSA (Fig. 11). PBA, a typical detergent-type chemical chaperone, prevented the thermal denaturation of BSA, but compound 1 and compound 6 did not have the same action. This suggests that the mechanism of action of the two compounds is different from that of PBA. One possible mechanism is that the assemblies of compounds 1 and 6 selectively interact with denatured proteins. BSA was fluorescently labeled with 5-carboxyfluorescein N-succimidyl ester, and the resulting fluorescently labeled BSA (BSA-flu) was denatured by heating at 80°C for 1 hour. Each compound was incubated with native or denatured BSA-flu (0.1 mg/mL) in the presence of excess unlabeled native BSA (2.0 mg/mL) in PBS (pH of 7.4). After 19 hours of incubation, the particles were examined with a confocal microscope (Figs. 7 and 30). Fig. 7 illustrates the formation of co-assembly between self-assembling chemicals (compounds of the present disclosure) and fluorescently labeled BSA (BSA-flu). Compound 1 or compound 6 was incubated with native (Nat.) or denatured (De.) BSA-flu (0.10 mg/mL) at 25°C for 19 hours in the presence of excess unlabeled BSA (2.0 mg/mL) and Nile red (1.0 µM). The mean fluorescence of the particles was quantified by using ImageJ software. Error bars represent s.d. (n>6). Statistical analysis was performed according to the Student's t-test. *P<0.05, **P<0.01; n.s.: not significant. From the images made by separating the transmitted light images and fluorescent images from the same images of Fig. 7, it was also confirmed that compound 1 and compound 6 selectively formed co-assemblies with heat-denatured BSA and hardly formed co-assemblies with undenatured (normal) BSA. These results indicate that in quantification of the fluorescent signal from the particles, compound 1 and compound 6 both showed enhanced fluorescence intensity of denatured BSA-flu compared to native BSA-flu, indicating that it is preferred that the compounds co-assemble with denatured BSA. Native PAGE analysis of heat-induced denaturation of BSA was performed as described above. Fluorescently labeled BSA (BSA-flu) was prepared as follows. To a solution of BSA (2.0 mg/mL) in 1.9 mL of PBS (pH of 7.4), 30 µL of 10 mM 5-carboxyfluorescein N-succimidyl ester in DMSO and 200 µL of 1.0 M sodium bicarbonate in water were added. The mixture was incubated at 25°C for 1 hour and then purified on a PD-10 desalting column (GE Healthcare). As described above, Fig. 11 is a photograph showing the results of CBB staining in electrophoresis on native Page (8% acrylamide gel). From the figure, it was confirmed that when BSA was heated at 75°C for 1 hour, almost no native BSA remained, regardless of whether compound 1 or compound 6 was present with BSA. This indicated that compound 1 and compound 6 does not exhibit activity to inhibit the thermal denaturation of BSA. Additionally, since PBA, an existing chemical chaperone, inhibited the thermal denaturation of BSA, compounds 1 and 6 were shown to have a mechanism of action different from that of the existing chemical chaperone. Taken together, these results indicate that the molecules of compound 1 and compound 6 do not stabilize native proteins and do not inhibit the process of protein denaturation, but form co-assemblies with pre-existing denatured proteins.

### Example 8: Cellular Activity of Self-Assemblies of Compound 1 and Self-Assemblies of Compound 6 (2)

An experiment was performed to confirm the role of co-assemblies in effecting activity. It is believed that molecules of compound 1 and compound 6 may accelerate the refolding of co-assembled proteins. To explore this possibility, a luciferase-based refolding assay in live cells was performed to monitor the effects of compounds 1 and 6 on protein refolding (Reference Material 19 below). Fig. 8 shows the results. Specifically, Fig. 8 is a graph showing the results of a luciferase-based refolding assay with living HEK293 cells. In this assay, HEK293 cells were transfected with a luciferase expression construct and treated with compound 1 or compound 6 for 20 hours. Live cells (luciferase-expressing cells) were heated in a water bath at 45°C for 30 minutes to denature the luciferase, and the denatured luciferase was then allowed to refold in the live cells at 37°C for various periods of time. Cycloheximide (20 µg/mL) was added to the cell cultures during the denaturation and refolding steps to eliminate the effects of newly synthesized luciferase. After the refolding step, the cells were lysed and measured for luciferase activity to estimate the refolding efficiency, thereby assessing the extent of refolding. Error bars represent s.d. (n=4). Statistical analysis was performed on the 4-h samples according to the one-way ANOVA and Dunnett's test. ***P<0.001; n.s., not significant. Unexpectedly, compounds 1 and 6 failed to accelerate the refolding of luciferase. Rather, compounds 1 and 6 slightly inhibited refolding, presumably by stabilizing the denatured state of the protein through co-assembly. These data indicate that refolding is not involved in the activity of compound 1 and compound 6.
Reference Material 19: Walther, T.V.; Maddalo, D. Intracellular Refolding Assay. J Vis Exp 2012, No. 59. https://doi.org/10.3791/3540.

### Example 9: Inhibition of Cytotoxicity by Compound 1 and Compound 6

Cellular protein aggregates are often cleared by cellular mechanisms, such as autophagy and ubiquitin-proteasome system. To examine the importance of these proteolytic pathways in the activity of compounds 1 and 6, their ability to inhibit **tunicamycin-induced** cytotoxicity in the presence of an autophagy or proteasome inhibitor (bafilomycin A1 or MG132, respectively) was measured. Fig. 9 is a graph showing the results of a tunicamycin assay using an autophagy or proteasome inhibitor. Cells were pre-incubated with compound 1 or compound 6 for 16 hours and then treated with tunicamycin (5.0 µg/mL) in the presence or absence of a specified substance for 48 hours. Fig. 9(A) is a graph showing measurements in the presence or absence of bafilomycin A1 (Baf A1, autophagy inhibitor). Fig. 9(B) is a graph showing measurements in the presence or absence of MG132 (proteasome inhibitor). Error bars represent **s.d.** (n=3). Statistical analysis was performed according to the one-way ANOVA and Dunnett's test. *P<0.05; n.s.: not significant. Bafilomycin A1 (Baf.) partially canceled the activity of compound 1 and compound 6 (Fig. 9A), whereas MG132 had no significant effect on the activity of molecules 1 and 6 (Fig. 9B). These results suggest a partial role for the autophagy system in the proteotoxicity resistance activity of both compound 1 and compound **6.** The concentrations used of the inhibitors were confirmed to be non-toxic to cells (Figs. 31 and 32). To further study the role of autophagy, autophagy-deficient atg7^{-/-} and p62^{-/-}mouse embryonic fibroblasts (MEFs) were subjected to a tunicamycin assay. Fig. 10 is a graph showing the results of the tunicamycin assay using the autophagy-deficient cells. Wild-type (wt), atg7 ^{-/-}, or p62 ^{-/-} mouse embryonic fibroblasts (MEFs) were treated with molecule 1 (100 µM) or molecule 6 (25 µM) for 18 hours and then incubated with tunicamycin (5.0 µg/mL) for 48 hours. Error bars represent **s.d.** (n=3). Statistical analysis was performed according to the one-way ANOVA and Dunnett's test. *P<0.05, **P<0.01. ATG7 is an E1-like enzyme important for phagophore expansion, and p62 is an autophagy receptor for ubiquitinated substrates (Reference Material 20 below). Wild-type (wt), atg7 ^{-/-}, or p62 ^{-/-} MEF cells were treated with compound 1 or compound 6 for 18 hours, followed by treatment with tunicamycin (5.0 µg/mL) for 48 hours. Compounds 1 and 6 rescued wild-type MEFs from ER stress-induced cell death, but that ability was severely impaired in atg7^{-/-} and p62^{-/-} MEFs. These results indicate that autophagy plays an important role in the activity of both molecules.
Reference 20: Dikic, I.; Elazar, Z. Mechanism and Medical Implications of Mammalian Autophagy. Nat Rev Mol Cell Bio 2018, 19 (6), 349-364. https://doi.org/10.1038/s41580-018-0003-4.

### Discussion of Examples 1 to 9

Self-assembling chemicals containing amphiphilic lipids have been investigated as carriers of biologically active molecules, including small-molecule drugs and nucleic acids (Reference Material 21 below). However, the biological activity of self-assembling chemicals themselves has not been thoroughly investigated. Reported biologically active self-organizing molecules usually use peptide building blocks, but most of them induce cell death (Reference Materials 22 to 27 below). More recently, Hamilton and his colleagues reported a peptidomimetic self-organaization that attenuates the cytotoxicity of amyloid-β fibers (Reference Material 28 below). However, so far, only a limited number of studies have been reported using non-peptide, self-organizing small molecules to control biological processes (Reference Materials 14 and 29-32 below). This lack of exploration is due to unfavorable physical and pharmacological properties for classical medicinal chemistry (Reference Material 18). This study demonstrates new aspects of self-organizing chemicals as a trapping agent or "molecular absorbent" for cytotoxic denatured proteins within cells. To the best of the inventors' knowledge, selfonamide (compound 6) represents the first self-organizing organic molecule that traps and potentially removes denatured proteins to alleviate ER stress-induced toxicity.

Denatured proteins are involved in a variety of diseases, including neurodegenerative diseases and metabolic diseases. Chemical chaperones have been considered as potential therapeutics for these diseases but have not been successful in clinical trials (NPL 2). A significant drawback of existing chemical chaperones is that they are effective only at the early stages of protein denaturation, having a limited ability to address already-denatured proteins. This characteristic of chemical chaperones differs from that of molecular absorbents (the compounds of the present disclosure used in the Examples) revealed in the inventors' study. Molecular absorbents were unable to prevent heat-induced protein denaturation, but appeared to preferentially co-assemble with pre-denatured proteins. Similar selective co-assembly with denatured proteins has been reported for cholesteryl-group-containing pullulan (CHP) hydrogel nanoparticles (Reference Materials 33 and 34 below). Research also reports various types of nanoparticles to promote protein refolding or reduce protein aggregation (Reference Materials 39 and 40 below). In general, denatured or misfolded proteins that expose hydrophobic amino acids on their surface are prone to hydrophobic interactions and formation of insoluble protein aggregates (Reference Material. 41). This characteristic may be involved in the co-assembly of denatured proteins with selfonamide. Further study is required to gain a deeper understanding of the selective co-assembly with denatured proteins, which may ultimately enable the rational design of molecular absorbents.

A unique characteristic of selfonamide is that its co-assembly with denatured proteins appears to be removed by autophagy. This finding raises several important questions that can serve as the basis for future in-depth investigations. A major issue is how the co-assembly of selfonamide and denatured proteins is recognized by the cellular autophagy mechanism. The inventors speculate that the clearance of biomolecular condensates or misfolded proteins may be mediated by selective autophagy, which has been shown to play a role in maintaining cellular homeostasis and preventing aggregation-induced diseases (Reference Material 42 below). Selfonamide assemblies may accelerate the selective autophagy of denatured proteins by functioning as a phase-separated scaffold for both denatured proteins and autophagy receptors.

The study by the present inventors demonstrated that the full activity of selfonamide requires the mammalian selective autophagy receptor p62 (Reference Materials 43 to 45 below). However, denatured proteins trapped in selfonamide are not necessarily ubiquitinated. How p62 is involved in the action of selfonamide remains to be elucidated.

Selfonamide (compound 6) and its derivatives have numerous potential medical applications. In this regard, various proteins are denatured in a variety of pathological conditions, such as neurological diseases (Reference Materials 6 and 7 below), metabolic diseases (Reference Material 9 below), and ocular diseases (Reference Material 46 below). In particular, self-organizing molecules may be suitable for ophthalmic applications in which eye drops or local injections are possible. Preclinical trials of such applications are currently underway. In addition, further clinical application of self-assembling molecular absorbents requires careful optimization and evaluation. The pharmacodynamics, formulation, and safety for each indication need to be evaluated. The novel mechanism of action of the compound of the present disclosure indicates that self-assembling molecules (the compound of the present disclosure) may serve as a rich source of "molecular absorbents" that reduce the toxicity of denatured proteins and protein aggregates.
Reference Material 21: Roesler, A.; Vandermeulen, G.W.M.; Klok, H.A. Advanced Drug Delivery Devices via Self-Assembly of Amphiphilic Block Copolymers. Adv Drug Deliver Rev 2012, 64, 270-279. https://doi.org/10.1016/j.addr.2012.09.026.
Reference Material 22: Tanaka, A.; Fukuoka, Y.; Morimoto, Y.; Honjo, T.; Koda, D.; Goto, M.; Maruyama, T. Cancer Cell Death Induced by the Intracellular Self-Assembly of an Enzyme-Responsive Supramolecular Gelator. J Am Chem Soc 2015, 137 (2), 770-775. https://doi.org/10.1021/ja510156v.
Reference Material 23: Zhou, J.; Du, X.; Yamagata, N.; Xu, B. Enzyme-Instructed Self-Assembly of Small D-Peptides as a Multiple-Step Process for Selectively Killing Cancer Cells. J Am Chem Soc 2016, 138 (11), 3813-3823. https://doi.org/10.1021/jacs.5b13541.
Reference Material 24: Feng, Z.; Wang, H.; Chen, X.; Xu, B. Self-Assembling Ability Determines the Activity of Enzyme-Instructed Self-Assembly for Inhibiting Cancer Cells. J Am Chem Soc 2017, 139 (43), 15377-15384. https: //doi.org/10.1021/jacs.7b07147.
Reference Material 25: Shoshan, M.S.; Vonderach, T.; Hattendorf, B.; Wennemers, H. Peptide-Coated Platinum Nanoparticles with Selective Toxicity against Liver Cancer Cells. Angewandte Chemie Int Ed 2019, 58 (15), 4901-4905. https://doi.org/10.1002/anie.201813149.
Reference Material 26: Hu, L.; Li, Y.; Lin, X.; Huo, Y.; Zhang, H.; Wang, H. Structure-Based Programming of Supramolecular Assemblies in Living Cells for Selective Cancer Cell Inhibition. Angewandte Chemie Int Ed 2021, 60 (40), 21807-21816. https://doi.org/10.1002/anie.202103507.
Reference Material 27: Zhang, L.; Jing, D.; Jiang, N.; Rojalin, T.; Baehr, C.M.; Zhang, D.; Xiao, W.; Wu, Y.; Cong, Z.; Li, J.J.; Li, Y.; Wang, L.; Lam, K.S. Transformable Peptide Nanoparticles Arrest HER2 Signalling and Cause Cancer Cell Death in Vivo. Nat Nanotechnol 2020, 15 (2), 145-153. https://doi.org/10.1038/s41565-019-0626-4.
Reference Material 28: Maity, D.; Howarth, M.; Vogel, M.C.; Magzoub, M.; Hamilton, A.D. Peptidomimetic-Based Vesicles Inhibit Amyloid-β Fibrillation and Attenuate Cytotoxicity. J Am Chem Soc 2021, 143 (8), 3086-3093. https://doi.org/10.1021/jacs.0c09967.
Reference Material 14: Jin, S.; Vu, H.T.; Hioki, K.; Noda, N.; Yoshida, H.; Shimane, T.; Ishizuka, S.; Takashima, I.; Mizuhata, Y.; Pe, K.B.; Ogawa, T.; Nishimura, N.; Packwood, D.; Tokitoh, N.; Kurata, H.; Yamasaki, S.; Ishii, K.J.; Uesugi, M. Discovery of Self-Assembling Small Molecules as Vaccine Adjuvants. Angewandte Chemie Int Ed 2021, 60 (2), 961-969.
Reference Material 29: Zorn, J.A.; Wille, H.; Wolan, D.W.; Wells, J.A. Self-Assembling Small Molecules Form Nanofibrils That Bind Procaspase-3 To Promote Activation. J Am Chem Soc 2011, 133 (49), 19630-19633. https://doi.org/10.1021/ja208350u.
Reference Material 30: Takemoto, N.; Suehara, T.; Frisco, H. L.; Sato, S.; Sezaki, T.; Kusamori, K.; Kawazoe, Y.; Park, S.M.; Yamazoe, S.; Mizuhata, Y.; Inoue, R.; Miller, G.J.; Hansen, S.U.; Jayson, G.C.; Gardiner, J.M.; Kanaya, T.; Tokitoh, N.; Ueda, K.; Takakura, Y.; Kioka, N.; Nishikawa, M.; Uesugi, M. Small-Molecule-Induced Clustering of Heparan Sulfate Promotes Cell Adhesion. J Am Chem Soc 2013, 135 (30), 11032-11039. https://doi.org/10.1021/ja4018682.
Reference Material 31: Lee, Y.; Kim, H.; Kang, S.; Lee, J.; Park, J.; Jon, S. Bilirubin Nanoparticles as a Nanomedicine for Anti-inflammation Therapy. Angewandte Chemie Int Ed 2016, 55 (26), 7460-7463. https://doi.org/10.1002/anie.201602525.
Reference Material 32: Slabicki, M.; Yoon, H.; Koeppel, J.; Nitsch, L.; Burman, S.S.R.; Genua, C.D.; Donovan, K.A.; Sperling, A.S.; Hunkeler, M.; Tsai, J. M.; Sharma, R.; Guirguis, A.; Zou, C.; Chudasama, P.; Gasser, J.A.; Miller, P.G.; Scholl, C.; Froehling, S.; Nowak, R.P.; Fischer, E. S.; Ebert, B.L. Small-Molecule-Induced Polymerization Triggers Degradation of BCL6. Nature 2020, 588 (7836), 164-168. https://doi.org/10.1038/s41586-020-2925-1.
Reference Material 33: Akiyoshi, K.; Sasaki, Y.; Sunamoto, J. Molecular Chaperone-Like Activity of Hydrogel Nanoparticles of Hydrophobized Pullulan: Thermal Stabilization with Refolding of Carbonic Anhydrase B. Bioconjugate Chem 1999, 10 (3), 321-324. https://doi.org/10.1021/bc9801272.
Reference Material 34: Sawada, S.I.; Nomura, Y.; Aoyama, Y.; Akiyoshi, K. Heat Shock Protein-like Activity of a Nanogel Artificial Chaperone for Citrate Synthase. J Bioact Compat Pol 2006, 21 (6), 487-501. https://doi.org/10.1177/0883911506070819.
Reference Material 39: Huang, F.; Wang, J.; Qu, A.; Shen, L.; Liu, J.; Liu, J.; Zhang, Z.; An, Y.; Shi, L. Maintenance of Amyloid β Peptide Homeostasis by Artificial Chaperones Based on Mixed-Shell Polymeric Micelles. Angewandte Chemie Int Ed 2014, 53 (34), 8985-8990. https://doi.org/10.1002/anie.201400735.
Reference Material 40: Luo, Q.; Lin, Y.X.; Yang, P.P.; Wang, Y.; Qi, G.B.; Qiao, Z.Y.; Li, B.N.; Zhang, K.; Zhang, J.P.; Wang, L.; Wang, H. A Self-Destructive Nanosweeper That Captures and Clears Amyloid β-Peptides. Nat Commun 2018, 9 (1), 1802. https://doi.org/10.1038/s41467-018-04255-z.
Reference Material 41: Herczenik, E.; Gebbink, M.F.B.G. Molecular and Cellular Aspects of Protein Misfolding and Disease. Faseb J 2008, 22 (7), 2115-2133. https://doi.org/10.1096/fj.07-099671.
Reference Material 42: Deng, Z.; Purtell, K.; Lachance, V.; Wold, M.S.; Chen, S.; Yue, Z. Autophagy Receptors and Neurodegenerative Diseases. Trends Cell Biol 2017, 27 (7), 491-504. https://doi.org/10.1016/j.tcb.2017.01.001.
Reference Material 43: Jakobi, A.J.; Huber, S.T.; Mortensen, S.A.; Schultz, S.W.; Palara, A.; Kuhm, T.; Shrestha, B.K.; Lamark, T.; Hagen, W.J.H.; Wilmanns, M.; Johansen, T.; Brech, A.; Sachse, C. Structural Basis of P62/SQSTM1 Helical Filaments and Their Role in Cellular Cargo Uptake. Nat Commun 2020, 11 (1), 440. https://doi.org/10.1038/s41467-020-14343-8.
Reference Material 44: Sun, D.; Wu, R.; Zheng, J.; Li, P.; Yu, L. Polyubiquitin Chain-Induced P62 Phase Separation Drives Autophagic Cargo Segregation. Cell Res 2018, 28 (4), 405-415. https://doi.org/10.1038/s41422-018-0017-7.
Reference Material 45: Zaffagnini, G.; Savova, A.; Danieli, A.; Romanov, J.; Tremel, S.; Ebner, M.; Peterbauer, T.; Sztacho, M.; Trapannone, R.; Tarafder, A. K.; Sachse, C.; Martens, S. P62 Filaments Capture and Present Ubiquitinated Cargos for Autophagy. Embo J 2018, 37 (5), e98308. https://doi.org/10.15252/embj.201798308.
Reference Material 6: Romoli, M.; Sen, A.; Parnetti, L.; Calabresi, P.; Costa, C. Amyloid-β: A Potential Link between Epilepsy and Cognitive Decline. Nat Rev Neurol 2021, 1-17. https://doi.org/10.1038/s41582-021-00505-9.
Reference Material 7: Aarsland, D.; Creese, B.; Politis, M.; Chaudhuri, K.R.; Ffytche, D.H.; Weintraub, D.; Ballard, C. Cognitive Decline in Parkinson Disease. Nat Rev Neurol 2017, 13 (4), 217-231. https://doi.org/10.1038/nrneurol.2017.27. Reference Material 9: Gomes, C. M. Protein Misfolding in Disease and Small Molecule Therapies. Curr Top Med Chem 2013, 12 (22), 2460-2469. https://doi.org/10.2174/1568026611212220002.
Reference Material 46: Makley, L.N.; McMenimen, K.A.; DeVree, B.T.; Goldman, J.W.; McGlasson, B.N.; Rajagopal, P.; Dunyak, B.M.; McQuade, T.J.; Thompson, A.D.; Sunahara, R.; Klevit, R.E.; Andley, U.P.; Gestwicki, J.E. Pharmacological Chaperone for α-Crystallin Partially Restores Transparency in Cataract Models. Science 2015, 350 (6261), 674-677. https://doi.org/10.1126/science.aac9145.
Reference Material 47: Ikeda et al., Nat Commun, 2022.

### Example 10: Production of Compounds XD8 to XD28 and Confirmation of Endoplasmic Reticulum Stress Inhibition Activity

Furthermore, compounds XD8 to XD28, which are derivatives of compound 1, were produced and measured for their biological activity. The structures of compounds XD8 to XD28 are as shown in the following chemical formulas. Compounds XD8 to XD28 were produced in the same manner as in the production of compound 1, except that a starting material having a structure corresponding to each of compounds XD8 to XD28 was used instead of TD-4C8. Compounds XD8 to XD28 were successfully produced, as their structures were confirmed by instrumental analysis in the same manner as for compounds 1 to 6. It was also confirmed by HPLC that the target compounds were obtained with high purity.

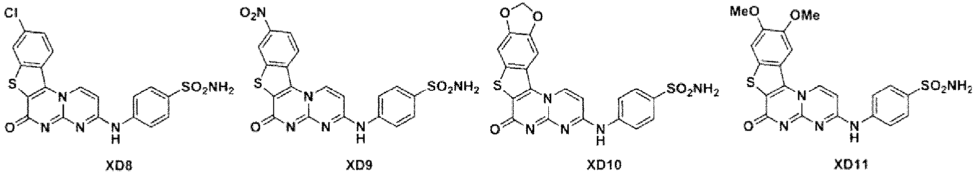

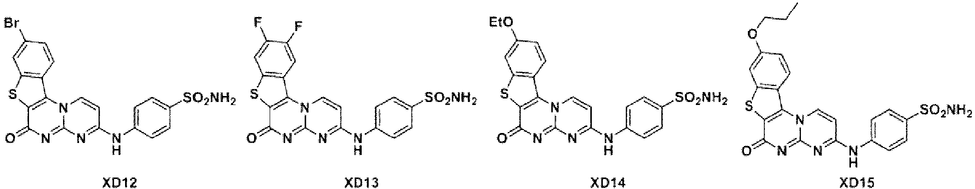

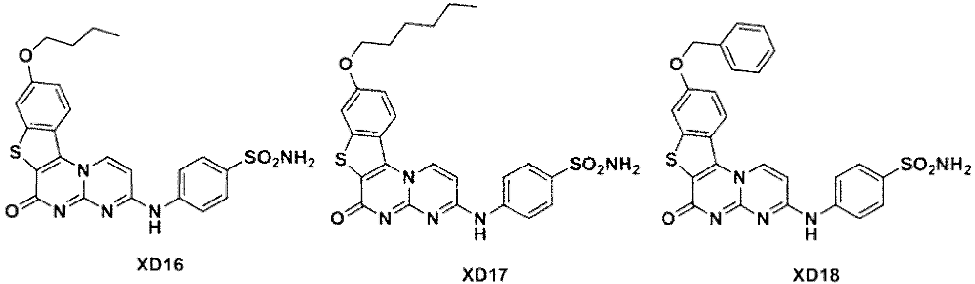

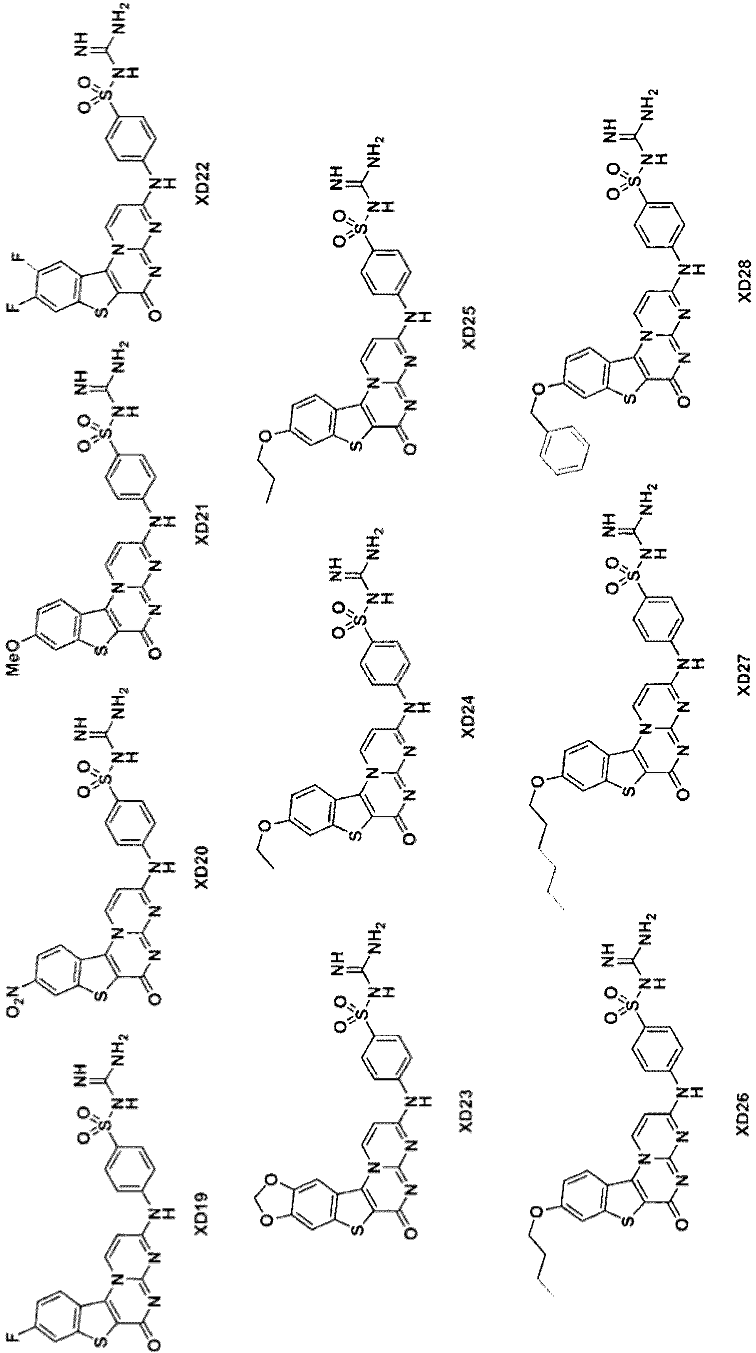

The following shows the calculated values and measured values of (ESI+) [M+H] of the molecular weights of compounds XD8 to XD28 by HRMS (LC-MS).
XD8: HRMS (ESI+) [M+H] calcd, 458.0143; found, 458.0147 (0.4 mmu) XD9: HRMS (ESI+) [M+H] calcd, 469.0383; found, 469.0384 (0.1 mmu) XD10: HRMS (ESI+) [M+H] calcd, 468.0431; found, 468.0433 (0.2 mmu)
XD11: HRMS (ESI+) [M+H] calcd, 484.0744; found, 484.0745 (0.1 mmu)
XD12: HRMS (ESI+) [M+H] calcd, 501.9638; found, 501.9638 (0.0 mmu)
XD13: HRMS (ESI+) [M+H] calcd, 460.0344; found, 460.0345 (0.1 mmu)
XD14: HRMS (ESI+) [M+H] calcd, 468.0795; found, 468.0792 (-0.3 mmu)
XD15: HRMS (ESI+) [M+H] calcd, 482.0951; found, 482.0953 (0.2 mmu)
XD16: HRMS (ESI+) [M+H] calcd, 496.1108; found, 496.1109 (0.1 mmu)
XD17: HRMS (ESI+) [M+H] calcd, 524.1421; found, 524.1421 (0.0 mmu)
XD18: HRMS (ESI+) [M+H] calcd, 530.0951; found, 530.0952 (0.1 mmu)
XD19: HRMS (ESI+) [M+H] calcd, 484.0656; found, 484.0657 (0.1 mmu)
XD20: HRMS (ESI+) [M+H] calcd, 511.0601; found, 511.0604 (0.3 mmu)
XD21: HRMS (ESI+) [M+H] calcd, 496.0856; found, 496.0853 (-0.3 mmu)
XD22: HRMS (ESI+) [M+H] calcd, 502.0562; found, 502.0559 (-0.3 mmu)
XD23: HRMS (ESI+) [M+H] calcd, 510.0649; found, 510.0651 (0.2 mmu)
XD24: HRMS (ESI+) [M+H] calcd, 510.1013; found, 510.1017 (0.4 mmu)
XD25: HRMS (ESI+) [M+H] calcd, 524.1169; found, 524.1171 (0.2 mmu)
XD26: HRMS (ESI+) [M+H] calcd, 538.1326; found, 538.1331 (0.5 mmu)
XD27: HRMS (ESI+) [M+H] calcd, 566.1639; found, 566.1640 (0.1 mmu)
XD28: HRMS (ESI+) [M+H] calcd, 572.1169; found, 572.1173 (0.4 mmu)

Produced compounds XD8 to XD28 were subjected to a tunicamycin assay in the same manner as in Examples 1 to 9. Specifically, HEK293 cells were first seeded in a 96-well plate (1×10⁴ cells/well) and incubated at 37°C for 2 hours. The medium was then replaced with 100 µL of DMEM (10% FBS) containing a self-assembling chemical (each of compounds XD8 to XD28) and 1% DMSO. After incubation with any of compounds XD8 to XD28 at a concentration of 5.0 µM, 25 µM, or 100 µM for 12 to 18 hours, HEK293 cells were treated with tunicamycin (5 µg/mL) for 48 hours, followed by measuring cell viability by using a WST-8 cell counting kit (Dojindo Laboratories). As positive controls, a sample incubated with PBA (2 mM) instead of compounds XD8 to XD28, a sample incubated with compound 1 (100 µM) instead of compounds XD8 to XD28, and a sample incubated with compound 6 (25 µM) instead of compounds XD8 to XD28 were used.

Tables 1 and 2 below summarize the results of the tunicamycin assay of compounds XD8 to XD28. The term "Efficacy" denotes ER (endoplasmic reticulum) stress inhibition activity. The term "Toxicity" denotes cytotoxicity. In the ER stress inhibition activity (Efficacy), "-" indicates no activity, "+" indicates weak activity, and "++" indicates strong activity. In addition, "XD7" in Table 1 below represents compound 6. That is, XD7 and compound 6 are the same compound.

**Table 1**

| | **Efficacy** | | | **Toxicity** |
|---|---|---|---|---|
| | **5 µM** | **25 µM** | **100 µM** | |
| XD7 (-OMe) | - | + + | + + | - |
| XD8 (-Cl) | - | + | + + | + (100 µM) |
| XD9 (-NO₂) | - | + + | + | - |
| XD10 (1,3-dioxol) | - | - | - | - |
| XD11 (-di OMe) | - | - | + + | - |
| XD12 (-Br) | - | + | + + | - |
| XD13 (-di F) | - | + + | + + | - |
| XD14 (-OEt) | - | - | - | - |
| XD15 (-On-Pr) | - | - | - | - |
| XD16 (-On-Bu) | - | - | - | - |
| XD17 (-On-Hex) | - | - | + | - |
| XD18 (-OBn) | - | - | - | - |

**Table 2**

| 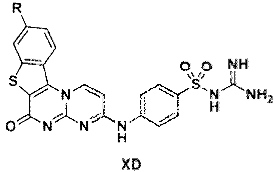 | **Efficacy** | | | **Toxicity** |
|---|---|---|---|---|
| | **5 µM** | **25 µM** | **100 µM** | |
| XD19 (-F) | - | - | - | + (100 µM) |
| XD20 (-NO₂) | - | - | + | + (100 µM) |
| XD21 (-OMe) | - | + + | + + | - |
| XD22 (-di F) | - | - | - | - |
| XD23 (1,3-dioxol) | - | - | - | - |
| XD24 (-OEt) | - | - | - | + (100 µM) |
| XD25 (-OPr) | - | + + | + | + (100 µM) |
| XD26 (-OBu) | - | + | ++ | - |
| XD27 (-Ohex) | - | - | - | + (100 µM) |
| XD28 (-OBn) | - | - | ++ | - |

The graphs in Figs. 12 to 15 show the original data of the data shown in Tables 1 and 2.

Furthermore, it was confirmed according to the same method as for compounds 1 to 6 that XD8 to XD28 also have self-assembly activity (self-assembling ability), like compounds 1 to **6.** Compounds 1 to 6 and compounds XD8 to XD28 are all considered to have self-assembly activity due to a common structure falling within the scope of chemical formula (I) described above. Figs. 33 and 34 are diagrams showing dynamic light scattering (DLS) of the molecules of compounds XD8 to XD28, and show the self-assembly activity of compounds XD8 to XD28. In Fig. 33, "Z-average" represents the average size of particles. In Fig. 34, "Derived count rate (kcps)" represents derived count rate. In Figs. 33 and 34, error bars are s.d. (n=3). As shown in Figs. 33 and 34, compounds XD8 to XD28 were all confirmed to have self-assembly activity, to a greater or lesser degree.

### Examples 11 to 13: Endoplasmic Reticulum Stress Inhibition Activity of Compound 1 and Compound 6

In Examples 11 to 13, the endoplasmic reticulum stress inhibition activity of compound 1 and compound 6 was further confirmed. In the figures and tables (Figs. 16 to 19, etc.), "X441A" represents compound 1, and "XD7" represents compound 6. That is, X441A and compound 1 are the same compound, and XD7 and compound 6 are the same compound.

### Preparation Example: Preparation of Immortalized Corneal Endothelial Cell Line (iFECD) Model Derived from Patient with Fuchs' Endothelial Corneal Dystrophy

In this Example, an immortalized corneal endothelial cell line (iFECD) was prepared from corneal endothelial cells derived from a patient with Fuchs' endothelial corneal dystrophy.

### Culture Method

Corneal endothelial cells were mechanically detached together with the basement membrane from a research cornea purchased from SightLife^{™}, and then the cells were peeled off from the basement membrane using collagenase and collected, followed by primary culture. The medium used as a base medium was Opti-MEM I Reduced-Serum Medium, Liquid (Invitrogen, Catalog No: 31985-070) supplemented with 8% FBS (Biowest, Catalog No: S1820-500), 200 mg/ml CaCl₂·2H₂O (Sigma, Catalog No: C7902-500G), 0.08% chondroitin sulfate (Sigma, Catalog No: C9819-5G), 20 µg/ml ascorbic acid (Sigma, Catalog No: A4544-25G), 50 µg/ml gentamicin (Invitrogen, Catalog No: 15710-064), and 5 ng/ml EGF (Invitrogen, Catalog No: PHG0311), which was conditioned for 3T3 feeder cells. The cells were cultured in the base medium supplemented with SB431542 (1 µmol/L) and SB203580 (4-(4-fluorophenyl)-2-(4-methylsulfonylphenyl)-5(4-pyridyl)imidazole <4-[4-(4-fluorophenyl)-2-(4-methylsulfinylphenyl)-1H-imidazol-5-yl]pyridine) (1 µmol/L) (also referred to as "SB203580+SB431542+3T3 conditioned medium" in the present specification).

### Acquisition Method

Corneal endothelial cells were obtained from three human patients who had undergone corneal endothelium transplantation (Descemet membrane endothelial keratoplasty: DMEK) due to bullous keratopathy resulting from clinically diagnosed Fuchs' endothelial corneal dystrophy, with written informed consent and approval from the ethics committee. During DMEK, pathological corneal endothelial cells and the basement membrane (Descemet's membrane) were mechanically peeled off and immersed in Optisol-GS (corneal preservative solution, Bausch & Lomb). Thereafter, collagenase treatment was performed, and the corneal endothelial cells were enzymatically collected and cultured in SB203580+SB431542+3T3 conditioned medium. SV40 large T antigen and hTERT genes from the cultured corneal endothelial cells derived from a patient with Fuchs' endothelial corneal dystrophy, which were amplified by PCR, were introduced into a lentiviral vector (pLenti6.3_V5-TOPO; Life Technologies Inc.). Subsequently, 293T cells (RCB2202; Riken Bioresource Center, Ibaraki, Japan) were transfected with the lentiviral vector together with three types of helper plasmids (pLP1, pLP2, and pLP/VSVG; Life Technologies Inc.) by using a transfection reagent (Fugene HD; Promega Corp., Madison, WI). After 48 hours of infection, the virus-containing culture supernatant was collected and added to a culture fluid of cultured corneal endothelial cells derived from a patient with Fuchs' endothelial corneal dystrophy to introduce the SV40 large T antigen and hTERT genes by using 5 µg/ml polybrene. A phase-contrast microscope image of an immortalized corneal endothelial cell line (iFECD) derived from a patient with Fuchs' endothelial corneal dystrophy was confirmed. For the control, corneal endothelial cells cultured from research corneas imported from the Seattle Eye Bank were immortalized in the same manner to prepare an immortalized cell line of normal corneal endothelial cells (iHCEC). In an image of the immortalized corneal endothelial cell line (iFECD) viewed under a phase-contrast microscope, all iFECD cells had monolayered polygonal morphology, as with normal corneal endothelial cells. iFECD was maintained and cultured in Dulbecco's modified Eagle medium (DMEM) + 10% fetal bovine serum (FBS).

### Example 11-1: Inhibition Effect of Compound 1 on Cell Damage Induced by TGF-β2

In this Example, the effect of compound 1, which is an endoplasmic reticulum stress inhibitor, on ocular cell damage was demonstrated.

### Materials and Methods

The medium was removed from the culture dish, in which iFECD was being cultured, and 1×PBS(-) that had been pre-warmed to 37°C was added to wash the cells. This procedure was performed twice. Again, 1×PBS(-) was added, and the cells were incubated at 37°C (5% CO₂) for 3 minutes. After the PBS(-) was removed, 0.05% Trypsin-EDTA (Nacalai Tesque, 32778-34) was added, and the cells were incubated at 37°C (5% CO₂) for 5 minutes. Thereafter, the cells were suspended in a medium and centrifuged at 1500 rpm for 3 minutes to recover the cells. The medium used was DMEM (Nacalai Tesque, 08456-36) + 10% FBS (Biowest, S1820-500) + 1% P/S (Nacalai Tesque, 26252-94).

iFECD cells were seeded in a 12-well plate at a density of 0.5 x 10⁵ cells per well and cultured at 37°C (5% CO₂) for 24 hours (the medium used was DMEM + 10% FBS + 1% P/S). After 24 hours, the medium was removed, and compound 1 was added, followed by culturing the cells for 24 hours (the medium used was DMEM + 2% FBS + 1% P/S). After 24 hours, the medium was removed, and a medium containing 10 ng/ml recombinant human TGF-β (Wako, 200-19911) and compound 1 was added, followed by culturing the cells for 24 hours (the medium used was DMEM + 2% FBS + 1% P/S). After 30 hours, cell morphology and apoptosis were observed with a phase-contrast microscope.

### Results

### Compound 1, which is an endoplasmic reticulum stress inhibitor, inhibits TGF-β2-induced cell damage.

Fig. 16 shows the results. When iFECD was stimulated with recombinant human TGF-β2 in the absence of compound 1, which is an endoplasmic reticulum stress inhibitor, the cells were found to have been noticeably damaged. On the other hand, pretreatment with compound 1 limited damage to corneal endothelial cells. Thus, compound 1 was found to inhibit the cell damage induced by recombinant human TGF-β2.

### Example 11-2: Inhibition Effect of Compound 6 on Cell Damage Induced by TGF-β2

In this Example, the effect of compound 6, which is an endoplasmic reticulum stress inhibitor, on ocular cell damage was demonstrated.

### Materials and Methods

The same method as described in the Materials and Methods section in Example 11-1 was used, except that compound 6 was used instead of compound 1, and the amount of iFECD seeded in a 12-well plate was changed from 0.5 x 10⁵ cells per well to 0.9 x 10⁵ cells per well.

### Results

### Compound 6, which is an endoplasmic reticulum stress inhibitor, inhibits TGF-β2-induced cell damage.

Fig. 17 shows the results. When iFECD was stimulated with recombinant human TGF-β2 in the absence of compound 6, which is an endoplasmic reticulum stress inhibitor, the cells were found to have been noticeably damaged. On the other hand, pretreatment with compound 6 limited damage to corneal endothelial cells. Thus, compound 6 was found to inhibit cell damage induced by recombinant human TGF-β2.

### Example 12-1: Inhibition Effect of Compound 1 on Caspase Activity Induced by TGF-β2

In this Example, the effect of compound 1, which is endoplasmic reticulum stress inhibitor, on caspase activity was demonstrated.

### Materials and Methods

The same method as described in the Materials and Methods section in Example 11-1 was used.

After observation, western blotting of proteins was performed according to the following procedure.

### 1) Protein Recovery

In order to collect floating cells and dead cells, the medium was collected on ice, and the solution resulting from washing cells with 1 x PBS(-) twice was also collected and centrifuged at 4°C at 800 g for 12 minutes, followed by discarding the supernatant, thereby obtaining a precipitate. Proteins were extracted from the washed cells by adding a protein extraction buffer (RIPA; 50 mM Tris-HCl (pH of 7.4), 150 mM NaCl, 1 mM EDTA, 0.1% SDS, 0.5% DOC, 1% NP-40) on ice. Thereafter, the precipitate obtained after centrifugation of the floating cells and dead cells was suspended together and extracted. The collected liquid was pulverized in cold water for 30 seconds three times with an ultrasonic device (BIORUPTOR, Tosho Denki) and then centrifuged at 4°C at 15000 rpm for 10 minutes to collect the protein supernatant.

### 2) Western Blotting

5 to 8 µg of the extracted proteins were separated by SDS-PAGE and transferred to a nitrocellulose membrane. The primary antibodies used were rabbit anti-Caspase 3 antibody (Cell Signaling, 9662), rabbit anti-PARP antibody (Cell Signaling, 9542), and mouse anti-GAPDH antibody (MBL, M171-3). The secondary antibodies used were peroxidase-labeled anti-rabbit antibody and anti-mouse antibody (GE Healthcare Biosciences, NA931V, NA934V). The primary antibodies were diluted as follows: rabbit anti-Caspase 3 antibody, 1000-fold; rabbit anti-PARP antibody, 1000-fold; and mouse anti-GAPDH antibody, 5000-fold. The secondary antibodies were diluted 5000-fold. For detection, Chemi Lumi ONE Ultra (Nacalai Tesque, 11644-40) was used. The intensity of the detected bands was analyzed using a LAS-4000mini lumino image analyzer (Fujifilm) and ImageQuant^{™} software (GE Healthcare).

### Results

### Compound 1, which is an endoplasmic reticulum stress inhibitor, inhibits TGF-β2-induced caspase activity.

Fig. 18 shows the results of western blotting on caspases. When iFECD was stimulated with recombinant human TGF-β2 in the absence of compound 1, cleaved active caspase 3 of about 17 kDa (about 17 kDa) was observed. However, in the group in which compound 1 was added, cleaved active caspase 3 was hardly detected. Thus, western blotting analysis demonstrated that compound 1 inhibits caspase activation induced by recombinant human TGF-β2.

### Example 12-2: Inhibition Effect of Compound 6 on Caspase Activity Induced by TGF-β2.

In this Example, the effect of compound 6, which is endoplasmic reticulum stress inihibitor, on caspase activity was demonstrated.

### Materials and Methods

The same method as described in the Materials and Methods section in Example 11-1 was performed, except that compound 6 was used instead of compound 1, and the amount of iFECD seeded in a 12-well plate was changed from 0.5 x 10⁵ cells per well to 0.9 x 10⁵ cells per well. That is, the experiment was performed in exactly the same manner as described in the Materials and Methods section in Example 11-2.

After observation, western blotting of proteins was performed. The methods and procedures for performing western blotting of proteins were the same as those described in 1) Protein Recovery and 2) Western Blotting described in Example 12-1.

### Results

### Compound 6, which is an endoplasmic reticulum stress inhibitor, inhibits TGF-β2-induced caspase activity.

Fig. 19 shows the results of western blotting on caspases. When iFECD was stimulated with recombinant human TGF-β2 in the absence of compound 6, cleaved active caspase 3 of about 17 kDa (about 17 kDa) was observed. However, in the group in which compound 6 was added, cleaved active caspase 3 was hardly detected. Thus, western blotting analysis demonstrated that compound 6 inhibits caspase activation induced by recombinant human TGF-β2.

### Example 13-1: Inhibition Effect of Compound 1 on Fibronectin Production Induced by TGF-β2

In this Example, the inhibition effect of compound 1, which is an endoplasmic reticulum stress inhibitor, on fibronectin production induced by TGF-β2 was demonstrated.

### Materials and Methods

The method used was the same as described in the Materials and Methods section in Examples 11-1 and 12-1.

After observation, western blotting of proteins was performed according to the following procedure.

### 1) Protein Recovery

Protein recovery was performed according to the same method and procedure as described in 1) Protein Recovery in Example 12-1.

### 2) Western Blotting

5 µg of the extracted proteins were separated by SDS-PAGE and transferred to a nitrocellulose membrane. The primary antibodies used were mouse anti-fibronectin antibody (BD Bioscience, 610077) and mouse anti-GAPDH antibody (MBL, M171-3). The secondary antibodies used were peroxidase-labeled anti-rabbit antibody and anti-mouse antibody (GE Healthcare Biosciences, NA931V, NA934V). The primary antibodies were diluted as follows: mouse anti-fibronectin antibody, 15,000-fold; and mouse anti-GAPDH antibody, 5,000-fold. The secondary antibodies were diluted 5,000-fold. For detection, Chemi Lumi ONE Ultra (Nacalai Tesque, 11644-40) was used. The intensity of the detected bands was analyzed using a LAS-4000mini lumino image analyzer (Fujifilm) and ImageQuant^{™} software (GE Healthcare).

### Results

### Compound 1 inhibits TGF-β2-induced fibronectin production.

Fig. 18 shows the results. When iFECD was stimulated with recombinant human TGF-β2 in the absence of compound 1, fibronectin production was observed in iFECD. However, in the group in which compound 1 was added, almost no production of fibronectin was observed. Thus, western blotting analysis demonstrated that compound 1 limits the expression levels of fibronectin induced by recombinant human TGF-β2.

### Example 13-2: Inhibition Effect of Compound 6 on Fibronectin Production Induced by TGF-β2

In this Example, the inhibition effect of compound 6, which is an endoplasmic reticulum stress inhibitor, on fibronectin production induced by TGF-β2 was demonstrated.

### Materials and Methods

The same method as described in the Materials and Methods section in Example 11-1 was performed, except that compound 6 was used instead of compound 1, and the amount of iFECD seeded in a 12-well plate was changed from 0.5 x 10⁵ cells per well to 0.9 x 10⁵ cells per well. That is, the experiment was performed in exactly the same manner as described in the Materials and Methods section of Examples 11-2 and 12-2.

After observation, western blotting of proteins was performed. The methods and procedures for performing western blotting of proteins were the same as those described in 1) Protein Recovery and 2) Western Blotting described in Example 13-1.

### Results

### Compound 6 inhibits TGF-β2-induced fibronectin production.

Fig. 19 shows the results. When iFECD was stimulated with recombinant human TGF-β2 in the absence of compound 6, fibronectin production was observed in iFECD. However, in the group in which compound 6 was added, almost no production of fibronectin was observed. Thus, western blotting analysis demonstrated that compound 6 limits the expression levels of fibronectin induced by recombinant human TGF-β2.

In Fuchs' endothelial corneal dystrophy, excessive production of extracellular matrix such as fibronectin and its deposition on Descemet's membrane cause damage, including Descemet's membrane thickening and formation of guttae. These kinds of damage typically begin to develop in patients with Fuchs' endothelial corneal dystrophy in their 30s and 40s and progress throughout their lifetime. As the condition progresses, visual impairments, such as blurred vision, halos, glare, and decreased vision, occur. Furthermore, in Fuchs' endothelial corneal dystrophy, corneal endothelial cells are continuously damaged, but corneal transparency is maintained as long as the cell density remains above about 1000 cells/mm², with the remaining corneal endothelia compensating for their pump function. If the cell density falls below about 1000 cells/mm², anterior aqueous humor enters the cornea, causing corneal edema and resulting in visual impairment. Thus, in patients with Fuchs' endothelial corneal dystrophy, visual impairment occurs mainly due to two causes: excessive production of extracellular matrix and corneal endothelial cell death. The action of the caspase inhibitor according to the present disclosure is considered to be particularly useful for treating Fuchs' endothelial corneal dystrophy because it works to inhibit both extracellular matrix production and corneal endothelial cell death.

As shown by the results of Examples 11 to 13, the present disclosure can provide a medicament for treating or preventing corneal endothelial damage caused by, for instance, overexpression of transforming growth factor-β (TGF-β) signaling and/or extracellular matrix in corneal endothelial cells. In particular, the present disclosure can provide a medicament for treating or preventing corneal endothelial damage caused by Fuchs' endothelial corneal dystrophy. The present disclosure is applicable, for example, in industries involved in technologies related to formulations based on such a technique (e.g., drug development).

### Example 14: Inhibition of Myopia Progression by X441A Ophthalmic Solution

In this Example, whether a X441A ophthalmic solution can inhibit the progression of myopia was demonstrated.

### Method

A -30 diopter (unit of refractive index: D) lens was attached to the right eye of 3-week-old male C57BL6J mice, while only a lens frame was attached to the left eye, which served as a control eye. The lenses were worn for three weeks, and the refractive index and axial length of eyes were measured before and after attachment of the lenses. Concurrently with myopia induction, an eye drop, which is a 0.025% solution of X441A in PBS, was administered by instillation once a day every day. PBS was administered by eye drop to the control group (group administered with PBS). After 3 weeks of myopia induction and eye drop administration, the eyeballs were enucleated from the mice, and the sclera was isolated. Then, western blotting was performed to analyze the expression levels of p62, Atg5, and Atg12 in each group.

### Results

### The X441A ophthalmic solution can suppress the progression of myopia.

Figs. 35 to 37 show the results of Example 14. Fig. 35 is a graph showing the change in axial length of eyes measured before and after attachment of the lens, and Fig. 36 is a graph showing the change in refractive index measured before and after attachment of the lens. In the group administered with PBS, the attachment of a minus lens resulted in axial length elongation of eyes and myopic shift in refractive index compared to the control eyes. In contrast, such changes were not observed in the group administered with X441A by eye drop.

After 3 weeks of myopia induction and eye drop administration, the eyeballs were enucleated from the mice, and the sclera was isolated. Then, western blotting was performed to analyze the expression levels of p62, Atg5, and Atg12 in the group administered with X441A and the group administered with PBS. Fig. 37 shows the results.

### X441A may promote autophagic flux in the sclera.

Although endoplasmic reticulum stress occurs in the myopic sclera and has been shown to be involved in the progression of myopia (Reference Material 47), activation of the endoplasmic reticulum stress pathway caused by myopia induction (assessed according to the ratios of pIRE1/IRE and p-eIF2/eIF2) did not occur in the group administered with X441A by eye drop. Thus, the attachment of a minus lens may enhance autophagy, and autophagy was not shown to be complete in the control group. Furthermore, as shown in Fig. 37, while the increase in expression of At5/Atg12, which is involved in the formation and elongation of autophagosomes, was enhanced regardless of the administration of X441A, the expression level of p62, which acts as a receptor for degraded proteins, was decreased in the group administered with X441A. This suggests that despite the induction of autophagy and the formation of autophagosomes due to myopia induction, the subsequent degradation process did not occur in the group administered with PBS, whereas the subsequent degradation process was activated with autophagic flux enhanced in the group administered with X441A. This is thought to be the mechanism by which myopia progression is inhibited.

As shown by the results of Example 14, the present disclosure indicates that the X441A ophthalmic solution can inhibit the progression of myopia, and further that X441A may promote autophagic flux in the sclera.

The present application claims priority based on Japanese Patent Application No. 2022-138149, filed on August 31, 2022, the disclosure of which is incorporated herein in its entirety.

The present disclosure can also be expressed, for example, as noted below. However, the following supplementary notes are merely examples, and the present disclosure is not limited to these.

### Appendix 1

A compound represented by chemical formula (I), a tautomer or stereoisomer of the compound, or a salt of the compound or of the tautomer or stereoisomer of the compound: 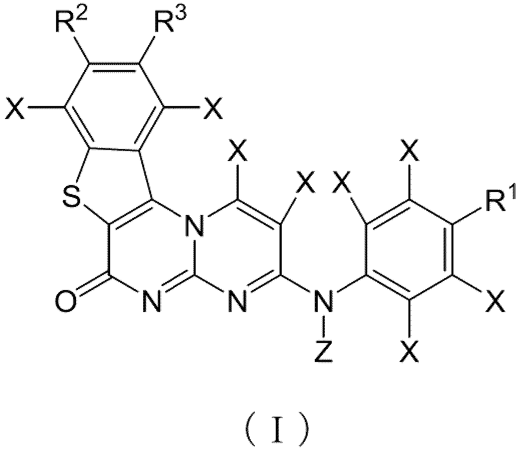 wherein in chemical formula (I)
R¹ is a hydrogen atom or a substituent represented by -SO₂-R¹¹, wherein R¹¹ is a hydrogen atom or any substituent,
R² and R³ each represent a hydrogen atom, a halogen, a nitro group, a linear or branched alkyl group, a linear or branched alkoxy group, or a cyano group, wherein at least one hydrogen atom of the alkyl group or of the alkoxy group is optionally further substituted with a substituent, or R² and R³ may be joined together to form a cyclic structure with the benzene ring to which they are attached,
Xs independently represent a hydrogen atom or any substituent, and each X may be the same or different, and
Z is a hydrogen atom or any substituent.

### Appendix 2

The compound according to Appendix 1, wherein in chemical formula (I), Z is a hydrogen atom, or a substituent selected from the group consisting of non-aromatic hydrocarbon groups (that may be linear or branched and saturated or unsaturated and optionally contain a cyclic structure), aromatic groups (e.g., an aromatic group containing no heteroatoms (an aryl group), or a heteroaromatic group containing a heteroatom (a heteroaryl group), and may be a single ring or a condensed ring), halogens, an amino group, a nitro group, a sulfo group, and a cyano group, wherein at least one hydrogen atom of each substituent is optionally further substituted with any other substituent;
a tautomer or a stereoisomer of the compound; or
a salt of the compound or the tautomer or stereoisomer of the compound.

### Appendix 3

The compound according to Appendix 1, wherein in chemical formula (I), Xs and Z are all a hydrogen atom;
a tautomer or a stereoisomer of the compound; or
a salt of the compound or of the tautomer or stereoisomer of the compound.

### Appendix 4

The compound according to any one of Appendices 1 to 3, wherein the linear or branched alkyl group as R² and R³ in chemical formula (I) is a C₁-C₆ linear or branched alkyl group, and the linear or branched alkoxy group as R² and R³ in chemical formula (I) is a C₁-C₆ linear or branched alkoxy group;
a tautomer or a stereoisomer of the compound; or
a salt of the compound or the tautomer or stereoisomer of the compound.

### Appendix 5

The compound according to any one of Appendices 1 to 4, wherein R¹¹ in R¹ of chemical formula (I) is a hydrogen atom, a linear or branched alkyl group, or an amino group, wherein at least one of the hydrogen atoms of the amino group is optionally substituted with a substituent;
a tautomer or stereoisomer of the compound; or
a salt of the compound or of the tautomer or stereoisomer of the compound.

### Appendix 6

The compound according to Appendix 5, wherein the substituent of the amino group is an aminoiminomethyl group;
a tautomer or a stereoisomer of the compound; or
a salt of the compound or of the tautomer or stereoisomer of the compound.

### Appendix 7

The compound according to Appendix 1,
a tautomer or stereoisomer of the compound, or
a salt of the compound or of the tautomer or stereoisomer of the compound,

wherein in chemical formula (I),
Xs and Z are all a hydrogen atom,
R¹ is a sulfonamide group, and
R² is a hydrogen atom, a fluorine atom, a C₁-C₆ alkyl group, or a C₁-C₆ alkoxy group.

### Appendix 8

The compound according to Appendix 1, wherein the compound represented by chemical formula (I) is a compound represented by any one of the following chemical formulas 1 to 6 and XD8 to XD28: 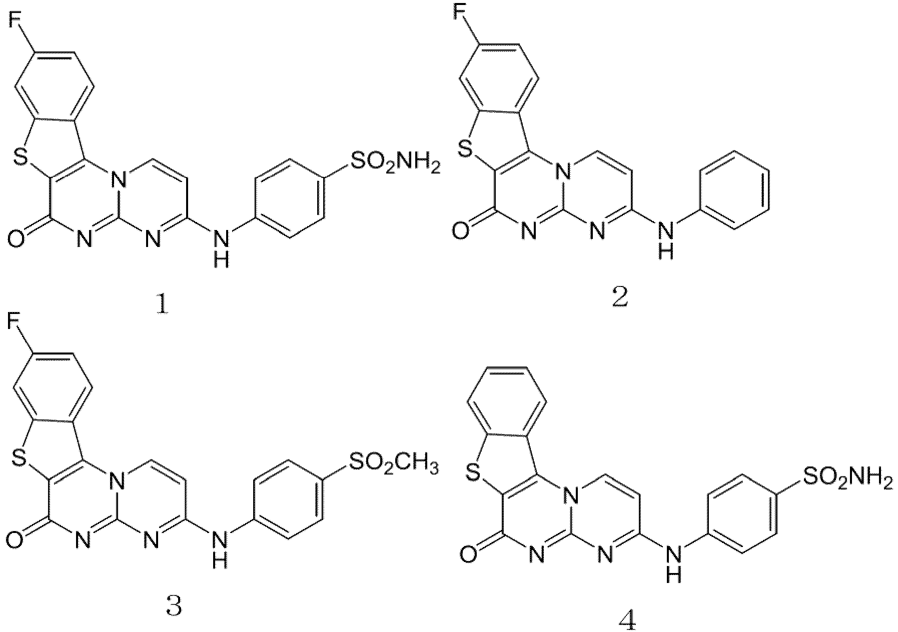 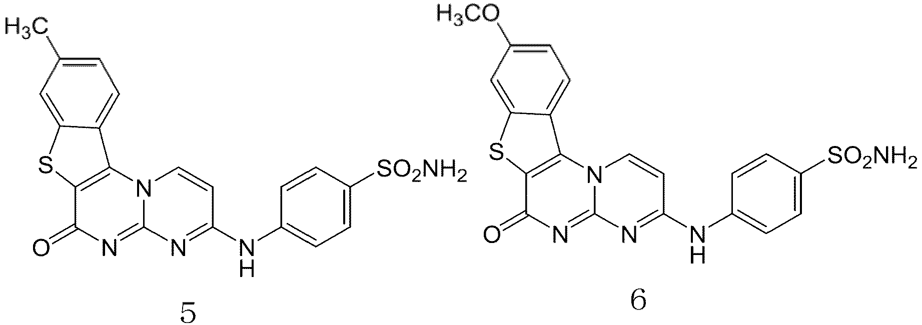 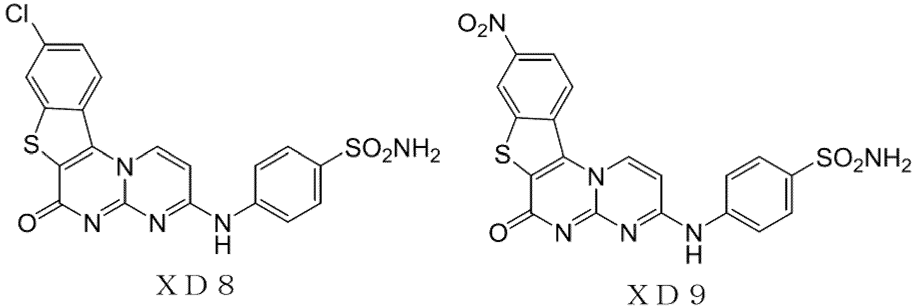 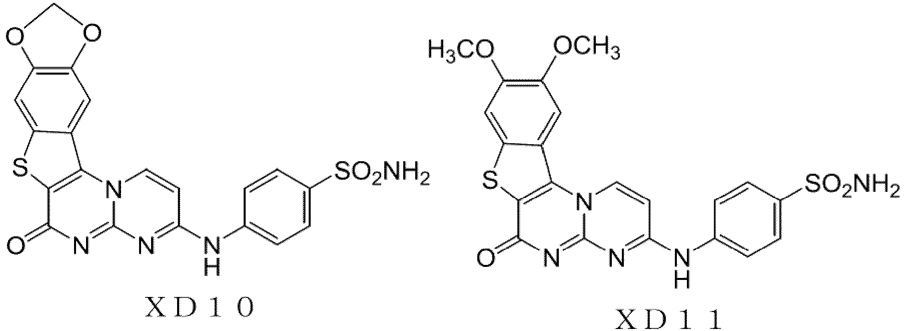 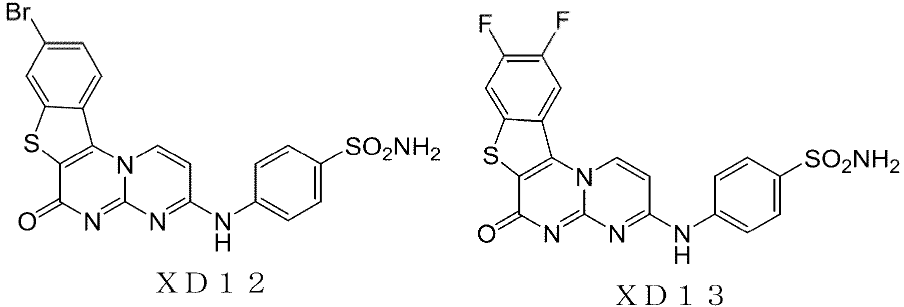 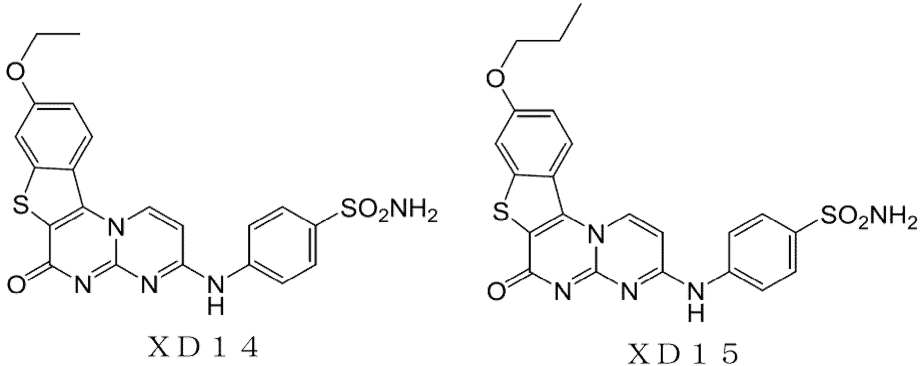 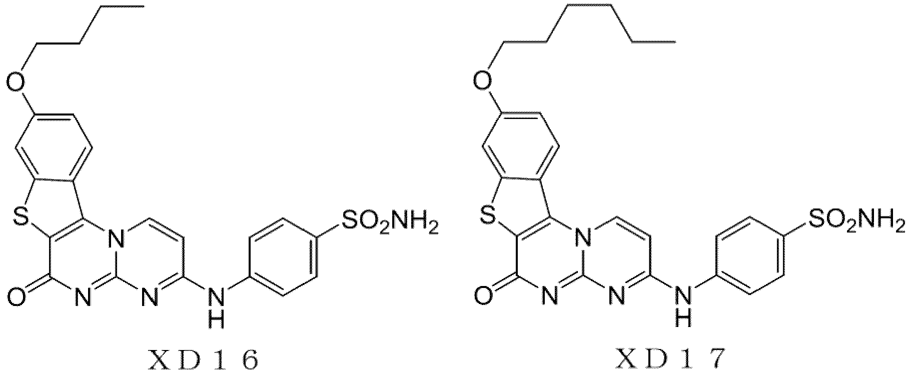 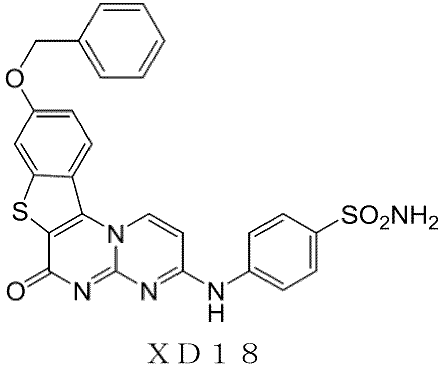 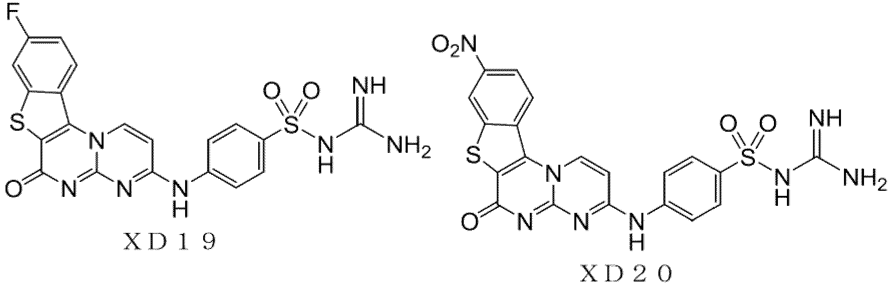 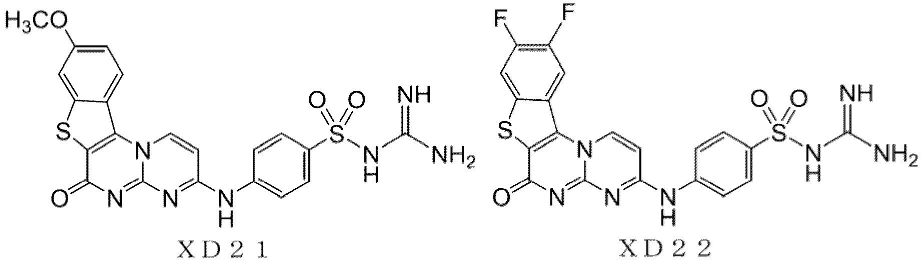 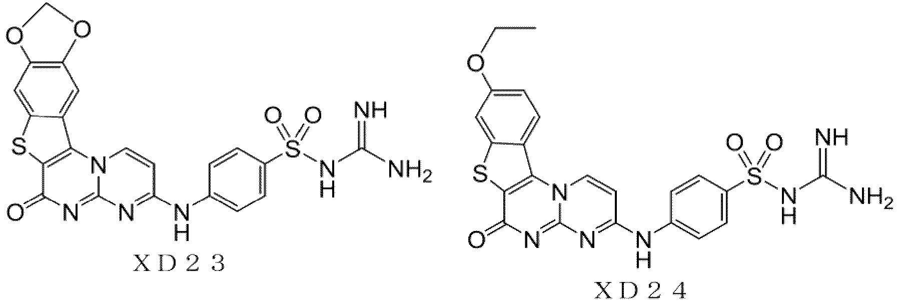 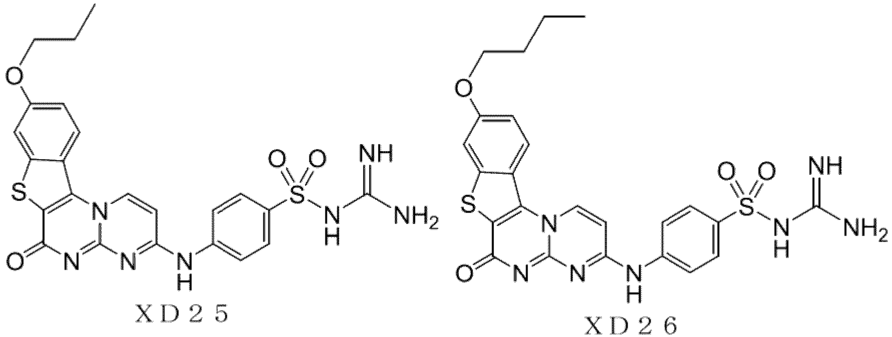 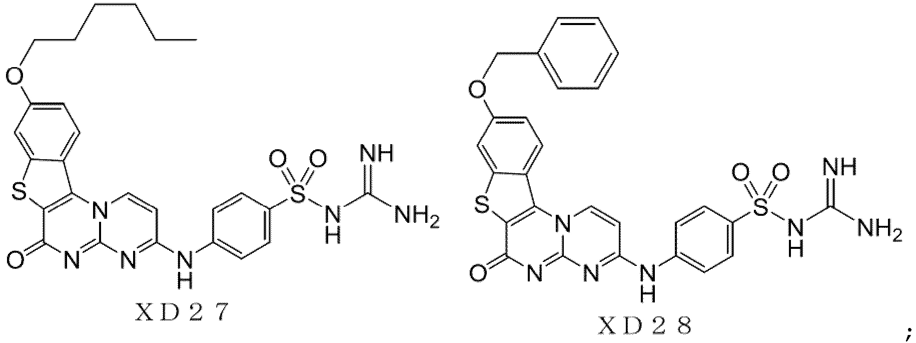
a tautomer or stereoisomer of the compound; or
a salt of the compound or of the tautomer or stereoisomer of the compound.

### Appendix 9

The compound according to Appendix 8, wherein the compound represented by chemical formula (I) is a compound represented by chemical formula 1, 4, 5, or 6;
a tautomer or stereoisomer of the compound; or
a salt of the compound or of the tautomer or stereoisomer of the compound.

### Appendix 10

The compound according to Appendix 8, wherein the compound represented by chemical formula (I) is a compound represented by chemical formula 6;
a tautomer or stereoisomer of the compound; or
a salt of the compound or of the tautomer or stereoisomer of the compound.

### Appendix 11

An endoplasmic reticulum stress inhibitor comprising the compound of any one of Appendices 1 to 10;
a tautomer or stereoisomer of the compound; or
a salt of the compound or of tautomer or stereoisomer of the compound.

### Appendix 12

A medicament comprising
the compound of any one of Appendices 1 to 10;
a tautomer or stereoisomer of the compound; or
a salt of the compound or of the tautomer or stereoisomer of the compound.

### Appendix 13

The medicament according to Appendix 12 for corneal endothelial dystrophy.

### Appendix 14

An ophthalmic preparation comprising
the compound of any one of Appendices 1 to 10;
a tautomer or stereoisomer of the compound; or
a salt of the compound or of the tautomer or stereoisomer of the compound.

### Appendix 15

An eye drop comprising
the compound of any one of Appendices 1 to 10;
a tautomer or stereoisomer of the compound; or
a salt of the compound or of the tautomer or stereoisomer of the compound.

### Appendix 16

A composition for preventing or treating an eye condition, disorder, or disease, comprising
the compound of any one of Appendices 1 to 10;
a tautomer or stereoisomer of the compound; or
a salt of the compound or of the tautomer or the stereoisomer of the compound.

### Appendix 17

The composition according to Appendix 16, wherein the eye condition, disorder, or disease is a condition, disorder, or disease of the corneal endothelium.

### Appendix 18

The composition according to Appendix 16 or 17, wherein the eye condition, disorder, or disease is a corneal endothelial condition, disorder, or disease due to transforming growth factor-β (TGF-β).

### Appendix 19

The composition according to Appendix 17 or 18, wherein the corneal endothelial condition, disorder, or disease is at least one member selected from the group consisting of Fuchs' endothelial corneal dystrophy, post-corneal transplant disorder, corneal endotheliitis, trauma, ophthalmic surgery, disorders after ophthalmic laser surgery, aging, posterior polymorphous corneal dystrophy (PPD), congenital hereditary corneal endothelial dystrophy (CHED), idiopathic corneal endothelial disorder, and cytomegalovirus corneal endotheliitis.

### Appendix 20

The composition according to any of Appendices 17 to 19, wherein the corneal endothelium condition, disorder, or disease is a condition, disorder, or disease of the corneal endothelium due to overexpression of the extracellular matrix (ECM) .

### Appendix 21

The composition according to Appendix 20, wherein the corneal endothelial condition, disorder, or disease is at least one member selected from the group consisting of Fuchs' endothelial dystrophy, guttae formation, Descemet's membrane thickening, corneal hypertropy, opacity, scarring, corneal stromal opacity, corneal epithelial edema, corneal epithelial disorder, nubecula corneae, corneal macula, corneal leukoma, photophobia, and blurred vision.

### Appendix 22

The composition according to any one of Appendices 16 to 21, wherein the condition, disorder, or disease includes Fuchs' endothelial corneal dystrophy.

### Appendix 23

The composition according to any of Appendices 16 to 22, wherein the eye condition, disorder, or disease is a condition, disorder, or disease of the eye due to overexpression of the extracellular matrix (ECM).

### Appendix 24

The composition according to any one of Appendices 16 to 23, wherein the compound of any one of Appendices 1 to 9, a tautomer or stereoisomer of the compound, or a salt of the compound or of the tautomer or stereoisomer of the compound has endoplasmic reticulum stress suppression activity.

### Appendix 25

A method for producing the compound of any one of Appendices 1 to 10, a tautomer or stereoisomer of the compound, or a salt of the compound or of the tautomer or stereoisomer of the compound, the method comprising
a hydrolysis step of hydrolyzing
   the compound represented by the following chemical formula (II): 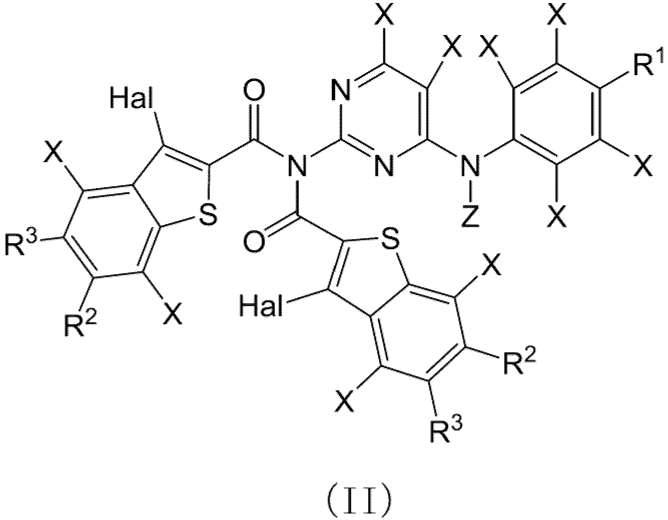 wherein in chemical formula (II), R¹, R², R³, Xs, and Z are the same as defined above in chemical formula (I), and Hal is a halogen,
   a tautomer or stereoisomer of the compound, or
   a salt of the compound, the tautomer, or the stereoisomer; and
a cyclization step of cyclizing the product of the hydrolysis step.

### Appendix 26

The medicament according to Appendix 12 for myopic eyes.

### Appendix 27

The medicament according to Appendix 16, wherein the eye condition, disorder, or disease is a myopic condition, disorder, or disease.

### Industrial Applicability

As explained above, according to the present disclosure, a novel compound having self-assembly activity, an endoplasmic reticulum stress inhibitor, a medicament, an ophthalmic agent, eye drops, a composition, and a method for producing the compound can be provided. The compound of the present disclosure can be used, for example, as a lead compound for medicaments for neurodegenerative diseases, metabolic diseases, and ophthalmological diseases. Further, the compound of the present disclosure can be used as a self-assembling material that co-assembles with a denatured protein to reduce proteotoxicity and endoplasmic reticulum (ER) stress, for example, as described above in the Examples. Accordingly, the compound of the present disclosure provides a valuable research approach for studying protein homeostasis and the pathogenesis of endoplasmic reticulum stress and protein toxicity. Further, the compound of the present disclosure can be used for any purpose related to the inhibition of endoplasmic reticulum stress. Applications related to the inhibition of endoplasmic reticulum stress are not particularly limited, and examples include the various applications mentioned above. Further, application of the compound of the present disclosure is not limited to applications related to the inhibition of endoplasmic reticulum stress, and the compound of the present disclosure can be used for any purpose.

## Claims

1. A compound represented by chemical formula (I), a tautomer or stereoisomer of the compound, or a salt of the compound or of the tautomer or stereoisomer of the compound: 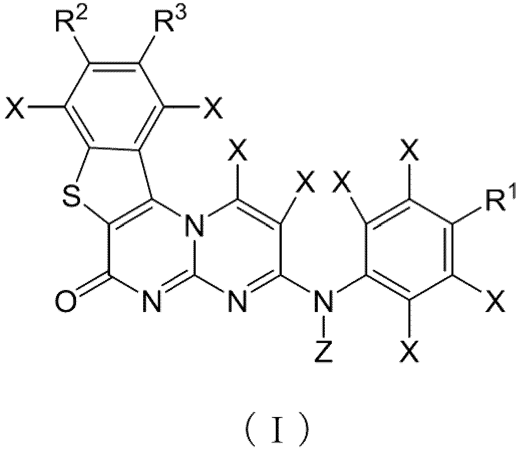 wherein in chemical formula (I)
R¹ is a hydrogen atom or a substituent represented by -SO₂-R¹¹, wherein R¹¹ is a hydrogen atom or any substituent,
R² and R³ are each a hydrogen atom, a halogen, a nitro group, a linear or branched alkyl group, a linear or branched alkoxy group, or a cyano group, wherein at least one hydrogen atom of the alkyl group or of the alkoxy group is optionally further substituted with a substituent, or
R² and R³ may be joined together to form a cyclic structure with the benzene ring to which they are attached,
Xs independently represent a hydrogen atom or any substituent and each X may be the same or different, and
Z is a hydrogen atom or any substituent.

2. The compound, tautomer or stereoisomer, or salt according to claim 1, wherein in chemical formula (I), Xs and Z are all a hydrogen atom.

3. The compound, tautomer or stereoisomer, or salt according to claim 1 or 2, wherein in chemical formula (I), the linear or branched alkyl group as R² and R³ is a C₁-C₆ linear or branched alkyl group and the linear or branched alkoxy group as R² and R³ is a C₁-C₆ linear or branched alkoxy group.

4. The compound, tautomer or stereoisomer, or salt according to any one of claims 1 to 3, wherein in chemical formula (I), R¹¹ in R¹ represents a hydrogen atom, a linear or branched alkyl group, or an amino group, wherein at least one hydrogen atom of the amino group is optionally substituted with a substituent.

5. The compound, tautomer or stereoisomer, or salt according to claim 4, wherein the substituent of the amino group is an aminoiminomethyl group.

6. The compound, tautomer or stereoisomer, or salt according to claim 1, wherein in chemical formula (I),
Xs and Z are all a hydrogen atom,
R¹ is a sulfonamide group, and
R² is a hydrogen atom, a fluorine atom, a C₁-C₆ alkyl group, or a C₁-C₆ alkoxy group.

7. The compound, tautomer or stereoisomer, or salt according to claim 1, wherein the compound represented by chemical formula (I) is a compound represented by any one of the following chemical formulas 1 to 6 and chemical formulas XD8 to XD28:

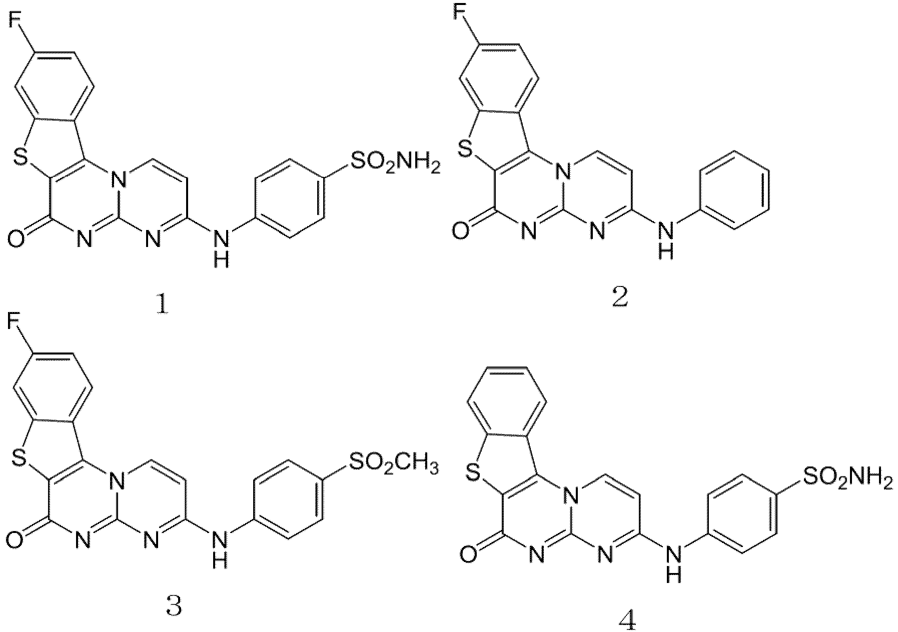

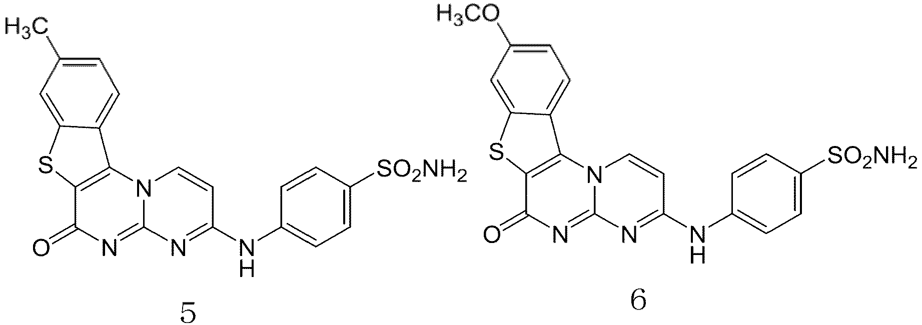

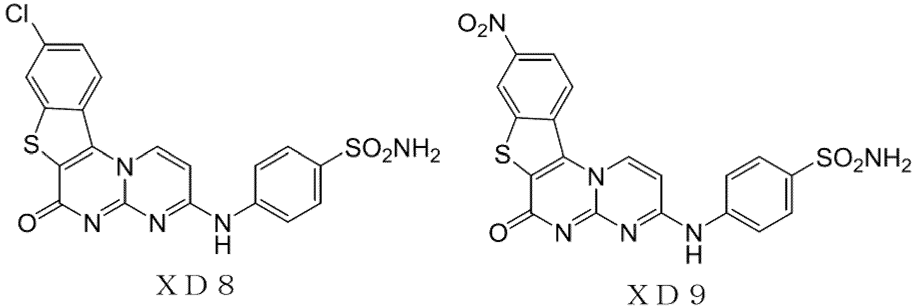

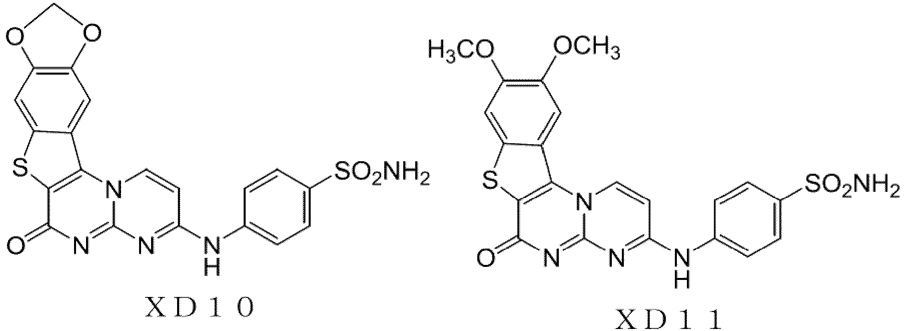

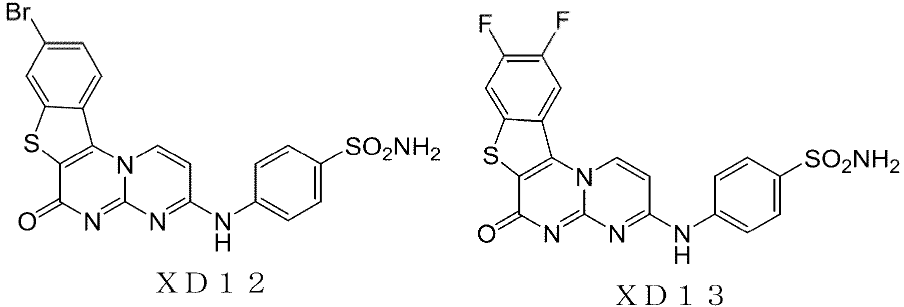

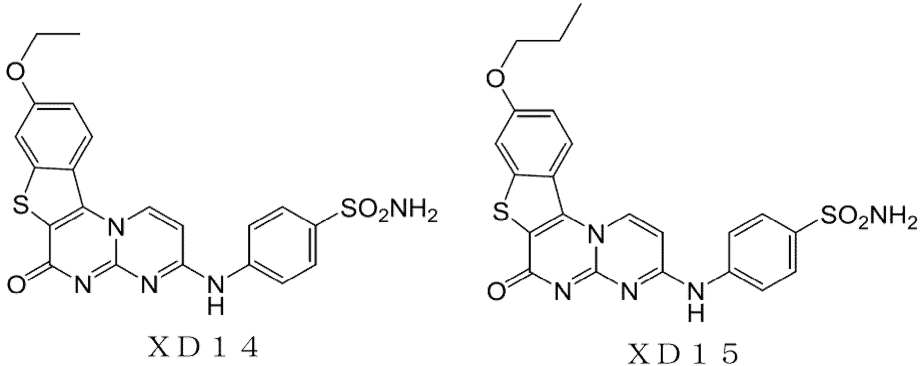

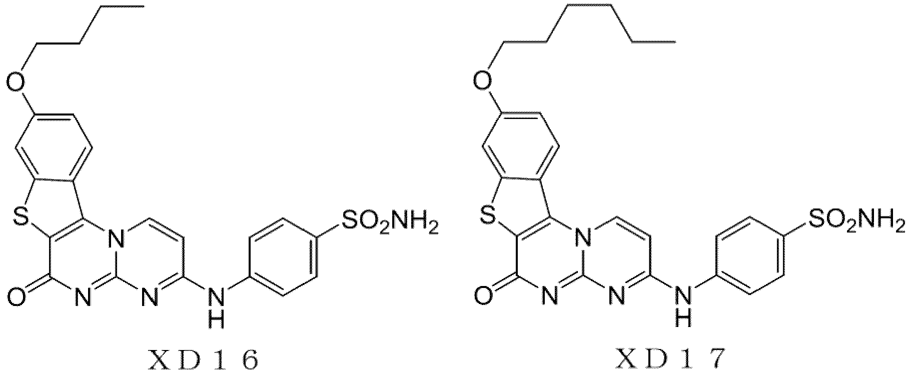

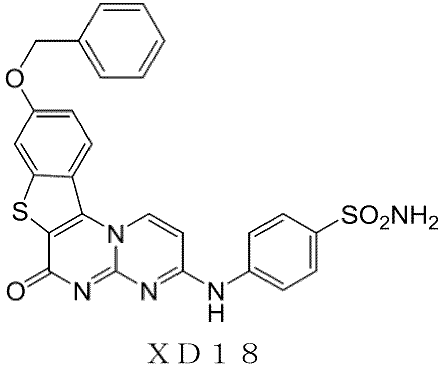

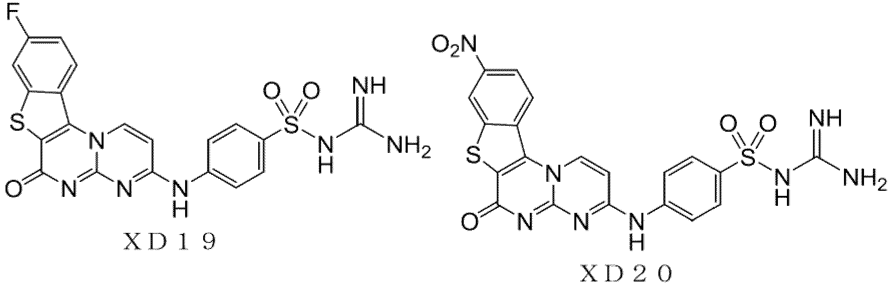

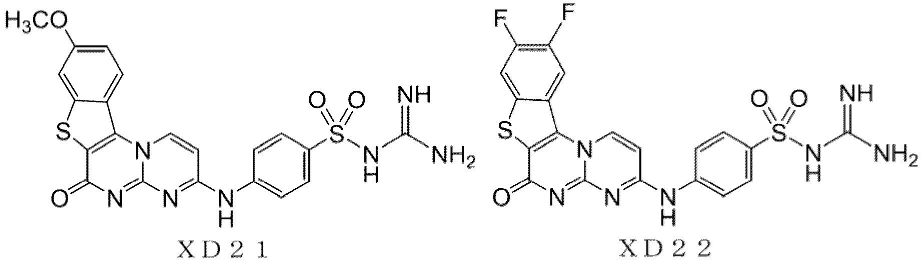

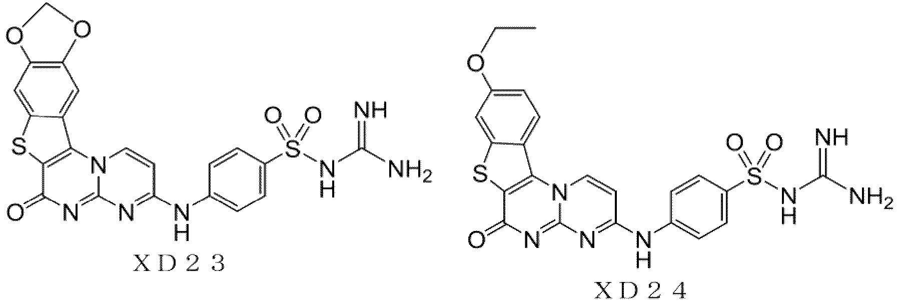

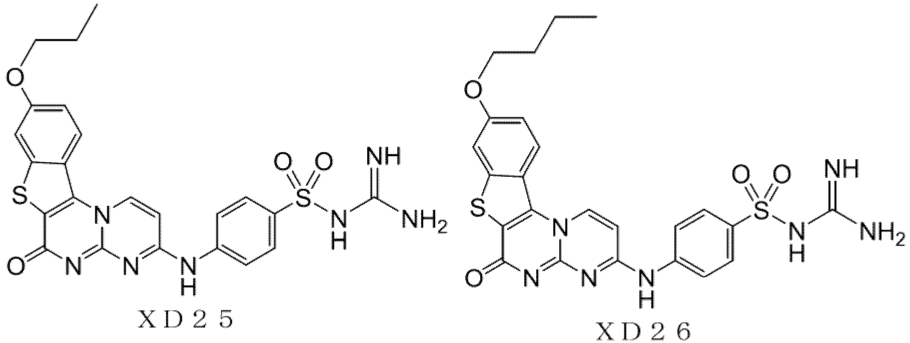

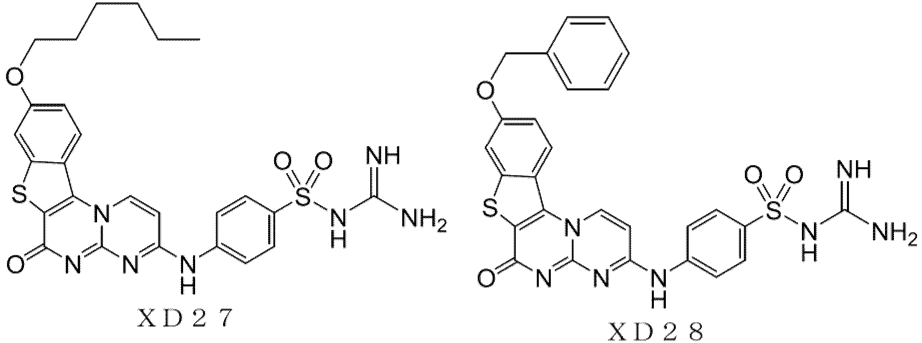

8. The compound, tautomer or stereoisomer, or salt according to claim 7, wherein the compound represented by chemical formula (I) is a compound represented by chemical formula 1, 4, 5, or 6.

9. The compound, tautomer or stereoisomer, or salt according to claim 7, wherein the compound represented by chemical formula (I) is a compound represented by chemical formula 6.

10. An endoplasmic reticulum stress inhibitor, comprising the compound, tautomer or stereoisomer, or salt of any one of claims 1 to 9.

11. A medicament comprising
the compound, tautomer or stereoisomer, or salt of any one of claims 1 to 9.

12. The medicament according to claim 11, which is for corneal endothelial dystrophy.

13. The medicament according to claim 11, which is for myopic eyes.

14. An ophthalmic agent or eye drop comprising
the compound, tautomer or stereoisomer, or salt of any one of claims 1 to 9.

15. A composition for preventing or treating an eye condition, disorder, or disease, comprising
the compound, tautomer or stereoisomer, or salt of any one of claims 1 to 9.

16. The composition of claim 15, wherein the eye condition, disorder, or disease is a condition, disorder, or disease of a corneal endothelium.

17. The composition according to claim 15 or 16, wherein the eye condition, disorder, or disease is a corneal endothelial condition, disorder, or disease due to transforming growth factor-β (TGF-β), or a corneal endothelial condition, disorder, or disease due to overexpression of extracellular matrix (ECM).

18. The composition according to any one of claims 15 to 17, wherein the eye condition, disorder, or disease is at least one member selected from the group consisting of Fuchs' endothelial corneal dystrophy due to transforming growth factor-β (TGF-β), post-corneal transplant disorder, corneal endotheliitis, trauma, ophthalmic surgery, disorders after ophthalmic laser surgery, aging, posterior polymorphous corneal dystrophy (PPD), congenital hereditary corneal endothelial dystrophy (CHED), idiopathic corneal endothelial disorder, and cytomegalovirus corneal endotheliitis, or at least one member selected from the group consisting of Fuchs' endothelial corneal dystrophy due to overexpression of extracellular matrix (ECM), guttae formation, Descemet's membrane thickening, corneal hypertropy, opacity, scar, corneal stromal opacity, corneal epithelial edema, corneal epithelial disorder, nubecula corneae, corneal macula, corneal leukoma, photophobia, and blurred vision.

19. The composition according to any one of claims 15 to 18, wherein the eye condition, disorder, or disease includes Fuchs' endothelial corneal dystrophy.

20. The composition according to claim 15, wherein the eye condition, disorder, or disease is a myopic condition, disorder, or disease.

21. The composition according to claim 15 or 20, wherein the eye condition, disorder, or disease is a condition, disorder, or disease of the eye caused by refractive error, axial elongation of eyes, choroidal thinning, or reduced choroidal blood flow.

22. A method for producing the compound, tautomer or stereoisomer, or salt of any one of claims 1 to 9, the method comprising
a hydrolysis step of hydrolyzing
a compound represented by the following chemical formula (II), a tautomer or stereoisomer of the compound, or a salt of the compound or of the tautomer or stereoisomer of the compound: 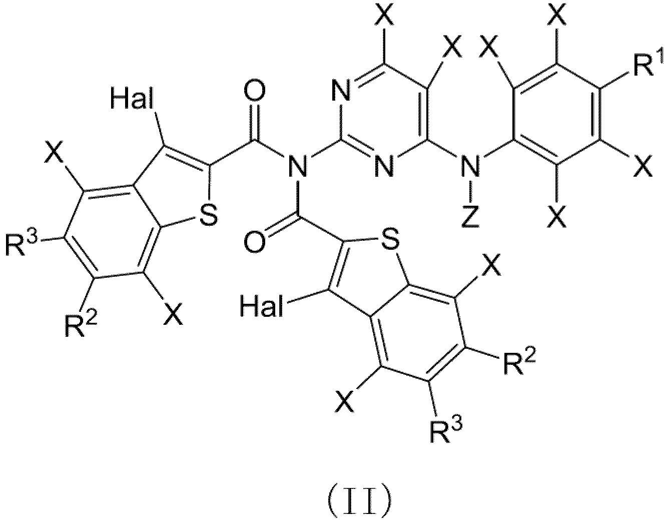 wherein in chemical formula (II), R¹, R², R³, Xs and Z are the same as those defined in chemical formula (I), and Hal is a halogen, and
a cyclization step of cyclizing the product of the hydrolysis step.
